(19) Europäisches Patentamt
European Patent Office
Office européen des brevets

(11) EP 4 218 810 A2

(12) EUROPEAN PATENT APPLICATION

(43) Date of publication:
02.08.2023 Bulletin 2023/31

(21) Application number: 22208629.0

(22) Date of filing: 02.10.2015

(51) International Patent Classification (IPC):
*A61K 39/395* (2006.01) *C07K 4/00* (2006.01)
*C07K 16/00* (2006.01)

(52) Cooperative Patent Classification (CPC):
**C07K 16/32; A61K 47/60; A61K 47/6801; A61K 47/6879; A61K 47/6889; A61P 35/00; C07K 16/00; C07K 16/2803; C07K 16/2863;**
A61K 2039/505; C07K 2317/35; C07K 2317/40; C07K 2317/56; C07K 2317/77; C07K 2317/92

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 02.10.2014 US 201462059146 P

(62) Document number(s) of the earlier application(s) in accordance with Art. 76 EPC:
15845573.3 / 3 200 823

(71) Applicant: **City of Hope**
**Duarte, CA 91010-3000 (US)**

(72) Inventor: **WILLIAMS, John, C.**
**CA, 91016 (US)**

(74) Representative: **Hartz, Nikolai et al**
**Wächtershäuser & Hartz**
**Patentanwaltspartnerschaft mbB**
**Weinstrasse 8**
**80333 München (DE)**

Remarks:
- The complete document including Reference Table(s) and the Sequence Listing(s) can be downloaded from the EPO website
- This application was filed on 21-11-2022 as a divisional application to the application mentioned under INID code 62.
- Claims filed after the date of filing/receipt of the divisional application (Rule 68(4) EPC).

# (54) MULTIVALENT MEDITOPES, MEDITOPE-BINDING ANTIBODIES AND USES THEREOF

(57) Provided are methods for altering the distribution of a cell surface antigen. Also provided are compositions for use in the methods, including multivalent meditopes, and methods of producing, using, testing, and screening the same, including therapeutic and diagnostic methods and uses.

EP 4 218 810 A2

## Description

### PRIORITY CLAIM

This application claims the benefit of United States Provisional Patent Application Serial Number 62/059,146, filed October 2, 2014, the contents of which are incorporated herein by reference in their entirety.

### INCORPORATION BY REFERENCE OF SEQUENCE LISTING

The present application is being filed along with a Sequence Listing in electronic format. The Sequence Listing is provided as a file entitled 706122000540SEQLIST.TXT, created October 2, 2015, which is 451 kilobytes in size. The information in the electronic format of the Sequence Listing is incorporated by reference in its entirety.

### BACKGROUND

Monoclonal antibodies (mAbs) are used in a number of therapeutic, diagnostic, and research applications. Therapeutic and diagnostic areas include cancer, antiviral treatment, autoimmune and inflammatory disease, allergy, cardiovascular disease, osteoporosis, and rheumatology.

Protein engineering and other efforts have generated mAbs with improved efficacy and targeting (e.g., bispecific mAbs), improved localization, tissue penetration, and blood clearance (e.g., single chain Fab variable fragments (scFvs), diabodies, minibodies, and other fragments), and altered immunostimulatory, safety, toxicity, and/or pharmacokinetic/pharmacodynamics properties, such as those containing modified Fc regions (e.g., through mutation or glycosylation). mAbs have been reengineered to permit site-specific conjugation of small molecules for improved delivery (e.g., ThioMABs) or to irreversibly bind to their cognate epitope (e.g., infinite affinity mAbs). mAbs have also been developed to improve the circulation and presentation of bioactive peptides and other biologics (e.g., CovX-bodies). Conjugation to various agents has allowed targeted immunotherapy and diagnostic methods. Hetero-multimeric scFvs and scFvs or mAbs fused to avidin have been developed for pre-targeted therapy and to improve the detection limits for tumor imaging.

Although mAbs can be effective and have advantages over small molecule approaches, existing antibodies and methods have various limitations. These can include adverse side effects resulting from off-target interactions, and/or collateral damage due to, among other things, long circulation times of antibody-drug conjugates. Addtionally, while some antibodies are capable of binding to an epitope on a cell-surface antigen effectively, they may require internalization by the cell to produce a desired or intended result. Merely binding to the cognate antigen may not be sufficient to elicit a therapeutic resonse. Increasing the rate of internalization of a cell surface antigen could improve the efficacy of an antibody or antibody therapy. In view of these short comings, there is a need for improved antibodies and associated compounds, including those providing improved efficacy, synergy, specificity, and safety, and methods and uses of the same. Provided herein are antibodies, compounds and compositions including peptides and other molecules, and related methods that address such needs.

### SUMMARY

The disclosed comprises a method of increasing or promoting cellular internalization of an antibody, comprising administering to the cell a meditope-enabled antibody that specifically binds to an epitope presented on a cell or a cell-surface antigen and a multivalent meditope, thereby promoting internalization of the cell-surface antigen on the surface of the target cell.

Also provided are methods of increasing or promoting clustering of a cell-surface antigen on a cell, comprising administering to the cell a meditope-enabled antibody that specifically binds to the cell-surface antigen and a multivalent meditope, thereby promoting clustering of the cell-surface antigen on the surface of the target cell. In one aspect, the degree of clustering following administration of the meditope-enabled antibody and multivalent meditope is increased compared to the degree of clustering observed following incubation with the antibody alone or is increased compared to incubation with the antibody and a monovalent meditope.

Another embodiment provides for a method of increasing or promoting internalization of a cell-surface antigen on a cell, comprising administering to the cell a meditope-enabled antibody that specifically binds to the cell-surface antigen and a multivalent meditope, thereby promoting internalization of the cell-surface antigen on the surface of the target cell. In one aspect, the degree of internalization following administration of the meditope-enabled antibody and multivalent meditope is increased compared to the degree of internalization of the antigen following incubation with the antibody alone or is increased compared to incubation with the antibody and a monovalent meditope.

Another embodiment provides delivering a multivalent meditope to a cell, comprising administering to the cell a meditope-enabled antibody that specifically binds to the cell-surface antigen and a multivalent meditope, thereby

promoting clustering of the cell-surface antigen on the surface of the target cell and/or internalization of the antigen. In another aspect, the antigen of interest is an internalizing or non-internalizing antigen. In another aspect, the binding of a monospecific bivalent antibody in the absence of meditope results in no internalization of the antigen, a degree of internalization that is no more than that resulting from metabolic turnover or in the absence of an antibody specific for the antigen, or that is not greater than 2-fold greater than that resulting from metabolic turnover or in the absence of an antigen-specific antibody.

In some embodiments, the meditope-enabled antibody is bivalent or bispecific. Another embodiment further comprising administering to the cell another meditope-enabled antibody, which binds to an epitope on the antigen that is distinct from the epitope bound by the meditope-enabled antibody or does not compete for binding therewith, or which specifically binds to a different epitope a different antigen on the surface of the cell.

In one example, the method of treatment includes administering to a subject one or more meditope-enabled antibody or fragment, e.g., any of those described herein. In one example, the method includes administering to the subject one or more meditope-enabled antibody or fragment and a meditope, e.g., any of those described herein, including multivalent meditopes and meditopes coupled to a therapeutic or diagnostic agent. In one aspect, the meditope-enabled antibody or fragment and one or more meditope are administered sequentially. In another aspect, they are administered simultaneously.

Generally, the one or more meditopes comprise a peptide that binds to a meditope binding site of the meditope-enabled antibody or antigen binding fragment thereof. In one aspect, the meditope-enabled antibody is administered simultaneously with, prior to, or after the administration of the one or more meditope. In some aspects, the one or more meditope is coupled to a therapeutic agent, such as a therapeutic agent selected from the group consisting of: drugs, small molecules, chemotherapeutic agents, therapeutic antibodies, toxins, radioisotopes, enzymes, nucleases, hormones, immunomodulators, oligonucleotides, RNAi molecules, siRNA molecules, chelators, boron compounds, photoactive agents, dyes, fluorescent or luminescent substances, metals, metal alloys, enhancing agents, and organic or inorganic nanoparticles.

Also among the provided meditopes are multivalent meditopes, such as those comprising two or more meditopes and one or more linker, e.g., where each of the two or more meditopes is a peptide that binds to a meditope binding site of a meditope-enabled antibody. Such multivalent meditopes may comprise any of the meditopes described herein. In one aspect, the two or more meditopes comprise at least three meditopes, or at least four meditopes. In one aspect, the one or more linker includes a peptide, a small chemical scaffold, a biotin-streptavidin, an organic or inorganic nanoparticle, a polynucleotide sequence, peptide nucleic acid, an organic polymer, or an immunoglobulin Fc domain.

Exemplary multivalent meditopes are bivalent, trivalent, and tetravalent meditopes, as well as multivalent meditopes containing 5, 6, 7, 8, 9, 10, 20, 30 40, 50, 60, 70, 80, 90, 100, 100s, 1000, 1000s, or more meditopes. In some aspects, such multivalent meditopes are achieved by coupling the meditopes to nanoparticles, viruses, and/or other supports such as other semisolid supports, for example, nanotubes, buckyballs, LGA particles, and/or quantum dots, for example, for use in inter operative imaging.

Among the multivalent meditopes are compounds including one or more chains that include a meditope linked via a linker to an immunoglobulin (Ig) constant region or portion thereof (e.g., $R^{4A}$ in the formulas below). The Ig constant region or portion thereof in some embodiments is an Fc region or portion thereof, e.g., a heavy chain CH1, CH2, and/or CH3 domain. In some aspects, the Ig constant region portion further includes a CH4 domain. The Ig in some aspects is an IgG. In some aspects, the constant region portion does not contain a CH2, does not contain a CH1, does not contain a CH4, and/or does not contain a variable region or antigen binding portion. Such compounds and meditopes are often bi- or multivalent, e.g., tetravalent, by virtue of the interaction between multiple Ig constant region portions, e.g., Fc portions. In one example, the multivalent meditope has one or more chain, such as one or more polypeptide chain, having the formula:

$$R^{3A}\text{-}L^{3A}\text{-}R^{4A} \qquad \text{(Formula I)},$$

in which $R^{3A}$ is a meditope or central cavity binding moiety, such as a peptide meditope or binding moiety, $L^{3A}$ is a linker, such as a peptidyl linker, linking $R^{3A}$ and $R^{4A}$, and $R^{4A}$ is an immunoglobulin (Ig) heavy chain constant region portion, comprising an Ig Fc region or functional fragment thereof.

In some embodiments, the chain has a formula:

$$R^{3A}\text{-}L^{3A}\text{-}R^{4A}\text{-}Z^{1} \qquad \text{(Formula II)},$$

in which $R^{3A}$ is a first meditope or central cavity binding moiety, such as a peptide meditope or binding moiety; $L^{3A}$ is a first linker such as a peptidyl linker; $R^{4A}$ is an immunoglobulin (Ig) heavy chain constant region portion, comprising an Ig Fc region or functional fragment thereof; $Z^1$ is hydrogen, an amino acid sequence comprising between 0 and 50 amino acids, $-L^{3B}\text{-}R^{3B}$ or $-L^{3B}\text{-}R^{3B}\text{-}R^{5B}$, in which $L^{3B}$ is a second linker; $R^{3B}$ is a second meditope; and $R^{5B}$ is an amino acid

sequence comprising between 0 and 50 amino acids.

In some cases, the meditope is a first meditope, the linker is a first linker, and the chain comprises the formula:

$R^{3A}$-$L^{3A}$-$R^{4A}$-$L^{3B}$-$R^{3B}$ (Formula III),

in which $L^{3B}$ is a second linker linking $R^{4A}$ and $R^{3B}$; and $R^{3B}$ is a second meditope or central cavity binding moiety.

In some contexts, the compound, chain, meditope or central cavity binding moiety, e.g., peptide meditope, includes a tail or leader sequence, for example, a sequence having one or more glycine or serine, or aspartate residue. Thus, in some embodiments, the chain comprises the formula:

$R^{5A}$-$R^{3A}$-$L^{3A}$-$R^{4A}$-$R^{5B}$ (Formula IV),

or

$R^{5A}$-$R^{3A}$-$L^{3A}$-$R^{4A}$-$L^{3B}$-$R^{3B}$-$R^{5B}$ (Formula V),

in which each of $R^{5A}$ and $R^{5A}$ is null or is a sequence comprising between 0 and 50 amino acids in length, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

In some embodiments, the compound comprises a chain, e.g., a first chain, having a meditope or central cavity binding moiety, such as a peptide meditope (such as an $R^{3A}$ or $R^{3B}$) and a heavy chain constant region portion, comprising an Ig Fc region or functional fragment thereof. These two components of the chain typically are linked via a linker, typically a peptidyl linker, such as a linker of $L^{3A}$ or $L^{3B}$. In some aspects, the chain further includes a tail and/or a leader sequence, which can be an amino acid sequence of between 1 and 50 amino acids.

In some embodiments, the various components of the chain are covalently linked, e.g., by amide bond(s). In some embodiments, the linkers of the chains and meditopes and compounds, e.g., $L^{3A}$, $L^{3B}$, or both, are between 25 and 40 amino acids in length, or is at or about 25, 26, 27, 28, 29, 30, 31, 32, 33, 34, 35, 36, 37, 38, 39, 40, 41, 42, 43, 44, 45, 46, 47, 48, 49, or 50 amino acids in length, for example, at or about 30, 36, 37, or 39 amino acids in length. In some aspects, the linker, e.g., $L^{3A}$, $L^{3B}$, or both, is between about 2Å and about 300Å, e.g., between about 2Å and about 200 Å in length; is between about 50Å and about 150 Å or between about 50Å and about 200Å in length; or is between about 100Å and about 140Å in length, such as at or about 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 150, 160, 170, 180, 190, or 200, 250, or 300 Å in length.

In some embodiments, the linker includes or has a sequence of SEQ ID NO: 199, 200, 201, 202, 203, or 204, or a modified variant thereof, such as one in which wherein any one, two, three, or four of the residues is optionally substituted with a conjugated amino acid, and/or in which one or more lysine is mutated to a different amino acid, such as an arginine. In some cases, the conjugated amino acid is substituted for (i.e., inserted in place of) a lysine.

Also provided are compositions, e.g., pharmaceutical compositions, comprising the meditopes (including multivalent and/or labeled meditopes), meditope-enabled antibodies, and complexes, and/or other compounds described herein, e.g., above. In one example, the composition includes the complex, meditope, and/or meditope-enabled antibody, and a pharmaceutically acceptable carrier.

Also provided are methods of treatment, such as those carried out by administering to a subject such pharmaceutical compositions, or any of the meditope-enabled antibodies, meditopes, complexes, and/or other compound described herein, e.g., above. In one example, the methods include administering to the subject an antibody or fragment as described above.

Also provided are methods of delivering a meditope, a meditope-enabled antibody-meditope complex, such as and including multivalent and/or labeled meditopes. In one aspect, such a method can comprise administering the complex or pharmaceutical composition or further includes administering a meditope-enabled antibody or antigen binding fragment thereof, and the administration induces increased aggregation and/or internalization of a cell-surface antigen specifically bound by the meditope-enabled antibody. The antigen can be a tumor-associated or tumor-specific antigen. The antigen can be an internalizing or non-internalizing antigen. Such methods can involve the delivery of a therapeutic or diagnostic agent to a cell expressing an antigen of interest. In various aspects the therapeutic or diagnostic agent can be coupled to one or more meditopes, to the meditope-enabled antibody, to the linker of the various meditopes, or to other structural features of the multivalent meditope suitable to accommodate the conjugation of such an agent. In certain aspects, the multivalent meditope is delivered to a lysosomal compartment of a cell expressing the antigen of interest within 1, 2, 3, 4, 5, 6, 7, 8, 9, 10, or 15 hours or within 1, 2, 3, 4, 5, 6, 7, 8, 9, 10, 14, 21 or 28 days of administration.

In some embodiments, the methods for altering the distribution of a cell surface antigen include contacting a cell with a plurality of meditope enabled antibodies or antigen binding fragments thereof capable of binding to a cell

surface antigen, contacting a meditope binding site of a first meditope enabled antibody or antigen binding fragment thereof of the plurality of meditope enabled antibodies with a first meditope from a multivalent meditope, contacting a meditope binding site of a second meditope enabled antibody or antigen binding fragment thereof of the plurality of meditope enabled antibodies with a second meditope from the multivalent meditope, resulting in crosslinking of the first and second meditope-enabled antibodies, whereby crosslinking the first and second meditope enabled antibodies with the multivalent meditope alters the distribution of the cell surface antigen.

In some embodiments, the methods for increasing co-localization of a cell surface antigen include contacting a cell with a plurality of meditope enabled antibodies or antigen binding fragments thereof capable of binding to a cell surface antigen, contacting a meditope binding site of a first meditope enabled antibody or antigen binding fragment thereof of the plurality of meditope enabled antibodies with a first meditope from a multivalent meditope, contacting a meditope binding site of a second meditope enabled antibody or antigen binding fragment thereof of the plurality of meditope enabled antibodies with a second meditope from the multivalent meditope, resulting in crosslinking of the first and second meditope-enabled antibodies, whereby crosslinking the first and second meditope enabled antibodies with the multivalent meditope increases the co-localization of the cell surface antigen or receptor.

In some embodiments the methods for increasing cellular internalization of a meditope enabled antibody include contacting a cell expressing a cell surface antigen with a plurality of meditope enabled antibodies or antigen binding fragments thereof, whereby said plurality of meditope enabled antibodies or antigen binding fragments thereof bind to the cell surface antigen, contacting a meditope binding site of a first meditope enabled antibody or antigen binding fragment thereof of the plurality of meditope enabled antibodies with a first meditope from a multivalent meditope, contacting a meditope binding site of a second meditope enabled antibody or antigen binding fragment thereof of the plurality of meditope enabled antibodies with a second meditope from the multivalent meditope, resulting in crosslinking of the first and second meditope-enabled antibodies, whereby crosslinking the first and second meditope enabled antibodies with the multivalent meditope increases cellular internalization of the first and second meditope enabled antibodies bound to the cell surface antigen.

In some embodiments, the methods for increasing cellular internalization of a cell surface antigen include contacting a first cell surface antigen with a first meditope enabled antibody or antigen binding fragment thereof that binds to the cell surface antigen, contacting a second cell surface antigen with a second meditope enabled antibody or antigen binding fragment thereof that binds to the second cell surface antigen, contacting a meditope binding site of the first meditope enabled antibody or antigen binding fragment thereof of the plurality of meditope enabled antibodies with a first meditope from a multivalent meditope, contacting a meditope binding site of the second meditope enabled antibody or antigen binding fragment thereof of the plurality of meditope enabled antibodies with a second meditope from the multivalent meditope, thereby crosslinking of the first and second meditope enabled antibodies, and increasing cellular internalization of the first and second cell surface antigen.

In some embodiments, the methods of increasing the efficacy of an antibody therapy include administering to a subject an effective amount of a meditope enabled antibody or antigen binding fragment thereof that specifically binds to a cell surface antigen and an effective amount of a multivalent meditope, forming a complex of a first meditope of the multivalent meditope bound to a first meditope enabled antibody or antigen binding fragment thereof bound to a first cell surface antigen, and a second meditope of the multivalent meditope bound to a second meditope enabled antibody or fragment thereof bound to a second cell surface antigen, resulting in crosslinking of the first and second meditope-enabled antibodies, whereby crosslinking the first and second antibodies increases the efficacy of the antibody therapy.

In some embodiments, the methods of decreasing a dosage of an antibody therapy needed to achieve a desired therapeutic effect in a subject include administering to a subject an effective amount of a meditope enabled antibody or fragment thereof and an effective amount of a multivalent meditope, contacting a first meditope of the multivalent meditope to a first meditope enabled antibody or fragment thereof, contacting a second meditope of the multivalent meditope to a second meditope enabled antibody or fragment thereof, resulting in crosslinking of the first and second meditope enabled antibodies, whereby crosslinking the first and second antibodies decreases the dosage of the antibody therapy needed to achieve the desired therapeutic effect in a subject.

In some embodiments, the methods of crosslinking a first meditope enabled antibody or antigen binding fragment thereof and a second meditope enabled antibody or antigen binding fragment thereof include contacting a cell with a first meditope enabled antibody or antigen binding fragment thereof, wherein said first meditope enabled antibody or antigen binding fragment thereof includes a first meditope binding site, contacting said first meditope binding site with a multivalent meditope, said multivalent meditope includes a first meditope attached to a second meditope, contacting said multivalent meditope with a second meditope enabled antibody or antigen binding fragment thereof, wherein said second meditope enabled antibody or antigen binding fragment thereof includes a second meditope binding site, allowing said first meditope binding site to bind said first meditope, said second meditope binding site to bind said second meditope, said first meditope enabled antibody or antigen binding fragment thereof to bind to said cell and said second meditope enabled antibody or antigen binding fragment thereof to bind to said cell, thereby crosslinking said first meditope enabled antibody or antigen binding fragment thereof and said second meditope enabled antibody or antigen binding fragment

thereof.

In some embodiments, the cell surface antigen is a receptor capable of receptor-mediated endocytosis.

In some embodiments, the multivalent meditope includes an immunoglobulin Fc region or portion thereof linked to a meditope. In some embodiments, the meditope is coupled to a linker. In some embodiments, the linker includes a sequence of SEQ ID NO: 199, 200, 201, 202, 203, or 204, or a variant thereof.

In some embodiments, the multivalent meditope is coupled to a therapeutic agent or diagnostic agent. In some embodiments, the therapeutic agent is selected from the group consisting of a chemotherapeutic agent, a therapeutic antibody, a toxin, a radioisotope, an enzyme, a chelator, a boron compound, a photoactive agent, a dye, a metal, a metal alloy, and a nanoparticle; or the diagnostic agent is an imaging agent selected from the group consisting of a fluorescent substance, a luminescent substance, a dye, and a radioactive isotope.

In some embodiments, the first meditope enabled antibody binds to a different epitope than the second meditope enabled antibody. In some embodiments, the meditope enabled antibody or antigen-binding fragment thereof specifically binds to an antigen expressed by a disease or condition of a cell or tissue thereof. In some embodiments, the disease or condition is a cancer.

In some embodiments, the meditope enabled antibody or antigen-binding fragment thereof and the meditope are administered sequentially. In some embodiments, at least one of the meditope enabled antibodies or antigen binding fragments thereof competes for antigen binding with, or binds to the same epitope as an antibody or antigen-binding fragment thereof selected from the group consisting of abagovomab, abciximab, adalimumab, adecatumumab, alemtuzumab, altumomab, altumomab pentetate, anatumomab, anatumomab mafenatox, arcitumomab, atlizumab, basiliximab, bectumomab, ectumomab, belimumab, benralizumab, bevacizumab, brentuximab, canakinumab, capromab, capromab pendetide, catumaxomab, certolizumab, clivatuzumab tetraxetan, daclizumab, denosumab, eculizumab, edrecolomab, efalizumab, etaracizumab, ertumaxomab, fanolesomab, fontolizumab, gemtuzumab, girentuximab, golimumab, ibritumomab, igovomab, infliximab, ipilimumab, labetuzumab, mepolizumab, muromonab, muromonab-CD3, natalizumab, necitumumab nimotuzumab, ofatumumab, omalizumab, oregovomab, palivizumab, panitumumab, ranibizumab, rituximab, satumomab, sulesomab, ibritumomab, ibritumomab tiuxetan, tocilizumab, tositumomab, trastuzumab, ustekinumab, visilizumab, votumumab, zalutumumab, brodalumab, anrukinzumab, bapineuzumab, dalotuzumab, demcizumab, ganitumab, inotuzumab, mavrilimumab, moxetumomab pasudotox, rilotumumab, sifalimumab, tanezumab, tralokinumab, tremelimumab, and urelumab; or the meditope-enabled antibody or fragment specifically binds to an antigen selected from the group consisting of: CA-125, glycoprotein (GP) IIb/IIIa receptor, TNF-alpha, CD52, TAG-72, Carcinoembryonic antigen (CEA), interleukin-6 receptor (IL-6R), IL-2, interleukin-2 receptor a-chain (CD25), CD22, B-cell activating factor, interleukin-5 receptor (CD125), VEGF, VEGF-A, CD30, IL-1beta, prostate specific membrane antigen (PSMA), CD3, EpCAM, EGF receptor (EGFR), MUC1, human interleukin-2 receptor, Tac, RANK ligand, C5 or other complement proteins, CD1 1a, alpha-v beta-3 integrin, HER2, neu, CD15, CD20, Interferon gamma, CD33, CA-IX, CTLA-4, IL-5, CD3 epsilon, CAM, Alpha-4-integrin, IgE, IgE Fc region, an RSV antigen, F (or fusion) protein of respiratory syncytial virus (RSV), NCA-90 (granulocyte cell antigen), IL-6, GD2, GD3, IL-12, IL-23, IL-17, CTAA16.88, beta-amyloid, IGF-1 receptor (IGF-1R), delta-like ligand 4 (DLL4), alpha subunit of granulocyte macrophage colony stimulating factor receptor, hepatocyte growth factor, IFN-alpha, nerve growth factor, IL-13, CD326, CD19, PD-L1, CD47, and CD137.

In some embodiments, the multivalent meditope includes a peptide having the formula:

X1-X2-X3-X4-X5-X6-X7-X8-X9-X10-X11-X12 (formula VI)

wherein:

X1 is Cys, Gly, β-alanine, diaminopropionic acid, β-azidoalanine, or null;
X2 is Gln or null;
X3 is Phe, Tyr, β,β'-diphenyl-Ala, His, Asp, 2-bromo-L-phenylalanine, 3-bromo-Lphenylalanine, 4-bromo-L-phenylalanine, Asn, Gln, a modified Phe, a hydratable carbonyl-containing residue, or a boronic acid-containing residue;
X4 is Asp or Asn;
X5 is Leu; β,β'-diphenyl-Ala; Phe; a non-natural analog of phenylalanine, tryptophan, or tyrosine; a hydratable carbonyl-containing residue; or a boronic acid-containing residue;
X6 is Ser or Cys;
X7 is Thr, Ser or Cys;
X8 is Arg, a modified Arg, or a hydratable carbonyl-containing residue or boronic acid-containing residue;
X9 is Arg or Ala;
X10 is Leu; Gln; Glu; β,β'-diphenyl-Ala; Phe; a non-natural analog of phenylalanine, tryptophan, or tyrosine; a hydratable carbonyl-containing residue; or a boronic acid-containing residue;
X11 is Lys; and
X12 is Cys, Gly, 7-aminoheptanoic acid, β- alanine, diaminopropionic acid, propargylglycine, isoaspartic acid, or null.

In some embodiments, the multivalent meditope includes the amino acid sequence of SEQ ID NO: 1, 2, 15, 16, 17, 18, 19, 20, 21, 22, 23, 24, 25, 26, 27, 28, 29, 30, 31, 32, 34, 35, 36, 37, 38, 39, 40, 41, 42, 43, 44, 45, 46, 47, 48, 49, 50, 51, 52, 53, 54, 55, 186, 187, 188, 189, or 207, or a cyclic peptide derived therefrom.

In some embodiments, a multivalent meditope includes a first meditope and a second meditope coupled to a first linker, wherein the first linker comprises a PASylation sequence, a sortase sequence, an Ssp Ic intein sequence, an Ssp IN intein sequence, and/or an aldehyde tag. In some embodiments, the multivalent meditope includes a second linker.

In some embodiments, the first linker includes an Ssp Ic intein sequence and an Ssp $I_N$ intein sequence. In some embodiments, the first linker includes an Ssp $I_c$ intein sequence and the second linker includes an Ssp $I_N$ intein sequence. In some embodiments, the sortase sequence includes the sequence LPXTG (SEQ ID NO: 249), wherein X is any amino acid. In some embodiments, the first linker and/or second linker further includes a glycine residue. In some embodiments, the aldehyde tag includes the sequence of SEQ ID NO: 247. In some embodiments, the first and/or second linker further includes a lysine, a tyrosine, a glutamic acid, an aspartic acid and/or an unnatural amino acid residue. In some embodiments, the first and/or second linker is conjugated to a therapeutic or diagnostic agent.

In some embodiments, a complex includes a first and second meditope enabled antibodies or antigen binding fragments thereof bound to a cell and to a multivalent meditope, wherein said first and second meditope enabled antibodies or antigen binding fragments thereof each includes a meditope binding site, and wherein the multivalent meditope comprises a first and second meditope, wherein the first meditope of the multivalent meditope is bound to the meditope binding site of the first meditope enabled antibody and the second meditope of the multivalent meditope is bound to the meditope binding site of the second meditope enabled antibody.

## BRIEF DESCRIPTION OF THE DRAWINGS

Figure 1 illustrates a mechanism of action in one embodiment for enhancing tumor therapy. In the exemplified embodiment, bivalent antibodies bound to an epitope on an antigen (e.g., ErbB receptor family) overexpressed on tumor cells (left panel) and blocks receptor signaling, alters endocytosis and receptor recycling and/or elicits an immune response. The addition of an antibody such as matuzumab which recognizes a different domain of EGFR in combination with cetuximab can, in some cases be more effective due to daisy chaining of the surface receptor (right panel 1). A multivalent meditope (in this particular example, trivalent) tethers/cross-links the therapeutic mAb and can enhance its therapeutic potential (right). In addition, the multivalent meditope (shown here as a trivalent version) can also carry an imaging agent, which can allow for both enhanced therapeutics as well as imaging.

Figure 2 shows an exemplary bivalent meditope. The meditope is directly fused to the N-terminus of a peptide linker that is directly fused to the Fc Region of an IgG. In this example, as the Fc is naturally homodimerized during expression, the end product from the meditope-Fc construct is bivalent.

Figure 3 is a depiction of an alternative meditope-Fc linker, a coiled coil, which may be used in accordance with certain embodiments.

Figure 4 shows SPR-determined binding kinetics for monovalent (left panel) and tetravalent (right panel) meditopes to cetuximab. The panel above the surface plasmon resonance traces depicts a cartoon of the monovalent meditopes being passed over the bivalent IgG. As shown in the right panel, avidin was saturated with biotinylated meditope and passed over the same immobilized cetuximab IgG. The off-rate of the multivalent meditope was reduced by at least 36 fold. (Note the time scales between the two experiments are different.)

Figure 5 illustrates the synthesis of dimeric and trimeric meditopes according to some embodiments, for meditopes containing lactam linkages. Sequence legend: (GQFDLSTRRLKG) SEQ ID NO: 173; (GKLRRTSLDFQG) SEQ ID NO: 174.

Figure 6 shows a modified Arg8 residue that may be used to optimize meditope binding parameters, and may be used in accordance with the embodiments described herein (R = alkyl, substituted alkyl, aromatic or NHR‴, where R‴ = alkyl, substituted alkyl, or aromatic).

Figure 7 shows the chemical structure of a meditope according to some embodiments. The circles indicate positions that can be modified, e.g., to improve meditope affinity for the Fab. The boxes indicate cyclization strategies. 'Click' chemistry and olefin metathesis, top and bottom far right boxes, respectively, are additional routes to cyclize the

meditope.

Figure 8 shows meditope peptides binding to framework loops of cetuximab. Figure 8A: The complex of cetuximab Fab (light chain is denoted by $V_L$ and $C_L$; heavy chain is denoted by $V_H$ and $C_H$) and cyclic CQFDLSTRRLKC (depicted within the shaded area and labeled with the word "meditope") (SEQ ID NO: 1) indicates that the meditope binds to an interface of the Fab framework, which is distinct from the CDR loops of cetuximab. Figure 8B (top) shows the stick representation of the cQFD meditope and (bottom) the stick representation of the cQYN meditope. The N- and C- terminal cysteines are solvent exposed and display high thermal factors.

Figure 9 illustrates that the cQFD and cQYN meditopes do not bind to the CDRs of cetuximab, as was previously hypothesized. Figure 9A shows an overlay of two crystal structures, one of a cetuximab Fab and its eptiope, EGFR domain III, the other showing the cQFD meditope and cetuximab Fab, in which the cQFD meditope binds to the central cavity of the cetuximab Fab. The antigen, EGFR domain III, binds at the complementarity determining regions at a significant distance from the meditope binding site. Figure 9B shows SDS-PAGE results on the left-hand side and the corresponding size exclusion chromatography results on the right-hand side. Size exclusion experiments of the individual components, Fab, EGFR domain III, and SMT-cQFD meditope, as well as an admixture of all three, indicate the formation of a hetero-trimeric complex that coeluted. The non-reducing SDS-PAGE gel shows the fraction that eluted first, indicating the presence of all three components within the new peak (the left-most peak for "complex," shaded light gray) observed from the admixture. Figure 9C shows the results of a FACS analysis, indicating that the cQFD meditope bound to EGFR positive MD-MBA-468 cells only in the presence of cetuximab (arrows). The meditope alone or the meditope in the presence of M425, a murine EGFR antibody, did not bind. Figure 9D shows results of surface plasmon resonance experiments using a sensor chip coupled with a cetuximab scFv or Fab. The experiments indicate that saturation of the scFv could not be achieved at concentrations as high as 100 $\mu$M of the cQFD meditope. The same experiments using the cetuximab Fab coupled sensor chip indicate full saturation. The dissociation constant from this experiment is 660 nM. Control SPR experiments (right panel) show that the cetuximab scFv readily binds to the soluble EGFR domain III fragment, indicating that the CDR loops were functional.

Figure 10 shows an scFv-meditope linker. scFvs are created by fusing the light chain variable domain to the heavy chain variable domain (or *vice versa*) through a 12-20 amino acid linker (typically $\{GGGS\}_{3-5}$). In this example, a portion of the flexible linker sequence can be replaced with the meditope sequence.

Figure 11 shows results of FACS analysis following preincubation of Cetuximab (C) or M425 with MDA-MB-468 cells. 3 $\mu$M of monomeric meditope (cQFD) were added, and were either washed or not washed before proceeding to FACS analysis.

Figure 12 shows results of a study showing meditope-enabling mutations did not interfere with antigen recognition. Commercial trastuzumab (Parental), trastuzumab produced from NS0 cells (NS0-Parental) and memAb trastuzumab (memAb) were labeled with Alexa Fluor 488 and applied to SKBR3 cells (top panel). Alexa Fluor 647-meditope-Fc (MFC) were applied after excess mAbs were washed off. The three mAbs bound to cells in the same range, but only memAb labeled cells have enhanced MFC binding.

Figure 13 shows a bar graph of HER2 distribution on MDA-MB-468 cells after 24 hours in the absence of antibody (MDA-MB-468 steady state), in the presence of trastuzumab (not meditope-enabled)(MDA-MB-468 10nM T), in the presence of memAb trastuzumab and a tetravalent meditope (M4Fc) for 3 minutes ( MDA-MB-468 10nM meTv2 10nM M4Fc 3min), and in the presence of memAb trastuzumab and a tetravalent meditope (M4Fc) for 10 minutes (MDA-MB-468 10nM meTv2 10nM M4Fc 10min).

Figure 14 shows SPR sensograms of the interaction of MPL-zHER2 with antibody. Figure 14A shows an SPR sensogram showing the interaction of MPL-zHER2 with immobilized meditope enabled trastuzumab IgG. Figure 14B shows an SPR sensogram showing the binding interaction of MPL-zHER2 with immobilized HER2.

Figure 15 shows the interaction of zIGF1R-MPL with memAb trastuzumab. Figure 15A shows an SPR sensogram showing the interaction of zIGF1R-MPL with immobilized memAb trastuzumab. Figure 15B shows analytical size exclusion chromatography traces showing the interaction of increasing ratios of IGF1R-MPL with memAb trastuzumab IgG.

Figure 16 shows gel filtration chromatograms of multivalent meditopes. Figure 16A depicts representative gel filtration

column chromatogram (HiLoad prep75 26/600 PG) of trivalent meditope. Figure 16B depicts representative gel filtration column chromatogram (HiLoad prep75 26/600 PG) of bivalent meditope.

Figure 17 shows multivalent meditope binding to meditope-enabled trastuzumab. Figure 17A shows BVM fractions 26-29 binding to meditope-enabled trastuzumab. Figure 17B shows binding analysis of SEC fractions from TVM purification to memAb trastuzumab.

Figure 18 shows analysis of purified trivalent meditope. Figure 18A shows analysis of purified trivalent meditope by HPLC. Figure 18B shows analysis of purified trivalent meditope by mass spectroscopy.

Figure 19 shows binding analysis of multivalent meditopes to meditope enabled antibodies at low (A), medium (B), and high (C) concentrations.

Figure 20 shows data fit quality to a 1:1 multivalent meditope:meditope-enabled antibody binding model using BiaEvaluation software for low (top panel), medium (middle panel), and high (bottom panel) concentrations of meditope enabled antibodies.

## DETAILED DESCRIPTION

Provided herein are complexes comprising a plurality of multivalent meditopes bound to a plurality of meditope enabled antibodies or antigen binding fragments thereof. The formation of these complexes alters the distribution of a cell surface antigen to which the plurality of antibodies or antigen binding fragments thereof bind. Complex formation through the interaction of the multivalent meditopes and meditope enabled antibodies, and its cognate epitope on the target antigen, can increase or promote clustering or co-localization of a cell surface antigen on a cell, which can increase or promote internalization of the complex. In turn, the increased internalization can be used to clear or reduce the concentration of a cell surface antigen from the surface of a target cell and to increase the rate of internalization of the meditope enabled antibody-meditope complex into the target cell. Increasing internalization rates can increase the efficacy of certain therapies, or reduce the amount of drug necessary to achieve a particular therapeutic effect. Methods of increasing or promoting clustering of a cell surface antigen can comprise administering to the cell one or more meditope-enabled antibodies that specifically binds to a cell-surface antigen and administering one or more multivalent meditopes, thereby promoting clustering of the cell-surface antigen on the surface of the target cell. In certain embodiments, the multivalent meditopes are conjugated to one or more agents.

### A. Multivalent Meditopes to Modify Cell Surface Antigen Display

Multivalent meditopes can be used with meditope enabled antibodies to target cell surface antigens and produce changes in their distribution on the surface of host cells. For example, the display of cell surface antigens, such as tumor associated antigens, receptors such as GPCRs, receptor tyrosine kinase, other signaling molecules such as CTLA4, and other epitopes present on cell surface proteins, can be manipulated and/or reorganized through the use of multivalent meditopes and meditope enabled antibodies that recognize epitopes on those antigens. Reorganization of the receptors and signaling molecules can have a profound effect on signaling pathways and cellular responses.

In vivo, this type of reorganization occurs through cell-cell contact, where ligands from one cell engage receptors on the other cell, effectively creating a multivalent interaction. Through this point of contact, the receptor/ligands are localized or brought into proximity to one another, creating a signaling "synapse". The signaling synapse initiates post-translational modifications within the cell, and can, for example, alter actin and microtubule organization, ultimately leading to specific transcriptional events.

The use of multivalent meditopes can be used in a similar fashion to alter the distribution of cell surface antigens. Specifically, one or more meditope-enabled antibodies can be administered and permitted to bind to a targeted antigen on the surface of a cell in conjunction with one or more multivalent meditopes. In one embodiment, the multivalent meditope permits the formation of a complex of linked meditope enabled antibodies bound to their cognate antigens. The meditope enabled antibodies associate with the multivalent meditopes, drawing or crosslinking the surface antigens toward one another, effectively altering the display of those antigens on the surface of the cell.

This effect is illustrated by the use of a bivalent meditope with a meditope enabled antibody. This combination allows for one Fab arm of a first meditope enabled antibody to engage with a Fab arm of a second meditope enabled antibody. In this scenario, one would expect "linear" arrangement of meditope enabled antibody-bound cell surface antigens and bivalent meditopes. It is possible to use a trivalent, tetravalent, or higher valence meditope and cluster additional meditope enabled antibody-associated, surface derived antigens. The multivalent meditope can be tailored to precisely control the geometry of the clustering. For instance, it may be advantageous to bring two receptors in close

proximity, potentially leading to higher activity of the intracellular signaling molecules. Here, the linker between two or more meditopes can be approximately 1 to 10 Å (for example) through a PEG linker, a peptide linker or DNA - e.g. DNA origami. Alternatively, it may be advantageous to keep receptors at a distance. Here, rigid linkers between the meditopes can be used to "push" apart specific receptors. These rigid linkers could be protein domains, such as entire IgGs where the meditope is fused to the N- and/or C-termini of the light and/or heavy chains. Alternatively, DNA can be used to directly control the distance of each meditope. Moreover, recent advances in DNA origami provide additional examples where the positioning of each meditope can be tightly controlled.

In a different embodiment, it is possible to use a meditope enabled antibody to a non-internalizing antigen and a second meditope enabled antibody to an antigen involved in signaling. In this case a multivalent meditope enabled antibody could be used to sequester the signaling receptor from regions of the cell membrane that are active in signaling. Alternative, it may be advantageous to ensure a signaling event remains active. Multivalent meditopes, flexible or rigid, and with defined lengths can be used to regulate the pair.

In a different embodiment, the meditope enabled antibodies and multivalent meditopes can be used mark a specific cell for an immune response. In this case, clustering of the meditope enabled antibody-associated receptors through a multivalent meditope would present a "high" concentration of Fc domain for the FcR on T cells. Multiple investigations have demonstrated that the Fc domain can be specifically tuned through a limited number of point mutations to elicit the appropriate response.

In another embodiment, the efficacy of antibody treatments can be altered and improved by manipulating the binding affinity of the multivalent meditope for the meditope enabled antibody, by selecting a meditope enabled antibody with a preferred binding affinity for its cognate epitope on a target antigen, or by manipulating both features to optimize the lifetime as well as the specificity of targeting of the meditope enabled antibody-multivalent meditope complex. Specifically, the lifetime of an interaction is related to the kinetic off-rate. In some instances, it can be envisioned that the longest possible lifetime would be advantageous. In other cases, a moderate lifetime (tau = 10 ms to 10 minutes) would be advantageous. In the latter case, there are specific receptors (e.g., EGFR or Her2) that are overexpressed on tumor tissues, but also expressed on certain healthy tissues, albeit at a lower level (10, 100 or 1000 fold). Clinically, it may be desirable to bind to the tumor and not bind to the healthy tissue. Using a combination of meditope enabled antibodies and multivalent meditopes with a moderate life-time enhances the selectivity of the multivalent meditope. Specifically, at low concentrations of antigen as found in a normal cell, a meditope enabled antibody could bind to the antigen and even a multivalent meditope could interact. However, the likelihood of a second meditope enabled antibody engaged antigen is low, allowing the multivalent meditope to dissociate. In the disease state, the likelihood of an adjacent meditope enabled antibody engaged antigen is higher, allowing each arm to bridge two or more meditope enabled antibody-associated antigens.

In other embodiments, the provided methods utilize properties of cells and alter the internalization and sorting pathway of conjugated biologics to efficiently release an agent associated with the multivalent meditope. Studies have demonstrated that the size of a particle localized to the cell membrane is important for mechanism of internalization (see Biochem J. 2004 Jan 1;377(Pt 1):159-69; J Nanobiotechnology. 2014 Feb 3;12:5. doi: 10.1186/1477-3155-12-5), suggesting the size of an associated ligand may effect the internalization pathway. The use of multivalent meditopes with meditope enabled antibodies would create the appearance of a ligand with a larger particle size as compared to a single, antibody bound to a cell surface antigen.

An alternative approach for promoting or altering internalization rates is by promoting or controlling the aggregation of cell bound receptors, as in certain embodiments provided herein. In some embodiments herein, co-localization, clustering, aggregation, internalization, and/or intracellular trafficking or sorting is effected, recapitulated, or enhanced or altered using multivalent meditopes in connection with meditope-enabled antibodies. Observed herein are differences in clustering and/or internalization of such surface receptors following administration to cells of meditope-enabled antibodies recognizing such receptor antigens and multivalent meditopes, in comparison to administration of antibodies alone. Among the provided embodiments are multivalent meditopes that promote co-localization and clustering of surface antigens recognized by meditope enabled antibodies, and/or internalization/trafficking/sorting of such antigens, such as cell-surface antigens such as receptors. Cell surface proteins on a host cell forced to internalize would be less available for interaction with their cognate ligands, which could impact the ability of the host cell to respond to that ligand.

Also provided are methods and uses of such multivalent meditopes in delivering meditopes and/or coupled agents to cells, for example, in therapeutic and imaging methods. Exemplary methods are those effecting delivery of a cytotoxin or radiolabeled agent coupled to the multivalent meditope. Some potentially useful antibodies that recognize specific antigens abundant on the surface of target cells are "poorly internalizable" (Matzku et al. Int. J. Cancer 1988 2:11-14; Reilly et al. Clin. Pharmacokinet. 1995 28:126-142). Poor or suboptimal internalization diminishes intracellular delivery and accumulation in target organs or tissues (Matzku et al. Int. J. Cancer 1988 2:11-14; Reilly et al. Clin. Pharmacokinet. 1995 28:126-142). Therefore, these "poorly internalizable" antibodies are not optimized for intracellular targeting. Accordingly, there is a need for antibody systems that enhance antibody targeting and internalization by cells. In some embodiments, the methods provide an increased degree and/or speed of internalization of a cell-surface antigen.

Increased or more rapid internalization can be advantageous, for example, in the context of uses in which the antibody is used to deliver an agent to a cell, such as an ADC. It can also be advantageous when an antibody is delivered to promote downregulation of a surface receptor or other surface antigen associated with or causative in the progression of disease. It can also be advantageous, for example, by reducing the amount of a receptor or other surface antigen on a cell's surface, for example, by targeting it for degradation. Some such receptors, including co-receptors, are capable of effecting or mediating cell signaling. Therefore, the methods described herein can reduce, interfere with, or eliminate harmful cell signaling pathways in some embodiments. In some embodiments, the compositions and methods described herein can increase antigen internalization by 1.5-, 2-, 3-, 4-, 5-, 6-,7-, 8-, 9-, 10-, 20-, 30-, 40-, 50-, 75-, 100-, 200-, 300-, 400-, 500-fold or more. In some embodiments, the compositions and methods described herein can reduce the amount of antigen present on a cell's surface by 1.5-, 2-, 3-, 4-, 5-, 6-,7-, 8-, 9-, 10-, 20-, 30-, 40-, 50-, 75-, 100-, 200-, 300-, 400-, 500-fold or more. In some embodiments, the compositions and methods described herein can increase antibody internalization by 1.5-, 2-, 3-, 4-, 5-, 6-,7-, 8-, 9-, 10-, 20-, 30-, 40-, 50-, 75-, 100-, 200-, 300-, 400-, 500-fold or more, including the internalization of antibodies with low internalization rates.

Some antibodies exert a therapeutic effect after entering a cell. In some embodiments, increased or more rapid internalization can increase an antibody's potency or efficacy. Therefore, in some embodiments, lower dosages can be used to achieve the same therapeutic effect. In some embodiments, administration of a meditope-enabled antibody contacted with a multivalent meditope can achieve the same therapeutic effect as administration of antibody alone using 1.5-, 2-, 3-, 4-, 5-, 6-,7-, 8-, 9-, 10-, 20-, 30-, 40-, 50-, 75-, 100-, 200-, 300-, 400-, or 500- fold less drug.

In some embodiments, increased or more rapid internalization results in increased or more rapid delivery of an antibody to endosomes. Some antibodies comprise linkers comprising a proteolytic cleavage site. The linkers can be conjugated to agents, including imaging or therapeutic agents, which are then released by the antibody upon internalization. In some embodiments, linker cleavage occurs in endosomes or lysosomes. Therefore, increased or more rapid delivery of an antibody to endosomes or lysosomes can increase an antibody's potency or efficacy.

A slower degree or less rapid internalization rate may be desirable in some contexts. In some embodiments, increasing co-localization or clustering of cell surface antigen may promote desirable effector functions, such as antibody-dependent cell-mediated cytotoxity (ADCC) and/or complement-mediated lysis, and/or to engage and promote signaling via the cell-surface receptor antigens.

Provided are methods for delivering meditopes and/or for enhancing or increasing co-localization or clustering of cell-surface antigens on target cells. In some embodiments, the methods are carried out by administering to the cells one or more meditope-enabled antibody that specifically binds to the cell surface antigen(s) and a multivalent meditope. In some embodiments, co-localization, clustering, and/or aggregation of the antigen is enhanced or increased. In some aspects, the increase is an increase in degree, speed, and/or duration in comparison to that observed prior to or in the absence of the addition of the meditope-enabled antibody and/or the multivalent meditope, or in the presence of the antibody without the meditope. In some embodiments, the method promotes or enhances the degree of a signal via the cell surface antigen and/or an effector function mediated via the bound antibody.

Provided are methods for delivering meditopes, agents coupled thereto, and/or for enhancing or increasing internalization and/or degradation of cell-surface antigens on target cells. In some embodiments, the methods are carried out by administering to the cells one or more meditope-enabled antibody that specifically binds to the cell surface antigen(s) and a multivalent meditope. In some embodiments, internalization and/or degradation of the antigen is promoted, enhanced or increased. In some aspects, the increase in internalization is an increase in degree, speed, and/or duration in comparison to that observed prior to or in the absence of the addition of the meditope-enabled antibody and/or the multivalent meditope, or in the presence of the antibody without the meditope. In some embodiments, the meditope is coupled to a therapeutic or diagnostic agent, such as a chemotherapeutic, cytotoxin, or radiolabeled agent, such as for use in ADC or radioimmunotherapy (RIT). In some embodiments, the methods alter internalization or intracellular trafficking. In some embodiments, the methods alter intercellular or intracellular signaling.

In some embodiments, the methods described herein are useful to enhance delivery of an agent, such as in ADC or RIT or other methods for antibody-mediated delivery to a cell or tissue expressing an antigen targeted by the antibody. In some embodiments, the methods are advantageous in that they promote clustering and therefore internalization of antigens, antibodies bound to the antigens, and agents associated with the antibodies, in the context of non-internalizing antigens, such as carcinoembryonic antigen (CEA), tumor-associated glycoprotein (TAG-72), KS1/4 (*see* Schmitd et al., Cancer Immunol Immunother. Dec 2008; 57(12): 1879-1890; Myer et al. Cancer Res 1993;53:3956-3963).

Despite the successful development of TDM1, an anti-Her2 ADC that is marketed as KADCYLA®, only a small fraction of the conjugated drug (~2%) is delivered to the tumor. The precise reason for such a low delivery yield is not clear, however, it likely stems from the different internalization routes utilized by the cell (e.g., clathrin-mediated endocytosis and/or caveolae-mediated endocytosis and/or macropinocytosis) as well as routing of the endocytosed material once inside the cell (recycling endosomes or late endosomes or lysosomes). Drugs conjugated to the antibody through a disulfide linker may be released due to the reducing environment of the cellular cytoplasm. Alternatively, some may be released by specific proteases active in endosomal vesicle (e.g., auristatin). Other studies using different linkers

indicate that the lysosome is an effective means in releasing the drug, independent of conjugation chemistry. Yet disulfides can be reduced in serum and proteases can be active in healthy tissue. In some embodiments, the provided methods, antibodies, and meditopes, provide improvements such as more specific and effective delivery of agents, such as toxins to treat disease.

In tumor immunotherapy, many antigens are merely tumor-associated or expressed at higher levels on tumor cells/environments, as opposed to being tumor-specific. For example, while Her2 and EGFR are significantly overexpressed in tumor tissues such as Her2+ breast cancer and colorectal cancers, they are also expressed in healthy tissues, which can result in on-target, off-tissue toxicities. Other types of antigens (such as tumor-specific antigens and oncofetal antigens, e.g., carcinoembryonic antigen (CEA)) are expressed solely in tumor tissues and/or otherwise minimally or at only certain stages of development. Combinations of monoclonal antibodies (mAbs) that recognize unique epitopes on the same antigen can produce synergistic effects, including enhancement of various antibody effector functions (e.g., ADCC, complement-dependent lysis, signaling inhibition), enhancement of cell death, and in the case of cancer-targeting antibodies, enhancement of tumor growth inhibition. See, for example, Dechant M et al., "Complement-dependent tumor cell lysis triggered by combinations of epidermal growth factor receptor antibodies," Cancer Res, 2008 Jul 1;68(13):4998-5003; Scheuer W et al., "Strongly enhanced antitumor activity of trastuzumab and pertuzumab combination treatment on HER2-positive human xenograft tumor models," Cancer Res, 2009 Dec 15;69(24):9330-6; Cardarelli PM et al., "Binding to CD20 by anti-B1 antibody or F(ab')(2) is sufficient for induction of apoptosis in B-cell lines," Cancer Immunol Immunother, 2002 Mar;51(1):15-24. Epub 2001 Dec 18. While the precise mechanism of this enhanced cell death remains debated, studies indicate that both mAbs should be multivalent (e.g., full antibodies or $F(ab)'_2$ as opposed to monovalent Fabs to achieve enhanced effects, suggesting that the second bivalent mAb (which binds to a unique epitope on the same antigen) can cluster cell surface antigens and act more efficiently, e.g., more efficiently kill tumor cells. See Figures 1 and 2.

In some aspects, the multivalent meditope is used instead of a second antibody or bispecific antibody, e.g., to achieve synergy in combination with a meditope-enabled antibody. In some aspects, the multivalent meditopes provide advantages compared to use of a second antibody recognizing a separate epitope on the same antigen. For example, production of the multivalent meditopes can be more efficient, and cost-effective when compared to a second antibody. For example, although a number of preclinical/clinical trials are investigating the co-administration of two monoclonal antibodies, the costs of producing and marketing such a therapeutic is likely to be prohibitive. In some aspects, the multivalent meditopes also are comparatively more easily targeted to disease sites, such as tumors. Given the nature of the meditope binding site (within the meditope binding site, separate from the antibody CDRs), and the broadly applicable methods provided herein for meditope-enabling any antibody of choice, the multivalent meditopes also have the advantage of being readily applicable to a large range of therapeutic antibodies, without the need to identify a second antibody or epitope with therapeutically acceptable characteristics. Thus, in some aspects, use of a multivalent meditope avoids the need to identify a second mAb with acceptable characteristics, and the associated and significant cost of its development thereof.

In some embodiments, the multivalent meditopes provide advantages over and/or improvements in bivalent antibodies. In some embodiments, the multivalent meditope is used with a bispecific meditope-enabled antibody, e.g., to enhance aggregation of two antigens, e.g., cell-surface antigens, bound by the different arms of the bispecific antibody, for example, to enhance affinity, signaling induced by the antibody, effector functions, and/or internalization, as compared to use of the bispecific antibody alone. Thus, in some aspects, the multivalent meditope is used in connection with a bispecific meditope-enabled antibody and provides an increased level of efficacy, for example, by causing an additional degree of clustering as compared to the bispecific antibody alone.

In some embodiments, two meditope-enabled antibodies, each of which specifically binds to a different antigen expressed on the surface of the same cell, together with a multivalent meditope, are used in place of a bivalent antibody, providing enhanced antigen (e.g., receptor) aggregation, and/or a certain level aggregation regardless of the ratio of the density or presence of the two antigens relative to one another on the cell surface. In some aspects, such embodiments enhance cell-surface clustering of antigens and/or co-localization. In one embodiment, a multivalent meditope is used to achieve such clustering together with multiple meditope-enabled antibodies recognizing multiple different antigens. Thus, in some embodiments, as compared to a bispecific antibody, in which clustering would occur only where each arm of the antibody bound its respective antigen, clustering in such an embodiment may be achieved even where the antigen bound by one of the antibodies is present at a much higher density on the cell surface compared to the antigen bound by another.

Similarly, with antibodies conjugated to various agents, such as imaging agents, the ability to cluster or co-localize cell and/or internalize surface antigens can be important. For example, drug-conjugated biologics such as antibody-drug conjugates (ADCs) are generally designed with a goal of selectively delivering an agent, e.g., an active drug, within the targeted tissue, often within a cell of the targeted tissue or targeted cell, such as a tumor cell. The targeting biologic, e.g., the antibody, is generally designed to preferentially bind to the cell of interest; selective binding to antigens present only on the target cells or tissues and/or present at very low amounts or infrequently on other tissues,

can be especially desirable. Particularly for drug-conjugated antibodies directly targeting diseased cells, e.g., tumor cells, it can be desirable or important that the agent-conjugated antibody is internalized within the target cell following binding to the target antigen on the cell surface. *See*, *e.g*., Trail et al., Antibodies 2013, 2, 113-129; Perez et al., H.L.. Perez, et al., "Antibody-drug conjugates: current status and future directions," Drug Discov Today (2013). In some contexts, antibodies that result in internalization of antigens are also desirable in the context of radioimmunotherapy (RIT). *See* Boudousq V et al. (2013) "Comparison between Internalizing Anti-HER2 mAbs and Non-Internalizing Anti-CEA mAbs in Alpha-Radioimmunotherapy of Small Volume Peritoneal Carcinomatosis Using 212Pb" PLoS ONE 8(7): e69613. In general, the conjugated agent, e.g., drug, is released and is active within the target cell. In some contexts, for example in targeting of certain extracellular matrix components and/or antigens specific to the tumor environment, internalization may not be necessary. *See* Ackerman et al. Cancer Immunol Immunother. Jul 2008; 57(7): 1017-1027.

In some embodiments, the non-internalizing antigen is one in which the binding of a monospecific bivalent antibody in the absence of meditope results in no internalization of the antigen, a degree of internalization that is no more than that resulting from metabolic turnover or in the absence of an antibody specific for the antigen, or that is not greater than 2-fold greater than that resulting from metabolic turnover or in the absence of an antigen-specific antibody. In some embodiments, the antigen following incubation with a monospecific bivalent antibody does not internalize at a rate that is 3-, 4-, 5-, 6-,7-, 8-, or 9-fold greater than that resulting from metabolic turnover or in the absence of an antigen-specific antibody.

In some embodiments, the non-internalizing antigen is one in which the binding of a natural binding partner or ligand for the cell-surface antigen alone results in no internalization of the antigen, or a degree of internalization that is no more than that resulting from metabolic turnover or in the absence of the binding partner or ligand, or that is not greater than 2-fold greater than that resulting from metabolic turnover or in the absence of ligand/binding partner In some embodiments, the antigen following incubation of cells with a natural ligand or binding partner for the antigen does not internalize at a rate that is 3-, 4-, 5-, 6-,7-, 8-, or 9-fold greater than that resulting from metabolic turnover or in the absence of the ligand or binding partner. In some embodiments, the antigen is internalized following binding of a monospecific bivalent antibody or natural ligand or binding partner at a half-time that is at least 10, 11, 12, 13, 14, 15, or 16 hours or more.

**B. Multivalent Meditopes**

Provided herein are multivalent meditopes, for use with the disclosed methods of altering the distribution of cell surface antigens, as well as vectors, cells, libraries, and other systems containing the same.

The term "multivalent meditope," as used herein, refers to a plurality of meditopes, and one or more linkers or linking means. Such multivalent meditopes may comprise any meditopes, including but not limited to the meditopes described herein. In one aspect, a multivalent meditope may comprise 2, 3, 4, 5, 6, 7, 8, 9, 10, or more meditopes. The meditopes comprising the multivalent meditope may have the same or different structure or sequence.

The term "meditope," as used herein, refers to a peptide or peptides that binds to a central cavity such as a meditope-binding site of a meditope-enabled antibody or antigen binding fragment thereof, which antibody or antigen binding fragment thereof has a threonine at position 40, an asparagine at position 41, and an aspartate at position 85 of its light chain, according to Kabat numbering, or contains a meditope binding site containing residues that correspond to those within the meditope-binding site of cetuximab, meditope-enabled trastuzumab, or meditope-enabled M5A, disclosed herein. Exemplary meditopes include, but are not limited to, the cQFD (SEQ ID NO: 1) and cQYN (SEQ ID NO: 2) peptides and variants thereof ("meditope variants" or "variant meditopes"). Other molecules may also bind to meditope binding sites of meditope-enabled antibodies, with functional characteristics similar to those of a meditope. Such molecules, defined as "meditope analogs," may include, but are not limited to, small molecules, aptamers, nucleic acid molecules, peptibodies and any other substance able to bind to the same meditope binding site as a meditope. In some embodiments, the meditope sequence is cyclized as disclosed herein (e.g. the terminal (or near terminal) cysteine residues form a disulfide bond).

The term "linker," as used herein refers to the means by which the plurality of meditopes are linked or connected. To illustrate one embodiment, in the case of a multivalent meditope comprising two or more meditopes, the meditopes are linked by one or more linkers. In one aspect, a multivalent meditope comprising two meditopes could be cyclized by using three liners.

The one or more linkers or linking means for linking the meditopes to form the multivalent meditope may be any structure suitable to link and separate the individual meditopes. Exemplary linkers include the use of one or more amino acids which may be used to form a peptide, in some embodiments having a modified peptide backbone, a small chemical scaffold, a biotin-streptavidin, an organic or inorganic nanoparticle, a polynucleotide sequence, peptide-nucleic acids, an organic polymer, or an immunoglobulin Fc domain. The means for linking can comprise covalent and/or noncovalent bonds.

The one or more linkers can include various sequences or other structural features that provide various functions

or properties. For example, the one or more linker can contain structural elements to allow the multivalent meditope to be derivatized. Exemplary structural elements include the presence or absence of a protease cleavage site, an immunogenic sequence, sequences that cause or aid in the circularization of a multivalent meditope, including, for example, PASylation and intein sequences, and residues, sites or tags including, for example aldehyde tags compatible with formylglycine-generating enzyme (FGE), lysines, tyrosines, glutamic acids, aspartic acids and unnatural amino acids, which can be used, for example in the conjugation of therapeutic or diagnostic agents.

In nature, specificity and affinity are often achieved through multivalency. For a bivalent ligand with a linker, this can be expressed as $\Delta G_{Total} = \Delta G1 + \Delta G2 - \Delta Glinker$. In other words, $K_{Total} = K_1 * K_2 / K_{linker}$. In the case where the linker makes no contribution to the free energy ($K_{linker} \sim 1$), the apparent affinity of the bivalent ligand for the bivalent target is the product of the monomeric binding constants. Thus, significant gains in affinity can be achieved through multivalency in general (e.g., for a meditope with $K_D = 1\ \mu M$, the affinity of a 'theoretical' bivalent meditope is 1 pM). While such large gains are rarely seen in general (primarily due to the geometry of bivalent/trivalent/multivalent receptors), synergy is observed. The geometry of a cell surface-expressed antigen can place strict constraints on the linker of a multivalent meditope, but can also ensure specificity, which can be an important goal in some contexts, such as for targeted delivery, e.g., by minimizing the risk of off-target effects.

In one example, the multivalent meditope contains an Fc region of an antibody or portion thereof, such as substantially all of the Fc region. The use of the Fc region to 'dimerize' ligands is established and described, for example, by Jazayeri JA & Carroll GJ., "Fc-based cytokines: prospects for engineering superior therapeutics," BioDrugs, 22(1):11-26 (2008) In some examples, to generate a bivalent meditope, the meditope is fused to an Fc region (e.g., to the N-terminus of the Fc region of an IgG) through a linker, typically a peptide linker, e.g., a flexible peptide linker. In one example, the length of the linker is chosen to roughly match the distance between the Fabs of an IgG, such as of 17, 30, 31, 32, 33, 34, 35, 36, 37, 38, 39, 40 amino acids in length. In some aspects, the linker contains glycines, serines, and/or a combination thereof. Exemplary "meditope-Fc" fusions are shown in SEQ ID NO: 3 and 4. See also Figure 2.

In some aspects, the meditope-Fc is or contains a meditope linked to immunoglobulin (Ig) constant region or constant region portion. The Ig constant region generally contains an Fc region or constant region domain (e.g., CH1, CH2, CH3) or functional fragment thereof.

In some embodiments, the meditope, e.g., the multivalent meditope is a compound having one or more chain (e.g., a polypeptide chain) having the formula:

$$R^{3A}\text{-}L^{3A}\text{-}R^{4A} \qquad \text{(Formula I)}$$

where $R^{3A}$ is a meditope (e.g., any of the meditopes provided herein), such as a peptide meditope, $L^{3A}$ is a linker, such as a peptidyl linker, linking $R^{3A}$ and $R^{4A}$, and $R^{4A}$ is an immunoglobulin (Ig) heavy chain constant region portion, comprising an Ig Fc region or functional fragment thereof.

In some aspects, the meditope is a first meditope, the linker is a first linker, and the chain comprises the formula:

$$R^{3A}\text{-}L^{3A}\text{-}R^{4A}\text{-}L^{3B}\text{-}R^{3B} \qquad \text{(Formula III)}$$

Where $L^{3B}$ is a second linker, such as a peptidyl linker, linking $R^{4A}$ and $R^{3B}$; and $R^{3B}$ is a second meditope (e.g., any of the meditopes provided herein). In some contexts, the chain includes the formula:

$$R^{5A}\text{-}R^{3A}\text{-}L^{3A}\text{-}R^{4A}\text{-}R^{5B} \qquad \text{(Formula IV)}$$

or

$$R^{5A}\text{-}R^{3A}\text{-}L^{3A}\text{-}R^{4A}\text{-}L^{3B}\text{-}R^{3B}\text{-}R^{5B} \qquad \text{(Formula V)}$$

where each of $R^{5A}$ and $R^{5A}$ is a tail or leader sequence, or null, e.g., is null or is a sequence comprising between 0 and 50 amino acids in length. The various components may be covalently linked, such as by amide bonds.

In some cases, the linkers of the provided compounds, such as meditope containing compounds including multivalent meditopes, e.g., meditope-Fcs, e.g., $L^{3A}$, $L^{3B}$, or both, is between 25 and 40 amino acids in length, such as at or about 25, 26, 27, 28, 29, 30, 31, 32, 33, 34, 35, 36, 37, 38, 39, or 40, or more, e.g., 41, 42, 43, 44, 45, 46, 47, 48, 49, 50, amino acids in length, and/or is between about 2Å and about 200 Å in length, such as between about 50Å and about 150 Å in length, between about 50Å and about 200 Å in length , between about 50Å and about 300 Å in length between about 100Å and about 140Å in length, such as at or about 130 Å, or at or about 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 150, 160, 170, 180, 190, 200, 210, 220, 230, 240, 250, 260, 270, 280, 290, or 300 Å in length.

In some aspects, the linker is designed to have a length that is the same or approximately the same as the distance between two receptors on a cell surface to be targeted. In some embodiments, the receptors can be the same or different. For example, the two receptors can comprise different amino acid sequences and/or different epitopes. In some contexts, two mAbs that recognize different epitopes on a single antigen act synergistically (see Kamat, V. et al., Cancer Biol Ther 7, 726-733 (2008)). In some examples, bivalent meditopes, such as the meditope-Fc, function as second monoclonal antibody-like molecules, but with the flexibility of being able to pair up with any meditope enabled antibody, hence in some aspects reducing the cost of identifying and developing a second therapeutic monoclonal antibody. In some embodiments, multivalent meditope variants (e.g., generated by altering valency and/or linker geometry) further enhance specificity and synergistic effects. Thus, in some examples, the length of the linker is designed to mimic or approximate the distance between CDRs of an antibody, such as of an IgG, e.g., full-length IgG.

In some aspects, the linkers of the provided compounds, such as meditope containing compounds including multivalent meditopes, e.g., meditope-Fcs, e.g., $L^{3A}$, $L^{3B}$, or both, comprises a proteolytic cleavage site, such as a cleavage site for a protease present in the lysosomal compartment, e.g., MMP cleavage sites, ADAM cleavage sites, and/or cathepsin cleavage sites.

In some aspects, the linkers of the provided compounds, such as meditope containing compounds including multivalent meditopes, e.g., meditope-Fcs, e.g., $L^{3A}$, $L^{3B}$, or both, is an unstructured, disordered, or relatively unstructured or disordered linker. In some aspects, the linker has one or more of the following properties: unstructured conformation, conformational flexibility, enhanced aqueous solubility, high degree of protease resistance, low immunogenicity, low binding to mammalian receptors, a defined degree of charge, and increased hydrodynamic (or Stokes) radii.

In some embodiments, the linker is systematically or randomly designed to prevent or cause steric hindrance of antibody/epitope interactions, antibody/antibody interactions, or antibody/meditope interactions. Example modifications include amino acid substitutions, additions, deletions, or modifications. In some embodiments, steric hindrance can prevent receptors from clustering, signaling, or from undergoing receptor-mediated endocytosis. In some embodiments, preventing steric hindrance can cause receptors to cluster, signal, or undergo receptor-mediated endocytosis.

In some aspects, the linker is designed to behave like a denatured peptide sequence under physiological conditions, such as having a large conformational freedom of the peptide backbone. In some examples, the linkers are essentially devoid of secondary structure, e.g., at physiological conditions or in aqueous solutions.

A variety of methods have been established in the art to discern the presence or absence of secondary and tertiary structures in a given polypeptide. In particular, secondary structure can be measured spectrophotometrically, e.g., by circular dichroism spectroscopy in the "far-UV" spectral region (190-250 nm). Secondary structure elements, such as alpha-helix and beta-sheet, each give rise to a characteristic shape and magnitude of CD spectra. Secondary structure can also be predicted for a polypeptide sequence via certain computer programs or algorithms, such as the well-known Chou-Fasman algorithm (Chou, P. Y., et al. (1974) Biochemistry, 13:222-45) and the Garnier-Osguthorpe-Robson ("GOR") algorithm (Garnier J, Gibrat J F, Robson B. (1996), GOR method for predicting protein secondary structure from amino acid sequence. Methods Enzymol 266:540-553), as described in US Patent Application Publication No. 20030228309A1. For a given sequence, the algorithms can predict whether there exists some or no secondary structure at all, expressed as the total and/or percentage of residues of the sequence that form, for example, alpha-helices or beta-sheets or the percentage of residues of the sequence predicted to result in random coil formation (which lacks secondary structure).

In one embodiment, the linker has an alpha-helix percentage ranging from 0% to less than about 5% as determined by the Chou-Fasman algorithm. In another embodiment, the linker has a beta-sheet percentage ranging from 0% to less than about 5% as determined by the Chou-Fasman algorithm. In some embodiments, the linker has an alpha-helix percentage ranging from 0% to less than about 5% and a beta-sheet percentage ranging from 0% to less than about 5% as determined by the Chou-Fasman algorithm. In some embodiments, the linker has an alpha helix percentage less than about 2% and a beta-sheet percentage less than about 2%, has a high degree of random coil percentage, as determined by the GOR algorithm, such as at least about 80%, at least about 90%, at least about 91%, at least about 92%, at least about 93%, at least about 94%, at least about 95%, at least about 96%, at least about 97%, at least about 98%, or at least about 99% random coil, as determined by the GOR algorithm. For example, in some aspects, the linker lacks or substantially lacks tertiary structure in aqueous solution or in physiological solution. In some aspects, it has greater than 90%, 85 %, 80 %, 75 %, or 70 %, random coil formation as determined by the GOR algorithm, and/or less than 5, 4, 3, 2, or 1 %, or 0 %, e.g., less than 2% alpha helices and/or beta sheets as determined by the Chou-Fasman algorithm. In some aspects, (i) the sum of glycine (G), alanine (A), serine (S), threonine (T), glutamate (E) and proline (P) residues constitutes at least about 80, 81, 82, 83, 84, 85, 86, 87, 88, 89, or 90 % of the total amino acid residues of the linker; the linker contains at least one proline, contains at least one glutamate, contains at least one lysine, contains at least one serine, and/or contains at least one glutamine; the linker contains more glutamine residue(s) than asparagine residue(s); the linker contains more serine residue(s) than threonine residues; the linker does not contain tryptophan, does not contain tyrosine, does not contain phenylalanine, does not contain cysteine, does not contain isoleucine, does not contain leucine, and/or does not contain asparagine; the linker does not contain W; does not contain

Y; and/or does not contain F; the linker does not contain an aromatic residue; the linker contains no more than one W, Y, and/or F, and/or contains no more than one aromatic residue; the linker does not contain histidine, does not contain cysteine, and/or does not contain histidine or cysteine; the linker contains no more than one histidine, no more than one histidine, and/or no more than one histidine or cysteine; and/or a majority of residues in the linker are charged. In some aspects, the linker is designed to avoid the presence of certain protease cleavage sites, such as sites for cleavage by serum protease(s) and/or to avoid immunogenicity.

In some aspects, the linker is designed to be non-repetitive or relatively non-repetitive. The term "non-repetitiveness" as used herein in the context of a polypeptide refers to a lack or limited degree of internal homology in a peptide or polypeptide sequence. The term "substantially non-repetitive" can mean, for example, that there are few or no instances of four contiguous amino acids in the sequence that are identical amino acid types; or that the polypeptide has an average subsequence score of 3 or less; or that there isn't a pattern in the order, from N- to C-terminus, of the sequence motifs that constitute the polypeptide sequence. The term "repetitiveness" as used herein in the context of a polypeptide refers to the degree of internal homology in a peptide or polypeptide sequence. In contrast, a "repetitive" sequence may contain multiple identical copies of short amino acid sequences. In the context of a polypeptide, a sequence can contain multiple copies of shorter sequences of defined or variable length, or motifs, in which the motifs themselves have non-repetitive sequences, rendering the full-length polypeptide substantially non-repetitive.

The degree of repetitiveness of a polypeptide or a gene can be measured by computer programs or algorithms or by other means known in the art. According to the current disclosure, algorithms to be used in calculating the degree of repetitiveness of a particular polypeptide, such as a linker disclosed herein, are disclosed. In one aspect, the repetitiveness of a polypeptide of a predetermined length can be calculated (hereinafter "subsequence score") according to the formula given by Equation 1:

$$\text{Subsequence Score} = \frac{\sum_{i=1}^{m} Count_i}{m}$$

wherein: m=(amino acid length of polypeptide)-(amino acid length of subsequence)+1;

and $Count_i$=cumulative number of occurrences of each unique subsequence within sequence; Examples for calculating the subsequence scores of particular peptide sequences are provided in U.S. Patent Application Publication No. US 2013/0017997, the disclosure of which is incorporated herein by reference for all purposes.

In some aspects, it contains no three contiguous amino acids that are identical unless the amino acids are serine or glycine or, has a subsequence score of less than 10, 9, 8, 7, 6, 5, 4, 3, or 2, or contains at least one residue other than glycine and serine. In some aspects, the linker does not contain a cleavage site for (or is not cleaved by) any serum protease, or for one or more or any of: proteins of the blood coagulation pathway family, Factors XIIIa, XIIa, XIa, Xa, IXa, and VIIa, thrombin, plasma kallikrein, activated PC, Prothrombinase, hK1, PSA/hK3, hK10, hK15, activated protein C (APC), Hageman factor (Coagulation factor XII), Factor Xa, ADAMTS13, von Willebrand factor-cleaving protease (VWFCP), protease nexin 2, Plasmin, Trypsin, $\alpha$-chymotrypsin, matrix metalloproteinase-2 , matrix metalloproteinase-9, Elastase, MASP-1 (mannose-binding lectin -associated serine protease-1), MASP-2, MASP-3, Cathepsin K, Cathepsin B, Streptokinase, Plasma Procarboxypeptidase B, Thrombin-Activatable Fibrinolysis Inhibitor (TAFI), plasminogen activator family (such as tissue plasminogen activator, urinary plasminogen activator (uPA)), Urokinase, complement convertase family proteins, Factor C1r, C1s, Factor D, and C3 convertases, such as C4b•2a, C3b•Bb, and C3b•Bb•C3b.

Among the provided linkers are those having the amino acid sequence set forth as SEQ ID NO: 199, 200, 201, 202, 203, or 204, or a modified variant thereof wherein any one, two, three, or four of the residues is optionally substituted with a conjugated amino acid, such as in place of a lysine residue.

In some embodiments, the linkers are designed to avoid or reduce potential immunogenicity, such as by minimizing the likelihood of T cell epitope(s). The ability of a peptide to bind a given MHC Class II molecule for presentation on the surface of an antigen presenting cell (APC) is dependent on a number of factors, including the primary sequence of the peptide. In one aspect, a peptide or linker of the present disclosure has a low degree of immunogenicity due to its sequence being resistant to antigen processing in APCs. In another aspect, a peptide or linker of the present disclosure has a sequence that substantially does not bind an MHC receptor, or does not bind an MHC receptor in a degree sufficient to elicit an immune response. The present disclosure provides linkers and peptides comprising the linkers, wherein the linkers and peptides are substantially non-repetitive in the primary sequence and are designed to reduce binding with MHC II receptors. In other aspects, the peptides and linkers of the present disclosure do not form epitopes for T-cell receptor or antibody binding, resulting in a low degree of immunogenicity. In one aspect, avoidance of immunogenicity

can attribute to, at least in part, a result of the conformational flexibility of the linker sequences; *i.e.*, the lack of secondary structure due to the selection and order of amino acid residues. For example, sequences having a tendency to adapt compactly folded conformations in an aqueous solution or under a physiologic condition are more likely to result in conformational epitopes, than sequences having a relatively lower tendency to adapt compactly folded conformations in the aqueous solution or under the physiologic condition. The administration of fusion proteins comprising a linker of the present disclosure, using conventional therapeutic practices and dosing, would generally not result in the formation of neutralizing antibodies to the linker sequence. In one aspect, a linker sequence of low immunogenicity reduces the immunogenicity of the fusion partner, for example, when the linker is fused to linked to a meditope.

In one embodiment, the linker may contain or may be substantially free of epitopes recognized by human T cells. The elimination of such epitopes for the purpose of generating less immunogenic proteins has been disclosed previously; see for example WO 98/52976, WO 02/079232, and WO 00/3317 which are incorporated by reference herein. The same principles, e.g. the identification of epitopes, can be used to introduce such epitopes into the linker. Assays for human T cell epitopes have been described (Stickler, M., et al. (2003) J Immunol Methods, 281: 95-108). Of particular interest are peptide sequences that can be oligomerized with or without generating T cell epitopes or non-human sequences. This is achieved by testing direct repeats of these sequences for the presence of T-cell epitopes and for the occurrence of 6 to 15-mer and, in particular, 9-mer sequences that are not human, and then altering the design of the linker sequence to include or to eliminate or disrupt the epitope sequence. In some embodiments, the linker sequences are substantially non-immunogenic by the restriction of the numbers of epitopes of the linker predicted to bind MHC receptors. With a reduction in the numbers of epitopes capable of binding to MHC receptors, there is a concomitant reduction in the potential for T cell activation as well as T cell helper function, reduced B cell activation or up-regulation and reduced antibody production. The low degree of predicted T-cell epitopes can be determined by epitope prediction algorithms such as, e.g., TEPITOPE (Sturniolo, T., et al. (1999) Nat Biotechnol, 17: 555-61). The TEPITOPE score of a given peptide frame within a protein is the log of the $K_d$ (dissociation constant, affinity) of the binding of that peptide frame to multiple of the most common human MHC alleles, as disclosed in Sturniolo, T. et al. (1999) Nature Biotechnology 17:555). The score ranges over at least 20 logs, from about 10 to about -10 (corresponding to binding constraints of $10e^{10}$ $K_d$ to $10e^{-10}K_d$), and can be reduced by avoiding hydrophobic amino acids that serve as anchor residues during peptide display on MHC, such as M, I, L, V, F. In some embodiments, a linker sequence does not have a predicted T-cell epitope at a TEPITOPE threshold score of about -5, or -6, or -7, or -8, or -9, or at a TEPITOPE score of -10. As used herein, a score of "-9" is a more stringent TEPITOPE threshold than a score of -5.

TEPITOPE scores of 9-mer peptide sequence can be calculated by adding pocket potentials as described by Sturniolo [Sturniolo, T., et al. (1999) Nat Biotechnol, 17: 555]. TEPITOPE scores calculated by this method range from approximately -10 to +10. However, 9-mer peptides that lack a hydrophobic amino acid (FKLMVWY)(SEQ ID NO: 240) in P1 position have calculated TEPITOPE scores in the range of -1009 to -989. This value is biologically meaningless and reflects the fact that a hydrophobic amino acid serves as an anchor residue for HLA binding and peptides lacking a hydrophobic residue in P1 are considered non binders to HLA.

In some embodiments, the linker is or is not predicted to contain any MHC Class II epitope, for example, as predicted by an epitope-identification program or algorithm. In one embodiment, a linker of the present disclosure has or is predicted to have 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 MHC Class II epitopes. In another embodiment, a linker disclosed herein has or is predicted to have no more than about 2, no more than about 3, no more than about 4, no more than about 5, no more than about 6, no more than about 7, no more than about 8, no more than about 9, no more than about 10, no more than about 11, no more than about 12, no more than about 13, no more than about 14, no more than about 15, no more than about 16, no more than about 17, no more than about 18, no more than about 19, no more than about 20, no more than about 21, no more than about 22, no more than about 23, no more than about 24, no more than about 25, no more than about 26, no more than about 27, no more than about 28, no more than about 29, or no more than about 30 MHC Class II epitopes.

In some embodiments, the linker may or may not contain any predicted MHC Class II epitope as predicted by the ProPred tool, which is described in Singh, H. and Raghava, G.P.S. (2001), ProPred: Prediction of HLA-DR binding sites, Bioinformatics, 17(12):1236-37. ProPred is a graphical web tool for predicting MHC Class II binding regions in antigenic protein sequences. In one aspect, prediction of MHC Class II epitope using the ProPred tool utilizes quantitative matrices derived from Sturniolo, T., et al. (1999), Nat Biotechnol, 17: 555.

Additionally, the non-repetitive sequence and corresponding lack of epitopes in a linker sequence limit or enhance the ability of B cells to bind to or be activated by the linker. A repetitive sequence is recognized and can form multivalent contacts with even a few B cells and, as a consequence of the cross-linking of multiple T-cell independent receptors, can stimulate B cell proliferation and antibody production. In contrast, while a linker can make contacts with many different B cells over its extended sequence, each individual B cell may only make one or a small number of contacts with an individual linker due to the lack of repetitiveness of the sequence. In one embodiment, a linker of the present disclosure typically has a much lower tendency to stimulate proliferation of B cells and thus an immune response. In one embodiment, a meditope fused to a linker has reduced immunogenicity as compared to the corresponding

meditope that is not fused to a linker.

In one embodiment, a non-repetitive linker typically forms weaker contacts with an antibody than a linker with a high degree of repetitiveness. Antibodies are multivalent molecules, for example, IgGs have two identical binding sites and IgMs contain 10 identical binding sites. Thus antibodies against repetitive sequences can form multivalent contacts with such repetitive sequences with high avidity, which can affect the potency and/or elimination of such repetitive sequences. In contrast, antibodies against non-repetitive linkers likely yield only monovalent interactions, resulting in less likelihood of immune clearance such that a peptide comprising a non-repetitive linker can remain in circulation for an increased period of time.

A linker sequence can be evaluated to identify candidate MHC Class I epitopes, e.g. using an algorithm for the prediction of an MHC Class I epitope. Many different predictive programs are available for the identification of potential MHC Class I epitopes, i.e., epitopes that are capable of being displayed on an MHC Class I molecule, in a given polypeptide. A number of exemplary algorithms are described in WO 2009/051555, the disclosure of which is incorporated herein by reference for all purposes. Examples of epitope-identification programs can be found at the websites of the following organizations and facilities: Institute of Microbial Technology (India), ProPred-I tool as described in Singh, H. and Raghava, G. P. S. Bioinformatics 22;19(8):1009-14 (2003). SYFPEITHI, as described in Hans-Georg Rammensee, et al., Immunogenetics (1999) 50: 213-219. Immune Epitope Database. See e.g. Kim Y, Sidney J, Pinilla C, Sette A, Peters B., BMC Bioinformatics 10:394 (2009). Max-Planck-Institute for Infection Biology. See e.g. J. Hakenberg, A. Nussbaum, H. Schild, H.-G. Rammensee, C. Kuttler, H.-G. Holzhütter, P.-M. Kloetzel, S.H.E. Kaufmann, H.-J. Mollenkopf (2003) MAPPP - MHC-I Antigenic Peptide Processing Prediction. Applied Bioinformatics 2(3): 155-158. Technical University of Denmark, NetChop Server, as described in Nielsen M, Lundegaard C, Lund O, Kesmir C. Immunogenetics.57(1-2): 33-41, 2005.

The algorithms used within these prediction web sites can be found at the same sites. The following is a non-exhaustive list of references describing suitable prediction algorithms: Parker et al. (1994) Scheme for ranking potential HLA-A2 binding peptides based on independent binding of individual peptide side-chains, J. Immunol., 152, 163-175; Hans-Georg Rammensee et al. (1999) SYFPEITHI: database for MHC ligands and peptide motifs, Immunogenetics 50: 213-219; Reche et al. (2002) Prediction of MHC Class I binding peptides using profile motifs. Hum Immunol. 63 (9):701-9; Greenbaum et al. (2007) Journal of Molecular Recognition 20 (2):75-82.

In one embodiment, a linker of the present disclosure does not contain any epitope for an MHC receptor. In one embodiment, a linker of the present disclosure does not contain any MHC Class I or Class II epitope. In another embodiment, a linker disclosed herein does not contain any predicted MHC Class I epitope, for example, as predicted by an epitope-identification program or algorithm. In one embodiment, a linker of the present disclosure has or is predicted to have 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 MHC Class I epitopes. In another embodiment, a linker disclosed herein has or is predicted to have no more than about 2, no more than about 3, no more than about 4, no more than about 5, no more than about 6, no more than about 7, no more than about 8, no more than about 9, no more than about 10, no more than about 11, no more than about 12, no more than about 13, no more than about 14, no more than about 15, no more than about 16, no more than about 17, no more than about 18, no more than about 19, no more than about 20, no more than about 21, no more than about 22, no more than about 23, no more than about 24, no more than about 25, no more than about 26, no more than about 27, no more than about 28, no more than about 29, or no more than about 30 MHC Class I epitopes.

The constant region portion of the provided meditopes and compounds, e.g., $R^{4A}$, generally includes one or more constant region found in the Fc region of an Ig molecule, or functional portion thereof, such as of an IgG molecule. The portion in some aspects does not include a variable region domain or functional fragment thereof. For example, in some contexts, the constant region portion contains one or more of a heavy chain CH1, CH2, and/or CH3. In some examples, the constant region portion may further include a CH4. In some cases, the portion includes only a CH3, e.g., does not include a CH1, CH2, or CH4 portion. Exemplary constant region portions include 205 and 206, and modified variants thereof, e.g., where any one, two, three, or four amino acids are substituted with a conjugated amino acid, such as a substitution at a lysine residue.

In some embodiments, the linker contains a reactive functionality, which can be used for derivatization, e.g., conjugation of the multivalent meditope, e.g., via the scaffold or linker, to an agent, such as a diagnostic, e.g., imaging agent or therapeutic agent. In some embodiments, the linker comprises one or more conjugation sites, including, for example, lysines, tyrosines, glutamic acids, aspartic acids, FGE sequences, or an unnatural amino acids useful in conjugating scaffolds, linkers, or agents. In some embodiments, the number and location of conjugation sites can be controlled by adding or removing conjugation sites, thereby controlling the amount or location of the subsequently conjugated agent. In some embodiments, a linker comprising a PASylation sequence, a sortase sequence, or an intein sequence further comprises one or more conjugation sites.For example, in some embodiments, the multivalent meditope comprises one or more PASylation sequences, including, for example, in the linker. PASylation sequences can comprise prolines, alanines, and serines in a defined or random sequence. PASylation sequences can therefore include peptides comprising neutral and hydrophobic amino acids. SEQ ID NO: 231 discloses an exemplary trivalent meditope. The

sequence comprises PASylation is described in Morath, et al. Mol Pharm 2015 May 4;12(5):1431-42. PASylation may improve drug delivery and prolong plasma half-life. For example PASylation my increase the hydrodynamic volume of a protein, thus prolonging circulation and/or boosting bioactivity before kidney filtration.

In some embodiments, thiol functionalities can be introduced in any suitable position on the linker and can be selectively modified using a number of external reagents containing diagnostic or therapeutic agents, such as imagining agents, other proteins and peptides, metal chelators, siRNAs, nanoparticles, cytotoxic agents, and the like.

For example, in some embodiments, the meditope, e.g., the multivalent meditope has one or more chains (e.g., a polypeptide chain) having the amino acid sequence of SEQ ID NO: 57, 58, 59, 64, 250, 251, 252, or 192, or a modified variant thereof wherein any one, two, three, or four of the residues is optionally substituted with a conjugated amino acid, such as at a lysine. The chain in some embodiments is a conjugated peptidyl moiety, comprising a metal chelator bound to a metal ion, a small molecule, a chemotherapeutic agent, a therapeutic antibody or a functional fragment thereof, a toxin, a radioisotope, an enzyme, a nuclease, a hormone, an immunomodulator, an oligonucleotide, an organic or inorganic nanoparticle, an RNAi molecule, an siRNA, a chelator, a boron compound, a photoactive agent, a dye, a fluorescent or luminescent substance, an enzyme, an enhancing agent, a radioactive substance, or a chelator.

In some embodiments, the multivalent meditope may be circularized. Circularized multivalent meditopes may be more stable or possess more constrained geometry than linear multivalent meditopes. Circularized multivalent meditopes may also have different functional properties over linear multivalent meditopes, including faster clearance. In some embodiments, the multivalent meditope comprises one or more sortase or "intein" sequences, which may assist with circularizing the multivalent meditope or creating linear chains of meditopes. In some embodiments, the linker of a multivalent meditope comprises a sortase or intein sequence. Intein sequences include Ssp $I_c$ and Ssp $I_N$ sequences, and are described in Scott et al., Proc Natl Acad Sci USA. 1999 Nov 23;96(24):13638-43. SEQ ID NO: 231 comprises a split sortase sequence comprising the "GG" sequence at positions 118 and 119 and the "PETG" sortase sequence at positions 254-258 of SEQ ID NO: 231. In some embodiments, the sortase sequence comprises the sequence LPXTG (SEQ ID NO: 249), wherein X comprises any amino acid. Sortase sequences are described in Antos et al., J Biol Chem. 2009 Jun 5;284(23):16028-36. SEQ ID NO: 237-239 disclose additional exemplary multivalent meditope peptides.

In some aspects, the chain of the multivalent meditope is a first chain and the meditope further includes a second chain, such as a chain as described above and herein, e.g., having the formula $R^{3A}$-$L^{3A}$-$R^{4A}$ (Formula I) or $R^{3A}$-$L^{3A}$-$R^{4A}$-$L^{3B}$-$R^{3B}$ (Formula III).

In some embodiments, the composition of the linker and/or the distance between the Fc and the meditope is systematically altered, e.g., to optimize affinity and specificity. In one embodiment, each natural or unnatural residue can be substituted at any position within the linker for optimization. In some aspects, the linker is between at or about 2 and 100 residues in length, e.g., at or about 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 125, 150, 175, or 200 residues (amino acids) in length, or more. In one example, such linkers are generated using current and future DNA synthesizers, and inserted between the meditope and the Fc regions. The linker may also be 'rigidified' to limit the radius of gyration and to enhance the affinity and specificity of the Fc-meditope. For example, a coiled coil domain may be placed between the meditope and the Fc (Figure 3). In other examples, inert protein domains (e.g., immunoglobulin folds) are substituted for the linker. Multiple immunoglobulin folds can be placed between the meditope and the Fc domain. In certain embodiments, the composition of the linker is of human origin to mitigate potential antigenicity.

In some examples, the provided meditopes are tethered to a scaffold to create a multivalent meditope, e.g., for enhanced selectivity and binding affinity. In some embodiments, multivalent meditope scaffolds are used to scaffold or "daisy-chain" meditope-enabled mAbs bound to tumor associated antigen to enhance ligand antagonism, alter receptor endocytosis, and/or improve an immune response through ADCC/CDC (see Figure 1). Thus, in some embodiments, the meditopes are used to tether two or more antibodies or functional fragments thereof. Such meditopes may be part of a multivalent meditope, for example, to enhance cancer or tumor therapy and imaging. A multivalent meditope may include two or more meditopes coupled through linker(s), such as through a long linker and biotin to streptavidin, to create a multivalent meditope tethering entity.

In one embodiment, the multivalent meditope tethering entity is a tetravalent meditope tethering agent. In one aspect, the tetrameric tethering entity is shown by surface plasmon resonance to have enhanced binding to an antibody as compared to the monovalent peptide, which is consistent with a multivalent interaction. In one example, use of such multivalent meditopes (e.g., tetravalent meditope) produces enhanced binding affinity of the meditope-enabled antibody to the antigen (e.g., in the case of cetuximab, binding affinity for EGFR positive cells), compared to use of the antibody alone or monovalent meditope alone. Such binding affinities can be determined using well-known methods, e.g., by FACS analysis. See Figure 4.

In some embodiments, to address the receptor constraints on the linker, unmodified or modified, e.g., variant, e.g., optimized, meditopes are coupled to a multivalent scaffold, such as by optimizing the linker. In some aspects, the multivalent meditope is able to "latch-on" to adjacent antibodies, e.g., IgGs, to form a "daisy-chain"-like array (see Figure 1), which can be used, for example, in antibodies targeting tumor antigens, given the high antigen density of tumor cells.

While an intramolecular association of a bivalent meditope and antibody is possible, the C2 symmetry of the antibody, e.g., IgG, can place geometrical constraints on the linker for such an interaction. Thus, in some aspects, the multivalent meditope is based on a trivalent or higher valency scaffold, ensuring that more than one antibody would be "daisy chained". By including a third meditope arm, the lifetime of the initial encounter of a trivalent meditope to antigen-bound antibody can increase. This, in turn, can increase the probability that an additional arm will bind to a neighboring antigen-bound antibody, thus stabilizing the overall complex. In other embodiments, a multifunctional meditope may be constructed to simultaneously bind a meditope enabled antibody and other targets, such as other B and T-cell surface proteins, such as T cell receptor and costimulatory molecules. In some embodiments, multiple meditope-enabled antibodies (for example, including one or more modified meditope-enabled antibodies) having specificities for different meditopes are used together with such different meditopes in such multivalent embodiments.

Various linkers and scaffolds are known in the art and may be used in connection with these embodiments. An exemplary scaffold synthesis scheme is shown in Figure 5 and discussed in the Examples herein. In some aspects, the use of templates 4 and 5 shown in Figure 5 allows for the formation of both bi- and trivalent meditopes, respectively. In some embodiments, different length polyethylene glycol (PEG) and/or other linkers are used, for example, to improve or alter binding or other properties. In some aspects, the PEG length is between at or about 10 A and at or about 1000 A. The synthetic approach is also amenable to DOTA incorporation for radionuclide imaging. For example, a 30 Å PEG bifunctional arm has been incorporated in the synthesis of a FITC-labeled divalent meditope, namely compound 13 (Figure 5). The distance between the CDR regions within an IgG in some examples is ~130 Å. The distance can vary, e.g., between about 50Å and about 150 Å in length; or between about 50Å and about 200 Å in length, or between about 100Å and about 140Å in length, such as at or about 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 150, 160, 170, 180, 190, or 200 Å in length. The length of the PEG linker may be systematically varied to ensure this approach is optimal. End-to-end distances of commercially available PEGs extend to 90 Å, which would exceed the IgG distance.

In other embodiments, other scaffolds and/or linkers are used, e.g., to generate high affinity multivalent meditopes and/or to create synergy. For example, DNA may be used as a scaffold for the meditopes to create a more rigid scaffold. For example, different scaffolds of biological and chemical origin may also be used to achieve multivalency. This includes, but is not limited to, constructing a bivalent or trivalent scaffold, using streptavidin or (see European Application, Publication No.: EP 2065402 A1), streptavidin as a tetravalent scaffold, unique scaffolds (Hutchins et al, JMB 406:4, 595 (2011)), Origami DNA (see Gu et al., Nature Nanotechnology 4, 245 - 248 (2009)) and the like. A chemical scaffold may also be created using molecules including, but not limited to, DNA (single strand, duplex, Holliday junctions, aptamers and the like), RNA (single strand, hairpin, stem loop, aptamers and the like), PNA (peptide nucleic acids), DNA/PNA duplexes and triplexes for rigidity, organic and or inorganic nanoparticles (directly coupled or coupled through organic polymers such as PEG), organic polymers that can form duplexes with themselves and/or with DNA or PNA.

*Characterization of multivalent meditopes*

Binding properties of the multivalent meditopes to meditope-enabled antibodies can be characterized by any of a number of known methods, including SPR and ITC, for example, to ensure that conjugation to the multivalent scaffold does not affect the meditope-Ig interaction. In some cases, such measurements can be limited in their ability to determine multivalency and synergistic effects, given that these approaches generally do not involve antigen present on a cell surface (such as on the surface of a tumor cell). Thus, in some aspects, FACS analysis and/or cell viability assays are used to quantify the effect of the multivalent meditope directly on cells expressing antigen recognized by the meditope-enabled antibody (e.g., cells that overexpress EGFR in the context of cetuximab). In general, a cell line expressing (e.g., over-expressing) the antigen recognized by the meditope-enabled antibody is incubated with the meditope-enabled antibody under conditions whereby the antibody binds to the antigen expressed on the cells. In some cases, varying concentrations of the antibody are used. Either simultaneously or subsequently, the cells are incubated with the multivalent meditope, in some cases in varying concentrations. Appropriate incubation times and washes are carried out. A second antibody and monovalent meditopes may be used as positive and negative controls, respectively. The antibodies and meditopes may be labeled with agents detectable by flow cytometry or microscopy, which are well known. In some examples, a shift (in the case of FACS) or increased signal in the presence of multivalent meditope, compared to monovalent meditope, indicates a synergistic or additive effect. In another example, to further confirm the additive effects of the multivalent meditope, the non-labeled, monovalent meditope is used in a competition assay, to determine whether it can compete with the labeled multivalent meditope for the antigen-bound meditope-enabled antibody.

In other examples, cell viability assays are used to determine the ability of a multivalent meditope to enhance cell-killing effects of a meditope-enabled antibody. For example, a cell expressing the antigen of interest may be incubated with varying concentrations of the meditope-enabled antibody and/or multivalent meditope. Monovalent meditopes and

second antibodies again are useful as controls. In some examples, MTT (3-(4,5-Dimethylthiazol-2-yl)-2,5-diphenyltetrazolium bromide), is used to quantify the number or percentage of viable cells. Other approaches for measuring cell viability, proliferation, and/or death are known in the art and may be used.

In another example, for multivalent meditopes that demonstrate activity in such assays, Western blot analysis or other biochemical or signaling approach is performed to investigate inhibition of signaling associated with a particular antigen, such as a cell surface receptor (e.g., in the case of cetuximab, to follow the phosphorylation status of EGFR, AKT, MAP which are part of the EGFR signaling pathway). Data from such studies may be compared with data from cells treated only with meditope-enabled antibody (i.e., without meditope), with monovalent meditopes, and/or with tyrosine kinase or other known inhibitors (AG1478, etc.). In some examples, an increase in cell death as a function of multivalent meditope concentration is observed, demonstrating synergistic cell killing effects of the multivalent meditopes.

**I. Variant meditopes**

Among the provided meditopes are meditope variants (also called variant meditopes), having one or more modifications, e.g., structural modifications, as compared to meditope 1 or 2 (meditope of SEQ ID NO: 1 or 2), and methods for producing the same. In some embodiments, cQFD and cQYN meditopes are used as starting points in the design of meditope variants. In some aspects, the meditope variants are designed to have altered properties, such as increased or altered affinity, altered pH dependence, or different affinities under different physiological conditions for one or more of the provided meditope-enabled antibodies, including cetuximab and other antibodies described herein, e.g., as compared to the unmodified meditopes, cQFD and cQYN. Meditope variants are designed and produced using various chemical and biophysical methods.

Meditope variants include, but are not limited to, variants incorporating modifications to meditopes, e.g., cQFD and cQYN and others described herein. Suitable modifications include, but are not limited to, any peptide modification known in the art, such as, but not limited to, modifications to the manner and/or position of peptide cyclization, modifications to one or more amino acid components of the cyclic peptide, or adding or deleting one or more amino acid from the cyclic peptide. In a particular example, cQFD may be altered with one or more of the following modifications: a modification of Arg8, a modification of Phe3, a modification of Leu5, a modification of Leu10, change to the mode of peptide cyclization, and/or an incorporation of hydratable carbonyl functionality at one or more positions, and one or more amino acid deletions or additions. In the case of cQYN, suitable modifications may include one or more of the following: a modification of Arg8, a modification of Leu5, a modification of Leu10, change to the mode of peptide cyclization, and/or an incorporation of hydratable carbonyl functionality at one or more positions, and one or more deletions or additions. Certain amino acid positions within the meditope may be deleted or replaced with a different natural amino acid or an unnatural amino acid, or the meditope may be chemically conjugated with a fragment, for example by using "click chemistry". It is shown herein that a meditope in which Arg9 of SEQ ID NO: 1 has been mutated to citrulline binds to cetuximab. In addition, the amino and carboxy termini can be extended with further amino acids beyond (i.e., in addition to) the cyclic portion of the meditope variant in order to make additional contact to the Fab. For example, Protein L has been added to the C-terminus of the cQFD meditope and preliminary data shows that this binds with much higher affinity.

In some embodiments, the meditopes include those listed in Tables 1 and 2, as well as such meditopes with additional amino acids, such as those up to 16 amino acids in length. For example, in some aspects, the meditope is one of meditopes 1, 2, or 15-55, further including a serine before the first residues, i.e., at position zero. The meditopes listed in Table 1 employ a disulfide linkage to connect the C and N termini, except that certain meditopes contain an additional sequence, such as a tail or a leader, such that the linkage is not between the two terminal residues. Exemplary tail and leader sequences are those having between 1 and 50 amino acids in length, such as between 1 and 40, 1 and 30, 1 and 25, 1 and 20, 1 and 15, 1 and 10, 9, 8, 7, 6, 5, 4, 3, or 2 amino acids in length, such as the tails set forth as $R^{5A}$ and $R^{5B}$, herein. Exemplary tails and leader sequences contain any one or more G, S, P, K, and/or D residue(s). Exemplary tails and leaders include GGGSK, G, GPGGSDPG, and GPGGSDPG. For example, the meditope having SEQ ID NO: 31 contained an additional tail, meaning that the disulfide linkage is not between the two terminal residues. Likewise, certain other meditopes in the table (SEQ ID NOs: 187-190, 207) contain tails and/or leaders, resulting in linkage between residues other than the terminal residues. For the peptides in Table 2, a lactam bridge, a linkage other than disulfide (such as [3+2] cycloaddition), or no linkage is used (e.g., an acyclic or linear variant). Additional meditopes that may be used according to the embodiments described herein include any meditope as defined herein peptide that binds to an antibody framework binding interface (i.e., between the Fab light and heavy chains) of cetuximab or any other therapeutic antibody. For example, in addition to the cyclic peptides cQFD and cQYN, some embodiments include one or more variants of cQFD and cQYN.

Table 1.

| SEQ ID NO | Meditope Number | Sequence | Modification (red) | Linkage method |
|---|---|---|---|---|
| 1 | 1 | C-QFDLSTRRLK-C | original | Disulfide 1-Cys : 12-Cys |
| 2 | 2 | C-QYNLSSRALK-C | original | Disulfide 1-Cys : 12-Cys |
| 15 | 15 | C-qFDLSTRRLK-C | q = D-glutamine | Disulfide 1-Cys : 12-Cys |
| 16 | 16 | C-QYDLSTRRLK-C | Y = tyrosine | Disulfide 1-Cys : 12-Cys |
| 17 | 17 | C-QXDLSTRRLK-C | X = β-β'-di-phenyl-Ala | Disulfide 1-Cys : 12-Cys |
| 18 | 18 | C-QFDXSTRRLK-C | X = β-β'-di-phenyl-Ala | Disulfide 1-Cys : 12-Cys |
| 19 | 19 | C-QFDFSTRXLK-C | F = phenylalanine, X = citrulline | Disulfide 1-Cys : 12-Cys |
| 20 | 20 | C-QFDFSTRRLK C | F = phenylalanine | Disulfide 1-Cys : 12-Cys |
| 21 | 21 | C-QFDESTRRLK-C | E = glutamic acid | Disulfide 1-Cys : 12-Cys |
| 22 | 22 | C-QFDYSTRRLK-C | Y = tyrosine | Disulfide 1-Cys : 12-Cys |
| 23 | 23 | C-QFDLSTRRQK-C | Q = glutamine | Disulfide 1-Cys : 12-Cys |
| 24 | 24 | C-QFDLSTRQLK-C | Q = glutamine | Disulfide 1-Cys : 12-Cys |
| 25 | 25 | C-QYNLSTARLK-C | Y = tyrosine; N = asparagine; A = alanine | Disulfide 1-Cys : 12-Cys |
| 26 | 26 | C-QADLSTRRLK-C | A = alanine | Disulfide 1-Cys : 12-Cys |
| 27 | 27 | C-QFDASTRRLK-C | A = alanine | Disulfide 1-Cys : 12-Cys |
| 28 | 28 | C-QFDLSTARLK-C | A = alanine | Disulfide 1-Cys : 12-Cys |
| 29 | 29 | C-QFDLSTRRAK-C | A = alanine | Disulfide 1-Cys : 12-Cys |
| 30 | 30 | C-QFDLSTRREK-C | E = glutamic acid | Disulfide 1-Cys : 12-Cys |
| 31 | 31 | AcC-QFDLSTRRLR-CGGGSK | AcC-N-acetylcysteine R = arginine | Disulfide 1-AcCys : 12 Cys |
| 186 | 186 | C-QFDLSTRRLR-C | R = arginine | Disulfide 1-Cys : 12-Cys |
| 187 | | GC-QFDLSTRRLR-C | R = arginine | Disulfide 2-Cys : 13-Cys |

(continued)

| SEQ ID NO | Meditope Number | Sequence | Modification (red) | Linkage method |
|---|---|---|---|---|
| 188 | | GPGGSDPGC-QFDLSTRRLR-C | R = arginine | Disulfide 9-Cys : 20-Cys |
| 189 | | C-QFDLSTRRLR-CG | R = arginine | Disulfide 1-Cys : 12-Cys |
| 190 | | GPGGSDPGC-QFDLSTRRLR-CG | R = arginine | Disulfide 9-Cys : 20-Cys |
| 207 | | GC-QFDLSTRRLR-CG | R = arginine | Disulfide 2-Cys : 13-Cys |

**Table 2**

| SEQ ID NO | | Sequence | Modification (red) | Linkage method |
|---|---|---|---|---|
| 32 | 32 | G-QFDLSTRRLK-G | G = glycine | Lactam 1-Gly : 12-Gly |
| 33 | 33 | G-QHDLSTRRLK-G | H = histidine | Lactam 1-Gly : 12-Gly |
| 34 | 34 | G-QNDLSTRRLK-G | N = asparagine | Lactam 1-Gly : 12-Gly |
| 35 | 35 | G-QQDLSTRRLK-G | Q = glutamine | Lactam 1-Gly : 12-Gly |
| 36 | 36 | G-QXDLSTRRLK-G | X = 2-bromo-L-phenylalanine | Lactam 1-Gly : 12-Gly |
| 37 | 37 | G-QXDLSTRRLK-G | X = 3-bromo-L-phenylalanine | Lactam 1-Gly : 12-Gly |
| 38 | 38 | G-QXDLSTRRLK-G | X = 4-bromo-L-phenylalanine | Lactam 1-Gly : 12-Gly |
| 39 | 39 | G-QFDLSTRXLK-G | X = citrulline | Lactam 1-Gly : 12-Gly |
| 40 | 40 | G-QFDLSTXXLK-G | X = citrulline | Lactam 1-Gly : 12-Gly |
| 41 | 41 | G-QFDLSTXRLK-G | X = citrulline | Lactam 1-Gly : 12-Gly |
| 42 | 42 | Q-FDLSTRRLK-X | X = 7-aminoheptanoic acid | Lactam 1-Gln: 11-X |
| 43 | 43 | X-QFDLSTRRLK-X | X = β -alanine | Lactam 1-X: 12-X |
| 44 | 44 | X-QFDLSTRRLK-X’ | X = diaminopropionic acid; X’ = iso-aspartic acid | Lactam 1-X: 12-X’ |
| 45 | 45 | X-QFDLSTRRLK-X’ | X = β -alanine; X’ = iso-aspartic acid | Lactam 1-X: 12-X’ |
| 46 | 46 | X-QFDLSTRRLK-X’ | X = diaminopropionic acid; X’ = β -alanine | Lactam 1-X: 12-X’ |
| 47 | 47 | F-DLSTRRL-K | | Lactam 1-Phe : 9-Lys |
| 48 | 48 | C-QFDLSTRRLK-C | | Disulfide 1 Cys : 12-Cys; Lactam 4-Asp to 11-Lys |
| 49 | 49 | Q-YDLSTRRLK-X | Y = tyrosine, X = 7-aminoheptanoic acid | Lactam 1-Gln: 11-X |
| 50 | 50 | X-QFDLSTRRLK-X’ | X = β-azidoalanine, X’ = propargylglycine | [3+2] cycloaddition Azide-1-X: alkyne-12-X’ |
| 51 | 51 | Q-XDLSTRRLK-X’ | X = β-β’-di-phenyl-Ala, X’ = 7-aminoheptanoic acid | Lactam 1 Gln: 11-X’ |

(continued)

| SEQ ID NO | | Sequence | Modification (red) | Linkage method |
|---|---|---|---|---|
| **52** | **52** | **qFDLSTRRLK-X** | **q = D-glutamine, X = 7-aminoheptanoic acid** | **Lactam 1-Gln: 11-X** |
| **53** | **53** | **Q-XDXSTRRLK-X'** | **X = β-β'-di-phenyl-Ala, X' = 7-aminoheptanoic acid** | **Lactam 1 Gln: 11-X'** |
| **54** | **54** | **Q-FDLSTXRLK-X'** | **X = n-butyl-arginine, X' = 7-aminoheptanoic acid** | **Lactam 1 Gln: 11-X'** |
| **55** | **55** | **SQFDLSTRRLKS** | | **No linkage** |

The meditope variants typically have an amino acid sequence length of between 5 and 16 amino acids, e.g., 5, 6, 7, 8, 9, 10, 11, 12, 13, 14, 15, or 16 amino acids in length, such as between 8 and 13 amino acids in length, e.g., between 9 and 12 amino acids in length. The meditope can additionally be conjugated to or associated with (e.g., as part of a fusion protein or peptide) with another molecule, such as another peptide, including a linker or agent. Thus, in this case, the compound containing the meditope may contain additional amino acid residues beyond the lengths described in this paragraph, where the meditope portion contains between 5 and 16 amino acids and the complex or compound contains additional amino acids. Examples are described herein, e.g., SEQ ID NO: 31 above.

In some embodiments, the variant meditopes are cyclic peptides. In other embodiments, they are linear or acyclic peptides.

The meditopes can include peptides, or cyclic peptides derived from such peptides, for example, where the peptides have the formula:

X1-X2-X3-X4-X5-X6-X7-X8-X9-X10-X11-X12 (Formula VI),

for example, where:

X1 = Cys, Gly, β-alanine, diaminopropionic acid, β-azidoalanine, or null;

X2 = Gln or null;

X3 = Phe, Tyr, β-β'-diphenyl-Ala, His, Asp, 2-bromo-L-phenylalanine, 3-bromo-L-phenylalanine, or 4-bromo-L-phenylalanine, Asn, Gln, a modified Phe, a hydratable carbonyl-containing residue; or a boronic acid-containing residue;

X4 = Asp or Asn;

X5 = Leu; β-β'-diphenyl-Ala; Phe; Trp; Tyr; a non-natural analog of phenylalanine, tryptophan, or tyrosine; a hydratable carbonyl-containing residue; or a boronic acid-containing residue;

X6 = Ser;

X7 = Thr or Ser;

X8 = Arg, Ser, a modified Arg, or a hydratable carbonyl or boronic acid-containing residue;

X9 = Arg, Ala;

X10 = Leu, Gln, Glu, β-β'-diphenyl-Ala; Phe; Trp; Tyr; a non-natural analog of phenylalanine, tryptophan, or tyrosine; a hydratable carbonyl-containing residue; or a boronic acid-containing residue;

X11 = Lys; and

X12 = Cys, Gly, 7-aminoheptanoic acid, β-alanine, diaminopropionic acid, propargylglycine, isoaspartic acid, or null.

In some aspects, the modified Arg has a structure of the formula shown in Figure 6. In some aspects, the modified Phe is a Phe with one or more halogen incorporated into the phenyl ring. In some aspects, formula VI is not SEQ ID NO: 1 or SEQ ID NO: 2.

In some embodiments, the meditopes are peptides having the structure of Formula VII:

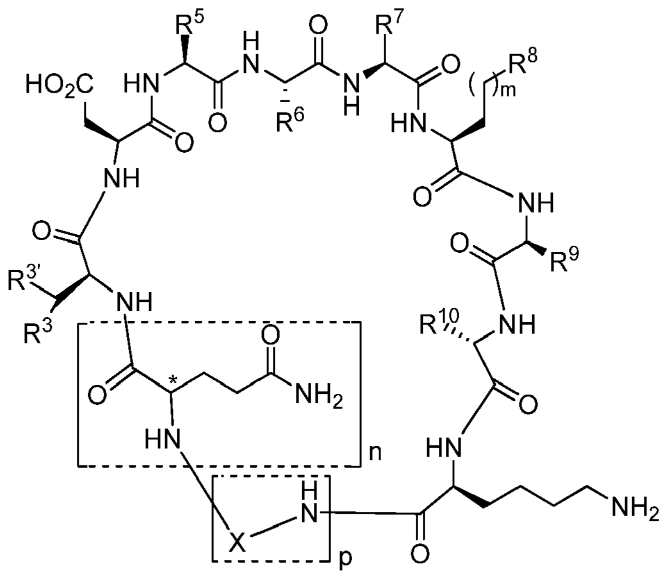

(X)

wherein:

the center marked with "*" is in the "R" or "S" configuration;

$R^3$ and $R^{3'}$ are each, independently, H or phenyl, optionally substituted with one, two, or three substituents independently selected from $C_{1-4}$alkyl, -OH, fluoro, chloro, bromo, and iodo;

$R^5$ is:

(A) $C_{1-8}$alkyl, optionally substituted with one or more substituents selected from the group consisting of oxo, acetal, ketal, $-B(OH)_2$, boronic ester, phosphonate ester, ortho ester, $-CO_2C_{1-4}$alkyl, -CH=CH-CHO, -CH=CH-C(O)$C_{1-4}$alkyl, -CH=CH-$CO_2C_{1-4}$alkyl, $-CO_2H$, and $-CONH_2$ group; or

(B) a $C_{1-4}$alkyl group substituted with:

a) one or two phenyl groups, wherein each phenyl is optionally substituted with one, two, or three substituents independently selected from -OH, fluoro, chloro, bromo, and iodo; or

b) a naphthyl, imidazole, or indole group;

$R^6$ is $-C_{1-4}$alkyl-OH or $-C_{1-4}$alkyl-SH;

$R^7$ is $-C_{1-4}$alkyl-OH or $-C_{1-4}$alkyl-SH;

m is 0, 1, 2, 3, 4, or 5;

$R^8$ is:

(a) -OH, $-NR^aR^b$, $-N(R^c)C(O)R^e$, or $-N(R^c)C(=NR^d)R^e$;

wherein:

$R^a$ is H;

$R^b$ is H or $C_{1-8}$alkyl optionally substituted with one or more substituents selected from the group consisting of oxo, acetal, and ketal,

$-B(OH)_2$, -SH, boronic ester, phosphonate ester, ortho ester, -CH=CH-CHO, - CH=CH-C(O)$C_{1-4}$alkyl, -CH=CH-$CO_2C_{1-4}$alkyl, $-CO_2H$, or $-CO_2C_{1-4}$alkyl group;

$R^c$ is H, $C_{1-8}$alkyl, $C_{3-8}$cycloalkyl, branched alkyl, or aryl;

$R^d$ is H or a $C_{1-8}$alkyl, $C_{2-8}$alkenyl, $C_{2-8}$alkynyl, $C_{3-8}$cycloalkyl, branched alkyl, or aryl group, each optionally substituted with one or more substituents selected from the group consisting of $-N_3$, $-NH_2$, -OH, -SH, halogen, oxo, acetal, ketal, $-B(OH)_2$, boronic ester, phosphonate ester, ortho ester, - CH=CH-CHO, -CH=CH-

$C(O)C_{1-4}$alkyl, -CH=CH-$CO_2C_{1-4}$alkyl, -$CO_2$H, and -$CO_2C_{1-4}$alkyl group; and

$R^e$ is H; -$NHR^d$; or a $C_{1-12}$alkyl, $C_{3-8}$cycloalkyl, $C_{2-12}$alkenyl, $C_{2-8}$alkynyl, or aryl group, each optionally substituted with one or more substituents selected from the group consisting of -$N_3$, -$NH_2$, -OH, -SH, oxo, $C_{2-4}$acetal, $C_{2-4}$ketal, -B$(OH)_2$, boronic ester, phosphonate ester, ortho ester, - CH=CH-CHO, -CH=CH-$C(O)C_{1-4}$alkyl, -CH=CH-$CO_2C_{1-4}$alkyl, and - $CO_2C_{1-4}$alkyl group; or

(b) a $C_{1-12}$ alkyl substituted with an oxo, acetal, ketal, -B$(OH)_2$, boronic ester, -SH, -OH, phosphonate ester, ortho ester, -CH=CH-CHO, -CH=CH-$C(O)C_{1-4}$alkyl, -CH=CH-$CO_2C_{1-4}$alkyl, or -$CO_2C_{1-4}$alkyl group;

$R^9$ is $C_{1-4}$alkyl or -$C_{1-2}$alkylene-$R^x$;

wherein $R^x$ is -$CO_2$H, -$CONH_2$, -$CH_2NHC(O)NH_2$, or -$CH_2NHC(=NH)NH_2$;

$R^{10}$ is:

(1) a $C_{1-8}$alkyl optionally substituted with one or more substituents selected from the group consisting of oxo, acetal, ketal, -B$(OH)_2$, boronic ester, phosphonate ester, ortho ester, -CH=CH-CHO, -CH=CH-$C(O)C_{1-4}$alkyl, -CH=CH-$CO_2C_{1-4}$alkyl, -$CO_2C_{1-4}$alkyl, -$CO_2$H, and -$CONH_2$ group; or

(2) a $C_{1-4}$alkyl group substituted with one or two phenyl groups, or one naphthyl, imidazole, or indole group, wherein each phenyl is optionally substituted with one, two, or three substituents independently selected from -OH, fluoro, chloro, bromo, and iodo;

n is 0 or 1;

p is 0 or 1;

X is $C_{1-8}$alkylene or $C_{2-8}$alkenylene, each carbon thereof optionally substituted with oxo, -C(O)-, -NHC(O)-, -$CO_2$H, -$NH_2$, or -NHC(O)$R^y$;

wherein one carbon of said alkylene is optionally replaced with -C(O)NH-, a 5-membered heteroaryl ring, or -S-S-; and

$R^y$ is -$C_{1-4}$alkyl, -CH($R^z$)C(O)- or -CH($R^z$)$CO_2$H;

wherein $R^z$ is -H or -$C_{1-4}$alkyl optionally substituted with -OH, -SH, or -$NH_2$;

or a pharmaceutically acceptable salt thereof.

In some cases X is $C_{1-8}$alkylene or $C_{2-8}$alkenylene, each carbon thereof optionally substituted with -$CO_2$H, -$NH_2$, or -NHC(O)$R^y$; wherein one carbon of said alkylene is optionally replaced with -C(O)NH-, a 5-membered heteroaryl ring, or -S-S-; and $R^y$ is -$C_{1-4}$alkyl or -CH($R^z$)$CO_2$H; wherein $R^z$ is -H or -$C_{1-4}$alkyl optionally substituted with -OH, -SH, or -$NH_2$; or a pharmaceutically acceptable salt thereof.

In some cases, such meditopes are not SEQ ID NO: 1 or 2, or are not cyclic peptides derived from such sequences, and/or are not meditope 1 or 2.

In some embodiments of the meditope of Formula VII, m is 0, 1, or 2. In other embodiments, $R^3$ is H or phenyl and $R^{3'}$ is phenyl, 2-bromophenyl, 3-bromophenyl, or 4-bromophenyl. In further embodiments, $R^5$ is methyl, ethyl, propyl, isopropyl, butyl, isobutyl, sec-butyl, or tert-butyl, each optionally substituted with an oxo, -B$(OH)_2$, -$CO_2$H, or -$CONH_2$ group, or with one or two phenyl groups each optionally substituted with a bromo or chloro substituent. In further embodiments, $R^8$ is -OH, -$NH_2$, -N($R^c$)C(O)$R^e$, or -N($R^c$)C(=$NR^d$)$R^e$. In still further embodiments, $R^c$ is H or methyl, $R^d$ is H or $C_{1-4}$alkyl, and $R^e$ is $C_{1-4}$alkyl, or -NH($C_{1-4}$alkyl). In other embodiments, $R^9$ is methyl or ethyl, optionally substituted with -$CO_2$H, -$CONH_2$, - $CH_2NHC(O)NH_2$, or -$CH_2NHC(=NH)NH_2$. In still other embodiments, $R^{10}$ is methyl, ethyl, propyl, isopropyl, butyl, isobutyl, sec-butyl, or tert-butyl, each optionally substituted with an oxo, -B$(OH)_2$, -$CO_2$H, or -$CONH_2$ group. In still other embodiments, -X-NH- is -Cys-Cys-, -Gly-Gly-, -C(O)$(CH_2)_6$-NH-, -β-Ala-β-Ala-, -C(O)CH($NH_2$)$CH_2$CH=CH$CH_2$CH($CO_2$H)-NH-, -C(O)CH($NH_2$)$CH_2$NHC(O)$CH_2$CH($CO_2$H)-NH-, -P-Ala-C(O)$CH_2$CH($CO_2$H)-NH-, or -C(O)CH($NH_2$)$CH_2$-triazinyl-$CH_2$-CH($CO_2$H)-NH-.

In some embodiments, the meditopes are peptides having the structure of Formula VIII:

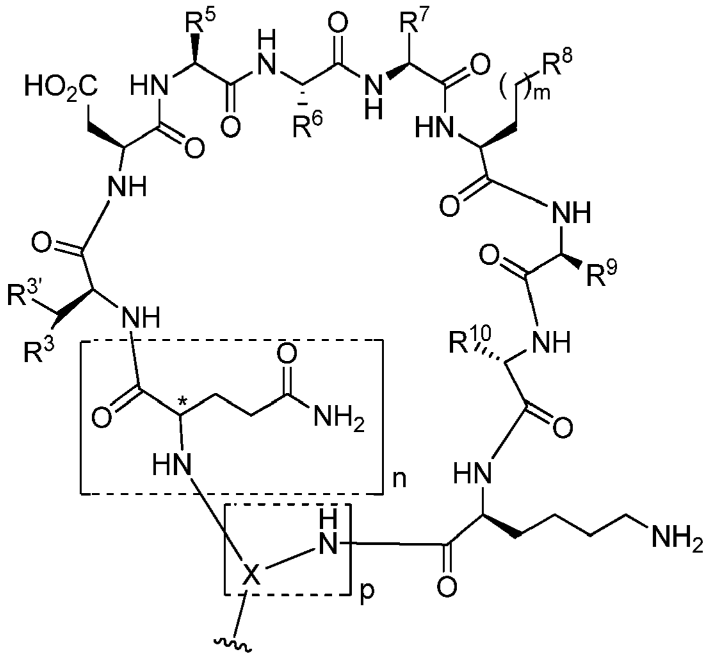

(XA).

The center marked with "*" is in the "R" or "S" configuration. The symbol ⌇ denotes the point of attachment of $R^{1A}$ to $L^{1A}$.

$R^3$ and $R^{3'}$ are each, independently, H or phenyl, optionally substituted with one, two, or three substituents independently selected from $C_{1-4}$alkyl, -OH, fluoro, chloro, bromo, and iodo;

$R^5$ is: (A) $C_{1-8}$alkyl, optionally substituted with one or more substituents selected from the group consisting of oxo, acetal, ketal, $-B(OH)_2$, boronic ester, phosphonate ester, ortho ester, $-CO_2C_{1-4}$alkyl, -CH=CH-CHO, -CH=CH-C(O)$C_{1-4}$alkyl, -CH=CH-$CO_2C_{1-4}$alkyl, $-CO_2H$, and $-CONH_2$ group; or (B) a $C_{1-4}$alkyl group substitute, with: a) one or two phenyl groups, wherein each phenyl is optionally substituted with one, two, or three substituents independently selected from -OH, fluoro, chloro, bromo, and iodo; or b) a naphthyl, imidazole, or indole group.

$R^6$ is $-C_{1-4}$alkyl-OH or $-C_{1-4}$alkyl-SH. $R^7$ is $-C_{1-4}$alkyl-OH or $-C_{1-4}$alkyl-SH. The symbol m is 0, 1, 2, 3, 4, or 5.

$R^8$ is -OH, $-NR^aR^b$, $-N(R^c)C(O)R^e$, or $-N(R^c)C(=NR^d)R^e$. $R^a$ is H. $R^b$ is H or $C_{1-8}$alkyl optionally substituted with one or more substituents selected from the group consisting of oxo, acetal, and ketal, $-B(OH)_2$, -SH, boronic ester, phosphonate ester, ortho ester, -CH=CH-CHO, -CH=CH-C(O)$C_{1-4}$alkyl, -CH=CH-$CO_2C_{1-4}$alkyl, $-CO_2H$, or $-CO_2C_{1-4}$alkyl group. $R^c$ is H, $C_{1-8}$alkyl, $C_{3-8}$cycloalkyl, branched alkyl, or aryl. $R^d$ is H or a $C_{1-8}$alkyl, $C_{2-8}$alkenyl, $C_{2-8}$alkynyl, $C_{3-8}$cycloalkyl, branched alkyl, or aryl group, each optionally substituted with one or more substituents selected from the group consisting of $-N_3$, $-NH_2$, -OH, -SH, halogen, oxo, acetal, ketal, $-B(OH)_2$, boronic ester, phosphonate ester, ortho ester, - CH=CH-CHO, -CH=CH-C(O)$C_{1-4}$alkyl, -CH=CH-$CO_2C_{1-4}$alkyl, $-CO_2H$, and $-CO_2C_{1-4}$alkyl group. $R^e$ is H; $-NHR^d$; or a $C_{1-12}$alkyl, $C_{3-8}$cycloalkyl, $C_{2-12}$alkenyl, $C_{2-8}$alkynyl, or aryl group, each optionally substituted with one or more substituents selected from the group consisting of $-N_3$, $-NH_2$, -OH, -SH, oxo, $C_{2-4}$acetal, $C_{2-4}$ketal, $-B(OH)_2$, boronic ester, phosphonate ester, ortho ester, -CH=CH-CHO, -CH=CH-C(O)$C_{1-4}$alkyl, -CH=CH-$CO_2C_{1-4}$alkyl, and $-CO_2C_{1-4}$alkyl group. Alternatively, $R^8$ is a $C_{1-12}$ alkyl substituted with an oxo, acetal, ketal, $-B(OH)_2$, boronic ester, -SH, -OH, phosphonate ester, ortho ester, -CH=CH-CHO, -CH=CH-C(O)$C_{1-4}$alkyl, -CH=CH-$CO_2C_{1-4}$alkyl, or $-CO_2C_{1-4}$alkyl group.

$R^9$ is $C_{1-4}$alkyl or $-C_{1-2}$alkylene-$R^x$. $R^x$ is $-CO_2H$, $-CONH_2$, - $CH_2NHC(O)NH_2$, or $-CH_2NHC(=NH)NH_2$.

$R^{10}$ is: (1) a $C_{1-8}$alkyl optionally substituted with one or more substituents selected from the group consisting of oxo, acetal, ketal, $-B(OH)_2$, boronic ester, phosphonate ester, ortho ester, -CH=CH-CHO, -CH=CH-C(O)$C_{1-4}$alkyl, - CH=CH-$CO_2C_{1-4}$alkyl, $-CO_2C_{1-4}$alkyl, $-CO_2H$, and $-CONH_2$ group; or (2) a $C_{1-4}$alkyl group substituted with one or two phenyl groups, or one naphthyl, imidazole, or indole group, wherein each phenyl is optionally substituted with one, two, or three substituents independently selected from -OH, fluoro, chloro, bromo, and iodo;

The symbol n is 0 or 1. The symbol p is 0 or 1.

X is: (1) a linker resulting from any of the meditope cyclization strategies discussed herein; (2) substituted alkylene, substituted heteroalkylene, substituted cycloalkylene, substituted heterocycloalkylene, substituted arylene or substituted heteroarylene or (3) $C_{1-8}$alkylene or $C_{2-8}$alkenylene, each carbon thereof optionally substituted with oxo, -C(O)-, $-NH_2$, -NHC(O)-or -NHC(O)$R^y$. One carbon of the X Ci-salkylene is optionally replaced with -C(O)NH-, a 5-membered heteroaryl ring, or -S-S-. $R^y$ is $-C_{1-4}$alkyl or $-CH(R^z)C(O)-$ or $-CH(R_z)CO_2H$. $R^z$ is -H or $-C_{1-4}$alkyl optionally substituted with -OH, -SH, or $-NH_2$. Formula VII includes all appropriate pharmaceutically acceptable salts. In (1), X is considered a substituted linker due to its chemical trivalency and because X may optionally include further substituents as set forth above (.e.g. $-NH_2$ and oxo). In some embodiments, X is:

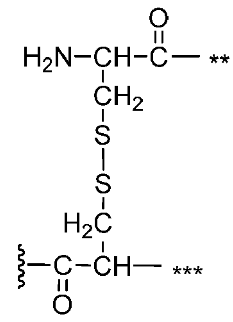
(X).

In Formula IX, ** represents the point of attachment to the glutamine attached to X in Formula VIII and *** represents the point of attachment to the nitrogen attached to X and lysine in Formula VIII. The symbol ⌇ denotes the point of attachment of X to the remainder of the molecule.

In some embodiments of the meditope of Formula VIII, m is 0, 1, or 2. In other embodiments, $R^3$ is H or phenyl and $R^{3'}$ is phenyl, 2-bromophenyl, 3-bromophenyl, or 4-bromophenyl. In further embodiments, $R^5$ is methyl, ethyl, propyl, isopropyl, butyl, isobutyl, sec-butyl, or tert-butyl, each optionally substituted with an oxo, $-B(OH)_2$, $-CO_2H$, or $-CONH_2$ group, or with one or two phenyl groups each optionally substituted with a bromo or chloro substituent. In further embodiments, $R^8$ is -OH, $-NH_2$, $-N(R^c)C(O)R^e$, or $-N(R^c)C(=NR^d)R^e$. In still further embodiments, $R^c$ is H or methyl, $R^d$ is H or $C_{1-4}$alkyl, and $R^e$ is $C_{1-4}$alkyl, or $-NH(C_{1-4}$alkyl). In other embodiments, $R^9$ is methyl or ethyl, optionally substituted with $-CO_2H$, $-CONH_2$, $- CH_2NHC(O)NH_2$, or $-CH_2NHC(=NH)NH_2$. In still other embodiments, $R^{10}$ is methyl, ethyl, propyl, isopropyl, butyl, isobutyl, sec-butyl, or tert-butyl, each optionally substituted with an oxo, $-B(OH)_2$, $-CO_2H$, or $-CONH_2$ group. In still other embodiments, -X-NH- is -Cys-Cys- (e.g. bound through a disulfide bridge), -Gly-Gly-, $-C(O)(CH_2)_6-NH-$, -β-Ala-β-Ala-, $-C(O)CH(NH_2)CH_2CH=CHCH_2CH(CO_2H)-NH-$, $-C(O)CH(NH_2)CH_2NHC(O)CH_2CH(CO_2H)-NH-$, -P-Ala-$C(O)CH_2CH(CO_2H)-NH-$, or
$-C(O)CH(NH_2)CH_2$-triazinyl-$CH_2-CH(CO_2H)-NH-$.

### *a. Modifications*

Based on the structural and thermodynamic data, multiple positions within the meditopes 1 and 2 described herein have been identified as target sites for modification, e.g., with different natural or non-natural amino acids, to enhance the overall binding affinity and/or to alter another property as described herein. Such modifications include, but are not limited to, modification of cQFD or cQYN to generate a head-to-tail cyclic lactam peptide, modification of Arg8, modification of position 3 (e.g., Phe3 of cQFD or variant thereof), modification ofLeu5, modification of Leu10, and/or incorporation of hydratable carbonyl functionality (see Figure 7). As demonstrated herein, mutation of each of Phe3, Leu5, and Arg8 to alanine in the original meditope of cQFD reduced the affinity of the resulting compounds for the meditope-enabled antibody binding interface by 10-140-fold. In some aspects, the variant meditopes include those having modifications at one or more of position 1, 3, 5, 8, 10, and 12 of the meditope of SEQ ID NO: 1 or 2, or other meditope listed in Table 1 or 2.

*Position 8*

In some embodiments, the meditope variants contain a modification in the position corresponding to position 8 (Arg8) of meditope 1 or 2. In the unmodified meditope (cQFD; SEQ ID NO: 1), Arg8 is extended, making a hydrogen bond with the heavy chain carbonyl of Q105 of the meditope-enabled antibody heavy chain. The immediate area about this residue is hydrophobic yet solvent exposed. In some aspects, the meditopes contain a modified residue at this position (e.g., modified Arg8). In some examples, the modified residue maintains the iminium functionality of the Arg8 residue useful for meditope-enabled antibody H-bonding, and introduces a substituted or unsubstituted hydrophobic arm to fill the cavity. Such modifications result in significant gains in binding, due to entropic increases, as supported by ligand docking calculations. Such modifications may be incorporated by using an N-alkyl guanidinium group, or an alkyl-amidine functionality. In either case, the substituted group of the terminal N-atom can be alkyl or aryl, wherein each position of the alkyl or aryl group may be optionally substituted with additional functionalities within the group including the terminal position. In one example, a modified Arginine (modified Arg8), having a structure as shown in Figure 6, is substituted for Arg8 of the meditope, e.g., of SEQ ID NO: 1 or 2 with the butyl group on the $NH_2$ (shown in Figure 6 as NHR). In some aspects, the variant meditope contains an n-butyl-arginine or butylamidine modification at position 8.

*Position 3*

In some embodiments, the meditope variants contain a modification in the position corresponding to position 3, such asPhe3 of meditope 1. The hydroxyl group of the meditope variant Phe3Tyr cQYN (SEQ ID NO: 2) has an alteration in the extended conformation of the Arg8 side chain as compared to cQFD (SEQ ID NO: 1). Data herein suggest the formation of a favorable hydrogen bond network, with water bound to the Fab. Enthalpy-driven optimization has proven successful in many small-molecule approaches in drug design and there are opportunities in the provided meditopes for engineering increases in entropy. In some embodiments, approaches resulting in enthalpic and/or entropic gains in meditope designs are used to generate the variant meditopes, e.g., to optimize binding.

For example, when bound to a meditope-enabled antibody, the hydrophobic phenyl ring of Phe3 is surrounded by a fairly polar array of side chain residues of the meditope-enabled antibody Fab. In some embodiments, one or more halogens is introduced on the phenyl ring of this residue, to allow for a halogen bonding interaction with the polar side chain residues. A halogen bond is a relatively strong non-covalent bond, similar to a hydrogen bond but involving the interaction of a halogen such as bromine or chlorine (or other halogen), with an oxygen atom. In some aspects, the residue at this position is modified to incorporate a halogen substituent. In some aspects, Phe3 is replaced with 2-bromo-, 3-bromo-, or 4-bromophenylalanine, in order to place a bromine atom in a position suitable for halogen bonding with a meditope-enabled antibody, e.g., at positions Tyr87 (light chain), Gln38, and/or Tyr91 (heavy chain) of a meditope-enabled antibody, respectively. Such phenylalanine derivatives are commercially available and in some aspects are incorporated into the cyclic peptide meditope variant by solid phase peptide synthesis (SPPS). Exemplary variant meditopes include those containing 2-bromo-L-phenylalanine, 3-bromo-L-phenylalanine, or 4-bromo-L-phenylalanine in the position corresponding to Phe3 of meditope 1.

In another example, the meditope incorporates an additional phenyl group at this position, for example, by replacing Phe3 with β,β'-diphenylalanine.

*Positions 5 and 10 (e.g., Leu5, Leu 10 of meditopes 1 or 2)*

In some embodiments, the meditope variants contain a modification in the position corresponding to position 5 or 10 (LeuS or Leu10) of meditopes 1 or 2. As shown herein, the side chains of Leu5 and Leu10 of meditope 1 make hydrophobic contacts to the meditope-enabled Fab, cetuximab. In certain embodiments, one or more properties of the meditopes, e.g., affinity, are altered by incorporating a different natural amino acid, or a non-natural amino acid at one or both of these positions, e.g., thereby changing the amount of surface area that can be extended. In one embodiment, natural amino acids (Phe/Tyr/Trp) and non-natural analogs (e.g., β,β'-diphenyl-L-alanine, or amino acids incorporating side chains that include branched alkyl, extended aromatics such as napthyl, or other hydrophobic groups) are systematically introduced via SPPS at one or both positions.

*"Hydratable " functionality*

In certain embodiments, one or more Fab hydroxyl-bearing side chains surrounding the meditope-binding site of a meditope-enabled antibody is/are exploited through selective trapping, by formation of a covalent bond with a meditope that incorporates a hydratable functionality. Thus, in some embodiments, the meditope contains one or more residues with hydratable substituents, e.g., in order to create a highly selective but irreversible interaction with a meditope-enabled antibody or other substance. For example, Arg8 of the meditope 1 extends in proximity to Ser43 of the light chain (3.5 Å) and Tyr91 (3.8 Å) of the meditope-enabled antibody heavy chain, according to Kabat numbering. Incorporation of a hydratable functionality at the end of Arg8 or Leu10 of the meditope would allow for selective formation of a serine or tyrosine hemi-acetal. Such a covalent adduct would essentially afford irreversible binding. In addition, residues containing boronic acid may also be integrated into the meditope as a hydratable group. Boronic acid plays an important role in the structural activity of bortezomib (Velcade®), which is used to treat multiple myeloma. Further representative examples of hydratable residues are also shown in Figure 6, where R = $-CH_2CHO$ or $-CH_2B(OH)_2$. In some examples, such variants are prepared by incorporating residues containing such groups using SPPS.

Such hydratable strategies can be applied to engineering new amino acid residues within the antibody Fab-meditope binding site and introducing the requisite complementary "hydratable" functionalities within different positions of the meditope. A similar strategy can be applied by introducing cysteine residues into the Fab region and use this

functionality for nucleophilic attack on a "hydratable" functionality such as an electrophilic carbonyl group or derivative thereof contained within the meditope or for making selective disulfide bonds (-S-S-) between the Fab region and the meditope containing the requisite thiol or thiol equivalent functionalities. Other embodiments of this idea would include introducing Michael acceptors contained in the meditope such as $\alpha,\beta$-unsaturated carbonyl functionalities. These functionalities are well-known to selectively react with thiols to form stable covalent carbon-sulfur bonds.

***Alternative cyclization strategies and replacement of disulfide bridge***

In certain embodiments, the variant meditopes include a disulfide bridge, as in cQFD and cQYN. Disulfide bridges may be formed by the reaction of the side chains of two cysteine residues. In certain embodiments, the disulfide bridge in a meditope, e.g., meditope 1 or 2, is replaced with an alternative linkage or is removed. Thus, among the variant meditopes are those having alternative linkages or lacking the disulfide bridge of the original meditopes.

In some aspects, the linkage is made between one or more unnatural amino acids within the amino acid chain. Examples of linkages that may be made with unnatural amino acids include linkages comprising (i) stable hydrazone or oxime-based linkages made by reaction of a residue comprising an aldehyde or ketone with a residue comprising an amine group, where the amine nitrogen is substituted with -NH2 or alkyloxy group (e.g., reaction of a p-acetylphenylalanine, m-acetylphenylalanine, or p-(3-oxobutanoyl)-L-phenylalanine residue with a p-(2-amino-3-hydroxyethyl) -phenylalanine residue), (ii) thiol reactive by incorporating phenylselenidylalanine, (iii) a UV crosslinker containing benzophenone by incorporating p-benzoyl-L-phenylalanine, (iv) amine reactive by incorporating p-isopropylthiocarbonyl-phenylalanine or p-ethylthiocarbonyl-phenylalanine, (v) heterocyclic linkages, such as a triazine, thiazole, thiazolidine, or oxazole linkage, made, for example, by reaction of a residue containing an azide group with a residue containing an alkyne group via Huisgen cycloaddition (e.g., reaction of a p-propargyloxyphenylalanine residue with a p-azidophenylalanine residue); (v) an amide bond made by reaction of an acid group in one residue with an amine group in another residue; (vi) an ester bond made by reaction of an acid group in one residue with an alcohol in another residue, such as a serine; (vii) a double bond, made by reaction of two residues each containing a terminal olefin, e.g., by olefin metathesis (e.g., reaction of two allylglycine residues or two N-allyl substituted amino acids), or (viii) by reaction of any other pair of suitable residues known in the art. For a review, see, for example, Davies, J.S., "The Cyclization of Peptides and Depsipeptides," J. Peptide Sci. 2003, 9, 471-501. In one embodiment, the meditope may direct a reactive group to an unnatural amino acid incorporated into the Fab, such as p-acetylphenylalanine.

Various methods for cyclization of a meditope may be used, e.g., to address *in vivo* stability and to enable chemoselective control for subsequent conjugation chemistry. In some embodiments, the cyclization strategy is a lactam cyclization strategy, including head-to-tail (head-tail) lactam cyclization (between the terminal residues of the acyclic peptide) and/or lactam linkage between other residues. Lactam formation may also be effected by incorporating residues such as glycine, $\beta$-Ala, 7-aminoheptanoic acid, and the like, into the acyclic meditope cyclization precursors to produce different lactam ring sizes and modes of connectivity. Additional cyclization strategies such as "click" chemistry and olefin metathesis also can be used (see Figure 7, right boxes). Such methods of peptide and peptidomimetic cyclization are well known in the art.

In some embodiments, the meditopes containing lactam linkages are more stable, *in vivo,* e.g., have a linkage that is more stable *in vivo* compared to meditopes with other linkages.

In some embodiments, the terminal residues of an acyclic peptide are reacted to form a cyclic meditope, e.g., cyclic meditope variant. In other embodiments, other positions are amenable to cyclization, including between residues 3 and 11 and 4 and 11. Thus, in some aspects, the meditopes contain a linkage formed between residues other than the N-terminal and C-terminal residues, such as between residues 3 and 11 and/or 4 and 11, e.g., of a 12-amino acid peptide.

In some embodiments, the meditopes, e.g., variant meditopes, contain a reactive amine functionality (e.g., Lys11), which can be used for subsequent conjugation of the meditope variant, e.g., to a scaffold or linker or to an agent, such as a diagnostic, e.g., imaging, agent or therapeutic agent as described herein. For example, Figure 5 shows a procedure for conjugation of a meditope variant with fluorescein for FACS analysis; this strategy can be applied to other imaging and other agents, including DOTA for *in vivo* PET imaging.

***Characterization of meditopes***

In some embodiments, the meditopes, such as variant meditopes, are characterized, for example, by ITC, SPR and/or diffraction and/or other methods, such as those described herein in the Examples. In one example, the synthesis of meditopes and characterization thereof is carried out in an iterative fashion, e.g., such that the meditope variant with the most desirable property, e.g., highest affinity for one or more meditope-enabled antibodies or other desired property, such as pH dependence, is subsequently modified to improve the desired property.

In one example, for characterization of binding of meditopes to meditope-enabled antibodies, the meditope is

purified to >95% homogeneity and structurally characterized by mass spectrometry. Peptides are dialyzed in water, their concentrations measured by UV-Vis and calibrated with elemental analysis, and diluted (>100x) into the appropriate buffer. Binding to a meditope-enabled antibody is rigorously characterized by ITC, SPR, X-ray diffraction, or a combination thereof. ITC measurements may be performed on a TA Instruments nanoITC, with only 1-2 mg of peptide per measurement. In one example, for SPR measurements, low density and high density chips are conjugated with a meditope-enabled antibody, e.g., a Fab or a full IgG. In some cases, the chips are first characterized using an antigen, such as a soluble fragment of the entire extracellular domain of EGFR (residues 1-621) in the case of cetuximab. In a study reported herein, the cQFD meditope bound with similar enthalpy and entropy to the cetuximab Fab fragment as compared to the fully intact cetuximab IgG, e.g., as measured by SPR and ITC. Accordingly, binding measurements may be carried out using the full IgG or Fab fragment of cetuximab or other meditope-enabled antibody. In one example, for diffraction, the co-crystallization conditions of the cetuximab Fab fragment and the meditope of SEQ ID NO: 1 are well-established and diffraction quality crystals are typically obtained in 1 to 3 days, typically 1 day. Full data sets are collected in 8 to 12 hours with an in-house source (Rigaku 007-HF and an R-Axis IV++) and in 10 min at the Stanford Synchrotron Radiation Lightsource, which allows for rapid characterization of the interactions of the meditope variants with meditope-enabled antibodies.

In some aspects, ITC, SPR and X-ray diffraction data, e.g., collectively, provide atomic detail to guide subsequent chemical modifications and ultimately improve the affinity of the meditopes and/or make other alterations to the meditopes. A calculation based on $\Delta G = -RT \ln Ka$ shows that the difference between micromolar and nanomolar affinity of a meditope for cetuximab results from a change in free energy at 300 K of ~4 kCal/mol, which is on the order of a strong hydrogen bond. Thus, the loss of an ordered water molecule from a protein binding pocket or the reorientation of an amino acid residue-chain may be sufficient to alter binding by orders of magnitude.

In some examples, other approaches are used to alter properties of the meditopes, e.g., to improve the affinity of the meditope-Fab interaction. In one example, structural data, such as those obtained in the studies described above, may be used to replace residues in the Fab, by mutagenesis, for example, to add additional hydrogen bonds, substitute amino acids for unnatural amino acids or alter the hydrophobic interface, for example, in ways that might better complement meditope binding. In some examples, fluorescence polarization assays are used to identify meditope variants that can displace a given meditope, such as SEQ ID NO: 1. In other examples, the same technique is used to identify small molecules that can displace the meditope and then use these small molecules as templates to further improve the binding affinity of the meditopes.

In some examples, the meditope variants are designed based on pH dependence, e.g., to have differing affinities at different pH conditions. Thus, in certain embodiments, the meditope enabled antibody -meditope interaction is tailored with respect to pH. Examples of such meditopes are described herein. In some aspects, the binding affinities of the meditope variants are measured as a function of buffer pH. For example, variant meditopes include meditopes with a binding affinity for one or more meditope-enabled antibody or fragment that is decreased at a lysosomal pH level or is increased in a hypoxic environment. For example, provided are variant meditopes that exhibit higher binding affinity for a meditope-enabled antibody at a neutral pH (e.g., pH 7-8, e.g., pH 7.3-7.5) and exhibit a relatively lower binding affinity for the antibody at a more acidic pH, such as a pH between at or about 4 and 6.5, e.g., at an endosomal pH (e.g., between at or about 5 and at or about 6.5) or at a lysosomal pH (e.g., between at or about 4.5 and at or about 5). In another example, the meditopes have increased binding affinity for the antibody in a hypoxic environment, such as a tumor environment, for example, as compared to the affinity observed under other physiological conditions, such as in the blood. In some embodiments, modification of meditope variants or meditope "analogs" for the specific release at low pHs (e.g., in lysosomes for drug delivery) is provided; and modification of meditopes so that they bind with higher affinity in a hypoxic environment (e.g., tumor stroma pH is often lower than normal tissues).

In another example, a meditope may have decreased or limited binding affinity to a meditope enabled antibody at a first pH, and increased binding affinity at a second pH. In some embodiments, the first pH may be a non-physiological pH and the second pH may be a physiological pH or the pH of a target tissue or disease condition. In some embodiments, the meditope and meditope enabled antibody can be manufactured or stored together in a single composition at the first pH such that the meditope and meditope enabled antibody exhibit reduced or limited crosslinking during manufacture or storage. Thus, the meditope and meditope enabled antibody may remain relatively soluble and unbound in solution, but bind, form clusters, and increase co-localization after use or administration at a second pH, including under physiological conditions. Also provided are methods for generating such meditope variants.

According to some embodiments, the meditope binding site may be exploited or optimized to enhance binding of, purification of, and/or imaging or other method using the meditope-enabled antibodies and functional fragments thereof. In a separate embodiment, a meditope may contain one or more cysteine residue that binds to one or more engineered cysteine in the Fab at the meditope binding site (e.g., ThioMAbs). The meditope is thereby conjugated to any diagnostic and/or therapeutic substance, molecule or compound. For example, the substance may be a small molecule diagnostic molecule, such as a marker. The "Cys meditope" directs the conjugate to the antibody and binds via a covalent linkage. Alternatively, the meditope may be conjugated to the Fab to one or more unnatural amino acids

that are incorporated into the meditope binding site.

## II. Meditope-agent conjugates

In certain embodiments, the multivalent meditopes are conjugated to one or more therapeutic or diagnostic agent, e.g., imaging agents, therapeutically effective agents or compounds in therapeutically effective amounts or both. Provided are such complexes, in some embodiments comprising a plurality of meditopes, one or more linkers, and one or more agents.

The current disclosure contemplates the use of a multivalent meditope or variant thereof conjugated to a therapeutic or diagnostic (e.g., imaging) agent or compound to treat, prevent, diagnose or monitor a disease or condition. In one aspect, such conjugation of a multivalent meditope, to an agent, when used in connection with meditope-enabled monoclonal antibodies, provides a highly versatile platform technology that will significantly improve mAb based therapeutics and imaging methods to treat and detect disease (see Figure 1).

Agents can be conjugated by any means known in the art. In some embodiments, the agent is conjugated to a lysine, tyrosine, glutamic acid, aspartic acid, formylglycine or aldehyde tag, an unnatural amino acid, etc. A formylglycine or aldehyde tag can comprise a 6-amino acid (LCTPSR; SEQ ID NO: 247) or 13-amino acid (LCTPSRGSLFTGR, SEQ ID NO: 248) sequence that can be recognized and targeted by formylglycine-generating enzyme (FGE) to generate a reactive aldehyde group. The reactive aldehyde group can be useful in subsequence reactions comprising conjugating meditopes or multivalent meditopes to agents. See Carrico et al., Nature Chemical Biology 3, 321-322 (2007).

The diagnostic and therapeutic agents include any such agent, which are well-known in the relevant art. Among the imaging agents are fluorescent and luminescent substances, including, but not limited to, a variety of organic or inorganic small molecules commonly referred to as "dyes," "labels," or "indicators." Examples include fluorescein, rhodamine, acridine dyes, Alexa dyes, and cyanine dyes. Enzymes that may be used as imaging agents in accordance with the embodiments of the disclosure include, but are not limited to, horseradish peroxidase, alkaline phosphatase, acid phosphatase, glucose oxidase, β-galactosidase, β-glucoronidase or β-lactamase. Such enzymes may be used in combination with a chromogen, a fluorogenic compound or a luminogenic compound to generate a detectable signal.

Radioactive substances that may be used as imaging agents in accordance with the embodiments of the disclosure include, but are not limited to, $^{18}F$, $^{32}P$, $^{33}P$, $^{45}Ti$, $^{47}Sc$, $^{52}Fe$, $^{59}Fe$, $^{62}Cu$, $^{64}Cu$, $^{67}Cu$, $^{67}Ga$, $^{68}Ga$, $^{77}As$, $^{86}Y$, $^{90}Y$. $^{89}Sr$, $^{89}Zr$, $^{94}Tc$, $^{94}Tc$, 99mTc, $^{99}Mo$, $^{105}Pd$, $^{105}Rh$, $^{111}Ag$, $^{111}In$, $^{123}I$, $^{124}I$, $^{125}I$, $^{131}I$, $^{142}Pr$, $^{143}Pr$, $^{149}Pm$, $^{153}Sm$, $^{154\text{-}1581}Gd$, $^{161}Tb$, $^{166}Dy$, $^{166}Ho$, $^{169}Er$, $^{175}Lu$, $^{177}Lu$, $^{186}Re$, $^{188}Re$, $^{189}Re$, $^{194}Ir$, $^{198}Au$, $^{199}Au$, $^{211}At$, $^{211}Pb$, $^{212}Bi$, $^{212}Pb$, $^{213}Bi$, $^{223}Ra$ and $^{225}Ac$. Paramagnetic ions that may be used as additional imaging agents in accordance with the embodiments of the disclosure include, but are not limited to, ions of transition and lanthanide metals (e.g. metals having atomic numbers of 21-29, 42, 43, 44, or 57-71). These metals include ions of Cr, V, Mn, Fe, Co, Ni, Cu, La, Ce, Pr, Nd, Pm, Sm, Eu, Gd, Tb, Dy, Ho, Er, Tm, Yb and Lu.

When the imaging agent is a radioactive metal or paramagnetic ion, the agent may be reacted with another long-tailed reagent having a long tail with one or more chelating groups attached to the long tail for binding to these ions. The long tail may be a polymer such as a polylysine, polysaccharide, or other derivatized or derivatizable chain having pendant groups to which the metals or ions may be added for binding. Examples of chelating groups that may be used according to the disclosure include, but are not limited to, ethylenediaminetetraacetic acid (EDTA), diethylenetriaminepentaacetic acid (DTPA), DOTA, NOTA, NETA, TETA, porphyrins, polyamines, crown ethers, bis-thiosemicarbazones, polyoximes, and like groups. The chelate is normally linked to the PSMA antibody or functional antibody fragment by a group, which enables the formation of a bond to the molecule with minimal loss of immunoreactivity and minimal aggregation and/or internal crosslinking. The same chelates, when complexed with non-radioactive metals, such as manganese, iron and gadolinium are useful for MRI, when used along with the antibodies and carriers described herein. Macrocyclic chelates such as NOTA, DOTA, and TETA are of use with a variety of metals and radiometals including, but not limited to, radionuclides of gallium, yttrium and copper, respectively. Other ring-type chelates such as macrocyclic polyethers, which are of interest for stably binding nuclides, such as $^{223}Ra$ for RAIT may be used. In certain embodiments, chelating moieties may be used to attach a PET imaging agent, such as an Al-$^{18}F$ complex, to a targeting molecule for use in PET analysis.

Exemplary therapeutic agents include, but are not limited to, drugs, chemotherapeutic agents, therapeutic antibodies and antibody fragments, toxins, radioisotopes, enzymes (e.g., enzymes to cleave prodrugs to a cytotoxic agent at the site of the tumor), nucleases, hormones, immunomodulators, antisense oligonucleotides, RNAi molecules (e.g., siRNA or shRNA), chelators, boron compounds, photoactive agents and dyes. The therapeutic agent may also include a metal, metal alloy, intermetallic or core-shell nanoparticle bound to a chelator that acts as a radiosensitizer to render the targeted cells more sensitive to radiation therapy as compared to healthy cells. Further, the therapeutic agent may include paramagnetic nanoparticles for MRI contrast agents (e.g., magnetite or $Fe_3O_4$) and may be used with other types of therapies (e.g., photodynamic and hyperthermal therapies and imaging (e.g., fluorescent imaging (Au and CdSe)).

Chemotherapeutic agents are often cytotoxic or cytostatic in nature and may include alkylating agents, antimetabolites, anti-tumor antibiotics, topoisomerase inhibitors, mitotic inhibitors hormone therapy, targeted therapeutics and immunotherapeutics. In some embodiments the chemotherapeutic agents that may be used as therapeutic agents in accordance with the embodiments of the disclosure include, but are not limited to, 13-cis-retinoic acid, 2-chlorodeoxyadenosine, 5-azacitidine, 5-fluorouracil, 6-mercaptopurine, 6-thioguanine, actinomycin-D, adriamycin, aldesleukin, alemtuzumab, alitretinoin, all-transretinoic acid, alpha interferon, altretamine, amethopterin, amifostine, anagrelide, anastrozole, arabinosylcytosine, arsenic trioxide, amsacrine, aminocamptothecin, aminoglutethimide, asparaginase, auristatin, dimethylvaline-valine-dolaisoleuine-dolaproine-phenylalanine-p-phenylenediamine (AFP), dovaline-valine-dolaisoleuine-dolaproine-phenylalanine (MMAF), monomethyl auristatin E (MMAE), auromycins, azacytidine, bacillus calmetteguerin (BCG), bendamustine, bevacizumab, etanercept, bexarotene, bicalutamide, bismuth, bortezomib, bleomycin, busulfan, calcium leucovorin, citrovorum factor, capecitabine, canertinib, carboplatin, carmustine, cetuximab, chlorambucil, cisplatin, cladribine, cortisone, cyclophosphamide, cytarabine, cc1065, darbepoetin alfa, dasatinib, daunomycin, decitabine, denileukin diftitox, dexamethasone, dexasone, dexrazoxane, dactinomycin, daunorubicin, decarbazine, docetaxel, dolostatins, doxorubicin, doxifluridine, eniluracil, epirubicin, epoetin alfa, erlotinib, everolimus, exemestane, estramustine, ethidium bromide, etoposide, filgrastim, fluoxymesterone, fulvestrant, flavopiridol, floxuridine, fludarabine, fluorouracil, flutamide, gefitinib, gemcitabine, gemtuzumab ozogamicin, goserelin, granulocyte - colony stimulating factor, granulocyte macrophage-colony stimulating factor, hexamethylmelamine, hydrocortisone hydroxyurea, ibritumomab, interferon alpha, interleukin - 2, interleukin-11, isotretinoin, ixabepilone, idarubicin, imatinib mesylate, ifosfamide, irinotecan, lapatinib, lenalidomide, letrozole, leucovorin, leuprolide, liposomal Ara-C, lomustine, mechlorethamine, megestrol, melphalan, mercaptopurine, mesna, methotrexate, methylprednisolone, mitomycin C, mitotane, mitoxantrone, maytonsinoids, nelarabine, nilutamide, octreotide, oprelvekin, oxaliplatin, paclitaxel, pamidronate, pemetrexed, panitumumab, PEG Interferon, pegaspargase, pegfilgrastim, PEG-L-asparaginase, pentostatin, plicamycin, prednisolone, prednisone, procarbazine, raloxifene, ricin, ricin A-chain, rituximab, romiplostim, ralitrexed, sapacitabine, sargramostim, satraplatin, sorafenib, sunitinib, semustine, streptozocin, taxol, tamoxifen, tegafur, tegafur-uracil, temsirolimus, temozolamide, teniposide, thalidomide, thioguanine, thiotepa, topotecan, toremifene, tositumomab, trastuzumab, tretinoin, trimitrexate, alrubicin, vincristine, vinblastine, vindestine, vinorelbine, vorinostat, yttrieum, or zoledronic acid.

Therapeutic antibodies and functional fragments thereof, that may be used as therapeutic agents in accordance with the embodiments of the disclosure include, but are not limited to, alemtuzumab, bevacizumab, cetuximab, edrecolomab, gemtuzumab, ibritumomab tiuxetan, panitumumab, rituximab, tositumomab, and trastuzumab and other antibodies associated with specific diseases listed herein.

Toxins that may be used as therapeutic agents in accordance with the embodiments of the disclosure include, but are not limited to, ricin, abrin, ribonuclease (RNase), DNase I, Staphylococcal enterotoxin-A, pokeweed antiviral protein, gelonin, diphtheria toxin, Pseudomonas exotoxin, and Pseudomonas endotoxin.

Radioisotopes that may be used as therapeutic agents in accordance with the embodiments of the disclosure include, but are not limited to, $^{32}$P, $^{89}$Sr, $^{90}$Y. $^{99m}$Tc, $^{99}$Mo, $^{131}$I, $^{153}$Sm, $^{177}$LU, $^{186}$Re, $^{213}$Bi, $^{223}$Ra and $^{225}$Ac.

**C. Meditope Enabled Antibodies**

Provided are meditope-enabled antibodies and antigen binding fragments thereof that are capable of binding to one or more meditopes via meditope-binding sites. In some cases, the meditope-enabled antibody binds to a cyclic peptide of SEQ ID NO: 1 or 2 (meditope 1 or 2) and/or to one or more variants thereof, such as meditopes 1, 2, 16-18, 23, 29, 31, 32, 36, 39, 42, 43, 45, 46, 51, 52, 54, or 55 (meditopes based on peptides having the sequences set forth in SEQ ID NOs: 1, 2, 16-18, 23, 29, 31, 32, 36, 39, 42, 43, 45, 46, 51, 52, 54, or 55), or in some cases, any of meditopes 1, 2, or 15-55 and/or meditopes of any of SEQ ID NOs: 186-190 and 207. Among the provided meditope-enabled antibodies are those that bind to a meditope or meditopes with an affinity similar to that of cetuximab. For example, in certain aspects, the antibodies bind to the meditope(s) with a dissociation constant of less than at or about 10 $\mu$M, less than at or about 5 $\mu$M, or less than at or about 2 $\mu$M, less than at or about 1 $\mu$M, less than at or about 500, 400, 300, 200, 100 nM, or less, such as at or about 200 picomolar or less. In some cases, the dissociation constant, such as any of those listed herein, is that measured using a particular technique, such as surface plasmon resonance (SPR), isothermal titration calorimetry (ITC), fluorescence, fluorescence polarization, NMR, IR, calorimetry titrations; kinetic exclusion; circular dichroism, differential scanning calorimetry, or other known method. For example, in some cases, the analog or meditope exhibits a binding constant of less than at or about 10 $\mu$M, less than at or about 5 $\mu$M, or less than at or about 2 $\mu$M, less than at or about 1 $\mu$M, less than at or about 500, 400, 300, 200, 100 nm, or less, as measured by SPR or as measured by ITC or as measured by any of these methods.

In some examples, the meditope-binding site is a structural feature of the monoclonal antibody cetuximab. Thus, in some cases, the meditope-binding site contains residues corresponding to those within the meditope binding site of cetuximab. X-ray crystallographic analysis has revealed that the peptides of SEQ ID NO: 1 binds to a meditope-binding site within the central cavity of the cetuximab Fab fragment, defined by various residues of the heavy and light

chains (see Figures 8 and 9A), with a binding constant of ~700 nM.

In certain embodiments, the unique interactions between the meditopes and the cetuximab binding site are exploited to generate additional meditope-enabled antibodies. In some embodiments, the meditope-enabled antibodies are generated by modifying an antibody other than cetuximab (sometimes referred to as the template antibody), such as an antibody having one or more CDRs distinct from those of cetuximab, to confer the ability to bind to one or more of the provided meditopes, such as a meditope of SEQ ID NO: 1 or 2, or variant thereof. The template antibody can be a human or humanized antibody or a mouse antibody. In one aspect, the modifications include substituting residues within the central cavity of the Fab fragment, typically within the framework regions (FRs) of the heavy and light chain variable regions and/or the constant regions to render the template antibody meditope-enabled. For example, where the template antibody is a human or humanized antibody, the modifications generally include substitutions at residues within the heavy and light chain variable region FRs. In some embodiments, such residues are replaced with the corresponding residue present in cetuximab, or comparable amino acid. Thus, in certain embodiments, residues within the FRs of a human or humanized antibody are replaced with corresponding murine residues; in certain embodiments, they are replaced by other residues, such as those having similar functional groups or moieties for interacting with the meditopes. Typically, the residues replaced by corresponding murine (or other) residues are found within the central Fab cavity, and thus are not exposed to the immune system. As such, in some embodiments, introducing these amino acid substitutions in a human or humanized antibody do not increase or do not substantially increase the antigenicity of the modified template antibody, in the context of delivery to a human subject. In addition, antigenicity prediction algorithms may be further used to indicate that the human sequence with the point mutations should not be antigenic.

In some embodiments, the one or more residues that are replaced, are selected from light chain framework residues 10, 39-43, 45, 83, 85, 100 and/or 104, according to Kabat numbering (see Kabat E.A., et al. (1991) Sequences of Proteins of Immunological Interest, Fifth Edition. NIH Publication No. 91-3242, incorporated herein by reference in its entirety), and/or heavy chain framework residue numbers 40, 89 and/or 105, according to Kabat numbering. In general, unless otherwise specified, amino acid positions in the heavy or light chain of an antibody refer to Kabat numbering. Also encompassed within the present disclosure are residues in other antibodies corresponding to the residues of cetuximab described herein, such as those within the cetuximab meditope-binding site. In some embodiments, residues in the template antibody corresponding to light chain residues 9, 10, 39, 40, 41, 42, 43, 45, 83, 85, 100, and/or 104, and/or heavy chain residues 40, 89, and/or 105, are replaced, for example, with amino acids present at those positions within cetuximab. In some embodiments, the meditope-enabled antibody contains, within its light chain framework regions (or within one or more such FRs, e.g., FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of the template antibody; and/or contains, within its heavy chain framework regions (or within one or more such FRs, e.g., FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H(s)) of the template antibody; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of the template antibody; and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL regions of the template antibody. In some embodiments, the antibody contains all or at least 95, 96, 97, 98, or 99 % identity within one or more, e.g., all, of the CDRs within the heavy and/or light chain, compared to the template antibody. In some aspects, the antibody contains 1, 2, 3, 4, 5, 6, 7, 8, 9, 10, 11, 12, 13, 14, 15, 16, 17, 18, 19, or 20 modifications within the light chain (e.g., FR-Ls) compared to the template antibody and/or contains 1, 2, 3, 4, 5, 6, 7, 8, 9, 10, 11, 12, 13, 14, 15, 16, 17, 18, 19, or 20 modifications within the heavy chain (e.g., FR-Hs) compared to the template antibody.

In one embodiment, the one or more residues replaced are light chain framework residues including, but not limited to, 40, 41, 83 and 85, according to Kabat numbering. In one embodiment, light chain residue 40 is replaced with threonine; light chain residue 41 is replaced with asparagine, light chain residue 83 is replaced with isoleucine or valine, and/or light chain residue 85 is replaced with aspartate. In one embodiment, light chain residue 40 is replaced with threonine; light chain residue 41 is replaced with asparagine, light chain residue 83 is replaced with glutamate, and/or light chain residue 85 is replaced with aspartate. In a particular example, light chain framework Pro40 is replaced with Thr (P40T) or Ser (P40S), light chain framework Gly41 is replaced with Asn (G41N), light chain framework residue Phe83 is replaced with Ile (F83I) or Val (F83V) or glutamate (F83E) and light chain framework residue Thr85 is replaced with Asp (T85D) or Asn (T85N).

Thus, among the provided meditope-enabled antibodies are antibodies having one or more modifications, typically amino acid substitutions, at residues that correspond to positions within the meditope binding site of cetuximab or other meditope-enabled antibody, such as those described herein, including meditope-enabled trastuzumab and meditope-enabled M5A. Among the antibodies are those having a VL region with a threonine, serine, or aspartate at position 40, a residue other than glycine at position 41, and an aspartate or asparagine at position 85, according to Kabat numbering, for example, an antibody with a VL region having a threonine at position 40, an asparagine at position 41,

and an aspartate at position 85. In some embodiments, the antibody has a VH region with a serine at position 40 and an isoleucine at position 89 and/or a VH region with a serine or proline at position 40 and an isoleucine, tyrosine, methionine, phenylalanine, or tryptophan at position 89, according to Kabat numbering. In some embodiments, the VL region has an isoleucine or leucine at position 10, and/or an isoleucine at position 83 or a glutamate at position 83. In some embodiments, the VL region has a valine or isoleucine at position 9 and/or a residue other than glutamine at position 100.

In some examples, the VL region has a valine or isoleucine at position 9, an isoleucine or leucine at position 10, an arginine at position 39, a threonine at position 40, an asparagine at position 41, a glycine at position 42, a serine at position 43, an isoleucine or glutamate at position 83, an aspartate at position 85, and an alanine at position 100; and the VH region has a serine at position 40 and an isoleucine at position 89, according to Kabat numbering. In some examples, the VL region does not contain a proline at position 40, a glycine at position 41, or a threonine at position 85, according to Kabat numbering, and/or the VH region does not contain an asparagine or alanine at position 40 or a valine at position 89, according to Kabat numbering. In some examples, the VL region does not contain an serine at position 10, a proline at position 40, a glycine at position 41, an phenylalanine at position 83, or a threonine at position 85, according to Kabat numbering, and/or the VH region does not contain an asparagine or alanine at position 40 or a valine at position 89, according to Kabat numbering.

In some aspects, the antibody has a light chain having P8 , V9 or I9, I10 or L10, Q38, R39, T40, N41 G42, S43, P44, R45, D82, I83, A84, D85, Y86, Y87, G99, A100, G101, T102, K103, L104, E105, R142, S162, V163, T164, E165, Q166, D167, S168, and Y173, according to Kabat numbering, and/or a heavy chain having Q6, P9, R38, Q39, S40, P41, G42, K43, G44, L45, S84, D86, T87, A88, I89, Y90, Y91, W103, G104, Q105, G106, T107, L108, V109, T110, V111, Y147, E150, P151, V152, T173, F174, P175, A176, V177, Y185, S186, and L187, according to Kabat numbering.

In some aspects, the antibody has a light chain having P8 , V9 or I9, I10 or L10, Q38, R39, T40, N41 G42, S43, P44, R45, D82, E83, A84, D85, Y86, Y87, G99, A100, G101, T102, K103, L104, E105, R142, S162, V163, T164, E165, Q166, D167, S168, and Y173, according to Kabat numbering, and/or a heavy chain having Q6, P9, R38, Q39, S40, P41, G42, K43, G44, L45, S84, D86, T87, A88, I89, Y90, Y91, W103, G104, Q105, G106, T107, L108, V109, T110, V111, Y147, E150, P151, V152, T173, F174, P175, A176, V177, Y185, S186, and L187, according to Kabat numbering.

In some embodiments, the meditope-enabled antibody is generated by mutating 1, 2, 3, 4, 5, 6, 7, 8, 9, 10, or 11 residues in the light chain of an antibody, such as a human or humanized antibody, e.g., trastuzumab, for example, to resemble the corresponding positions in cetuximab (based on Kabat numbering). In some aspects, such an antibody contains mutated residues at positions 9, 10, 39, 40, 41, 42, 43, 45, 83, 85, and 100 of the light chain, based on Kabat numbering, compared to the human or humanized sequence. In one aspect, the light chain of the meditope-enabled antibody contains: V9 or I9, I10 or L10, R39, T40, N41 G42, S43, R45, I83 or E83, D85, and A100, e.g.: V9, I10, R39, T40, N41, G42, S43, R45, I83, D85, and A100, based on Kabat numbering. In some embodiments, the antibody is otherwise human or humanized.

In some embodiments, the meditope-enabled antibody is generated by mutating 1, 2, 3, 4, 5, 6, 7, 8, 9, 10, or 11 residues such as 1 or 2 residues in the heavy chain of an antibody, such as a human or humanized antibody, e.g., trastuzumab, for example, to resemble the corresponding positions in cetuximab. In some aspects, such an antibody contains mutated residues at positions 40 and 89 in the heavy chain, based on Kabat numbering, compared to the human or humanized sequence. In one aspect, the heavy chain contains S40 and I89, based on Kabat numbering. In some embodiments, the antibody is otherwise human or humanized.

In some embodiments the meditope-enabled antibody contains 13 mutations, e.g., compared to the humanized or human sequence. In one aspect, the meditope-enabled antibody contains a light chain having 11 mutations and a heavy chain having two mutations, compared to the humanized or human sequence. In some aspects, the antibody contains V9 or I9, I10 or L10, R39, T40, N41 G42, S43, R45, I83, D85, and A100 in the light chain and S40 and I89 in the heavy chain, based on Kabat numbering. In some aspects, the antibody contains V9 or I9, I10 or L10, R39, T40, N41 G42, S43, R45, E83, D85, and A100 in the light chain and S40 and I89 in the heavy chain, based on Kabat numbering. In some aspects, the antibody contains V9, I10, R39, T40, N41 G42, S43, R45, I83, D85, and A100 in the light chain and S40 and I89 in the heavy chain, based on Kabat numbering. In some aspects, the antibody contains V9, I10, R39, T40, N41 G42, S43, R45, E83, D85, and A100 in the light chain and S40 and I89 in the heavy chain, based on Kabat numbering. In some embodiments, the antibody and/or the framework regions thereof is or are otherwise human or humanized.

In other embodiments, the meditope-enabled antibodies are generated via CDR grafting, typically by modifying one or more complementarity determining region (CDR) (e.g., one or more of CDRs 1-3) of the heavy and/or light chain of a meditope-enabled antibody, such as any of the meditope-enabled antibodies described herein, to replace them with other CDRs, such as CDRs of existing or new antibodies. CDR grafting is standard practice for producing humanized monoclonal antibodies, e.g., by grafting CDRs of an antibody generated in a non-human species, such as mouse, onto a human antibody framework. See U.S. Patent Nos. 5,558,864 and 8,133,982; Kettleborough et al., "Humanization of a mouse monoclonal antibody by CDR-grafting: the importance of framework residues on loop conformation," Protein

Eng., 4:773-783 (1991). Thus, in certain embodiments, the antigen specificity of a meditope-enabled antibody is altered by grafting the CDRs of preexisting or newly-generated antibodies of interest. Also among the provided meditope-enabled antibodies are such CDR-grafted meditope-enabled antibodies.

In some embodiments, the meditope-enabled antibodies are generated, using one of the antibodies disclosed herein (e.g., cetuximab, meditope-enabled trastuzumab, or meditope-enabled M5A (anti-CEA) antibody) as a template sequence, and carrying out one or more known antibody engineering methods to alter it, for example, to alter its antigen-binding characteristics, producing a meditope-enabled antibody with distinct characteristics. Known antibody engineering methods typically employed to alter antigen binding and other properties include various *in vitro* randomization, affinity maturation, and selection methods, including error-prone PCR, spiked PCR, site-directed mutagenesis, phage display and other selection methods. Also provided are constructs, libraries, and expression systems, including GPI-linked expression systems, for carrying out such methods. Thus, in certain embodiments, the provided meditope-enabled antibody has a light chain and/or heavy chain variable region with the framework region or regions (FRs) of a meditope-enabled antibody, such as cetuximab, a meditope-enabled trastuzumab, or a meditope-enabled M5A (or FR(s) with at least at or about 75, 80, 85, 90, 91, 92, 93, 94, 95, 96, 97, 98, or 99 % identity to the FR(s) of such an antibody). In some aspects, such an antibody has one or more CDRs that are distinct from the CDRs of that meditope-enabled antibody.

For example, in some embodiments, the VL region has an amino acid sequence comprising a light chain framework region (FR) 1 (FR-L1), an FR-L2, an FR-L3, and/or an FR-L4 of the light chain sequence set forth in SEQ ID NO: 71 (or an FR-L1, FR-L2, FR-L3, and/or FR-L4 that is at least at or about 75, 80, 85, 90, 91, 92, 93, 94, 95, 96, 97, 98, or 99 % identical to the FR-L1, FR-L2, FR-L3, and/or FR-L4 of SEQ ID NO: 71), and in some aspects at least one CDR that is distinct from the CDRs of the light chain sequence set forth in SEQ ID NO: 71; and/or a VH region with an amino acid sequence having a heavy chain FR1 (FR-H1), an FR-H2, an FR-H3, and/or an FR-H4, of the heavy chain sequence set forth in SEQ ID NO: 72 (or an FR-H1, FR-H2, FR-H3, and/or FR-H4 that is at least at or about 75, 80, 85, 90, 91, 92, 93, 94, 95, 96, 97, 98, or 99 % identical to the FR-H1, FR-H2, FR-H3, and/or FR-H4 of SEQ ID NO: 72), and in some aspects at least one CDR that is distinct from the CDRs of the heavy chain sequence set forth in SEQ ID NO: 72.

In some embodiments, the VL region has an amino acid sequence comprising a light chain framework region (FR) 1 (FR-L1), an FR-L2, an FR-L3, and/or an FR-L4 of the light chain sequence set forth in SEQ ID NO: 9 (or an FR-L1, FR-L2, FR-L3, and/or FR-L4 that is at least at or about 75, 80, 85, 90, 91, 92, 93, 94, 95, 96, 97, 98, or 99 % identical to the FR-L1, FR-L2, FR-L3, and/or FR-L4 of SEQ ID NO: 9), and in some aspects at least one CDR that is distinct from the CDRs of the light chain sequence set forth in SEQ ID NO: 9; and/or a VH region with an amino acid sequence having a heavy chain FR1 (FR-H1), an FR-H2, an FR-H3, and/or an FR-H4, of the heavy chain sequence set forth in SEQ ID NO: 6 (or an FR-H1, FR-H2, FR-H3, and/or FR-H4 that is at least at or about 75, 80, 85, 90, 91, 92, 93, 94, 95, 96, 97, 98, or 99 % identical to the FR-H1, FR-H2, FR-H3, and/or FR-H4 of SEQ ID NO: 6), and in some aspects at least one CDR that is distinct from the CDRs of the heavy chain sequence set forth in SEQ ID NO: 6.

In some embodiments, the VL region has an amino acid sequence comprising a light chain framework region (FR) 1 (FR-L1), an FR-L2, an FR-L3, and/or an FR-L4 of the light chain sequence set forth in SEQ ID NO: 68 (or an FR-L1, FR-L2, FR-L3, and/or FR-L4 that is at least at or about 75, 80, 85, 90, 91, 92, 93, 94, 95, 96, 97, 98, or 99 % identical to the FR-L1, FR-L2, FR-L3, and/or FR-L4 of SEQ ID NO: 68), and in some aspects at least one CDR distinct from CDRs of the light chain sequence set forth in SEQ ID NO: 68; and/or a VH region with an amino acid sequence having a heavy chain FR1 (FR-H1), an FR-H2, an FR-H3, and/or an FR-H4, of the heavy chain sequence set forth in SEQ ID NO: 70 (or an FR-H1, FR-H2, FR-H3, and/or FR-H4 that is at least at or about 75, 80, 85, 90, 91, 92, 93, 94, 95, 96, 97, 98, or 99 % identical to the FR-H1, FR-H2, FR-H3, and/or FR-H4 of SEQ ID NO: 70), and in some aspects at least one CDR distinct from CDRs of the heavy chain sequence set forth in SEQ ID NO: 70.

In some embodiments, the VL region has an amino acid sequence comprising a light chain framework region (FR) 1 (FR-L1), an FR-L2, an FR-L3, and/or an FR-L4 of the light chain sequence set forth in SEQ ID NO: 61 (or an FR-L1, FR-L2, FR-L3, and/or FR-L4 that is at least at or about 75, 80, 85, 90, 91, 92, 93, 94, 95, 96, 97, 98, or 99 % identical to the FR-L1, FR-L2, FR-L3, and/or FR-L4 of SEQ ID NO: 61), and in some aspects at least one CDR that is distinct from the CDRs of the light chain sequence set forth in SEQ ID NO: 61; and/or a VH region with an amino acid sequence having a heavy chain FR1 (FR-H1), an FR-H2, an FR-H3, and/or an FR-H4, of the heavy chain sequence set forth in SEQ ID NO: 63 (or an FR-H1, FR-H2, FR-H3, and/or FR-H4 that is at least at or about 75, 80, 85, 90, 91, 92, 93, 94, 95, 96, 97, 98, or 99 % identical to the FR-H1, FR-H2, FR-H3, and/or FR-H4 of SEQ ID NO: 63), and in some aspects at least one CDR that is distinct from the CDRs of the heavy chain sequence set forth in SEQ ID NO: 63. In some embodiments, the meditope-enabled antibody has one or more CDRs distinct from the CDRs set forth in SEQ ID NO: 6, 7, 9, 10, 12, 14, 61, 63, 68, 69, 70, 71, and/or 72.

In some embodiments, the meditope is an antibody other than cetuximab, does not specifically bind to an EGFR, binds to an antigen other than EGFR, and/or does not specifically bind to the epitope on EGFR that is specifically bound by cetuximab.

In some examples, the meditope-enabled antibody is generated based on a template antibody that is selected from among abagovomab, abciximab, adalimumab, adecatumumab, alemtuzumab, altumomab, altumomab pentetate,

anatumomab, anatumomab mafenatox, arcitumomab, atlizumab, basiliximab, bectumomab, ectumomab, belimumab, benralizumab, bevacizumab, brentuximab, canakinumab, capromab, capromab pendetide, catumaxomab, certolizumab, clivatuzumab tetraxetan, daclizumab, denosumab, eculizumab, edrecolomab, efalizumab, etaracizumab, ertumaxomab, fanolesomab, Fbta05, fontolizumab, gemtuzumab, girentuximab, golimumab, ibritumomab, igovomab, infliximab, ipilimumab, labetuzumab, mepolizumab, muromonab, muromonab-CD3, natalizumab, necitumumab nimotuzumab, ofatumumab, omalizumab, oregovomab, palivizumab, panitumumab, ranibizumab, rituximab, satumomab, sulesomab, ibritumomab, ibritumomab tiuxetan, tocilizumab, tositumomab, trastuzumab, Trbs07, ustekinumab, visilizumab, votumumab, zalutumumab, brodalumab, anrukinzumab, bapineuzumab, dalotuzumab, demcizumab, ganitumab, inotuzumab, mavrilimumab, moxetumomab pasudotox, rilotumumab, sifalimumab, tanezumab, tralokinumab, tremelimumab, the antibody produced by the hybridoma 10B5, B6H12.2, and urelumab, fragments thereof, antibodies having the CDRs and/or antigen-binding regions thereof, and/or antibodies that compete for binding with such antibodies; and/or antibodies having a sequence set forth in any of SEQ ID NOs: 78-124, and/or 125-170, fragments thereof antibodies having the CDRs and/or antigen-binding regions thereof, and/or antibodies that compete for binding with such antibodies. Table 3 lists CAS® Registry Numbers for certain antibodies.

**Table 3.**

| Antibody | CAS Registry number |
|---|---|
| abagovomab | 792921-10-9 |
| abciximab | 143653-53-6 |
| adalimumab | 331731-18-1 |
| adecatumumab | 503605-66-1 |
| alemtuzumab | 216503-57-0 |
| indium (111In) altumomab pentetate | 156586-92-4 |
| arcitumomab | 154361-48-5 |
| arcitumumab | 154361-48-5 |
| atlizumab | 375823-41-9 |
| basiliximab | 152923-56-3 |
| bectumomab | 158318-63-9 |
| belimumab | 356547-88-1 |
| benralizumab | 1044511-01-4 |
| bevacizumab | 216974-75-3 |
| brentuximab | 914088-09-8 |
| canakinumab | 914613-48-2 |
| capromab pendetide | 145464-28-4 |
| capromab | 151763-64-3 |
| catumaxomab | 509077-98-9 |
| certolizumab | 428863-50-7 |
| certolizumab | 428863-50-7 |
| cetuximab | 205923-56-4 |
| clivatuzumab tetraxetan | 943976-23-6 |
| daclizumab | 152923-56-3 |
| denosumab | 615258-40-7 |
| eculizumab | 219685-50-4 |
| edrecolomab | 156586-89-9 |
| efalizumab | 214745-43-4 |
| etaracizumab | 892553-42-3 |
| etrumaxomab | 509077-99-0 |
| fanolesomab | 225239-31-6 |
| FBTA05 | Lymphomun/FBTA05 |
| fontolizumab | 326859-36-3 |
| gemtuzumab | 220578-59-6 |
| girentuximab | 916138-87-9 |
| golimumab | 476181-74-5 |
| ibritumomab | 174722-31-7 |

(continued)

| Antibody | CAS Registry number |
|---|---|
| igovomab | 171656-50-1 |
| Infliximab | 170277-31-3 |
| ipilimumab | 477202-00-9 |
| labetuzumab | 219649-07-7 |
| mepolizumab | 196078-29-2 |
| muromonab-CD3 | 140608-64-6 |
| natalizumab | 189261-10-7 |
| nimotuzumab | 828933-61-3 |
| ofatumumab | 679818-59-8 |
| omalizumab | 242138-07-4 |
| oregovomab | 213327-37-8 |
| palivizumab | 188039-54-5 |
| panitumumab | 339177-26-3 |
| ranibizumab | 347396-82-1 |
| rituximab | 174722-31-7 |
| satumomab | 138955-26-7 |
| sulesomab | 167747-19-5 |
| tiuxetan (ibritumomab) | 174722-31-7 |
| tocilizumab | 375823-41-9 |
| tositumomab | 192391-48-3 |
| trastuzumab | 180288-69-1 |
| ustekinumab | 815610-63-0 |
| votumumab | 148189-70-2 |
| zalutumumab | 667901-13-5 |
| brodalumab | 1174395-19-7 |
| anrukinzumab | 910649-32-0 |
| bapineuzumab | 648895-38-9 |
| dalotuzumab | 1005389-60-5 |
| demcizumab (OMP-21M18) | 1292853-12-3 |
| ganitumab | 905703-97-1 |
| inotuzumab | 635715-01-4 |
| mavrilimumab | 1085337-57-0 |
| moxetumomab | 1020748-57-5 |
| moxetumomab pasudotox | 1020748-57-5 |
| rilotumumab | 872514-65-3 |
| sifalimumab | 1143503-67-6 |
| tanezumab | 880266-57-9 |
| tralokinumab | 1044515-88-9 |
| tremelimumab | 745013-59-6 |
| urelumab | 934823-49-1 |
| necitumumab | 906805-06-9 |

In other examples, the template antibody is selected from among: abagovomab, abciximab, adalimumab, adecatumumab, alemtuzumab, altumomab, altumomab pentetate, anatumomab, anatumomab mafenatox, arcitumomab, atlizumab, basiliximab, bectumomab, ectumomab, belimumab, benralizumab, bevacizumab, brentuximab, canakinumab, capromab, capromab pendetide, catumaxomab, certolizumab, clivatuzumab tetraxetan, daclizumab, denosumab, eculizumab, edrecolomab, efalizumab, etaracizumab, ertumaxomab, fanolesomab, Fbta05, fontolizumab, gemtuzumab, girentuximab, golimumab, ibritumomab, igovomab, infliximab, ipilimumab, labetuzumab, mepolizumab, muromonab, muromonab-CD3, natalizumab, necitumumab, nimotuzumab, ofatumumab, omalizumab, oregovomab, palivizumab, panitumumab, ranibizumab, rituximab, satumomab, sulesomab, ibritumomab, ibritumomab tiuxetan, tocilizumab, tositumomab, trastuzumab, Trbs07, ustekinumab, visilizumab, votumumab, zalutumumab, the antibody produced by the hybridoma 10B5, and brodalumab, or tiuzetan. In some such examples, the one or more CDRs are CDRs present in these template antibodies, and/or the antibodies specifically bind to the same antigen or epitope as such antibodies, and/or compete for binding with such antibodies to their antigens.

Thus, in some cases, the meditope-enabled antibodies (including fragments thereof) specifically binds to an antigen selected from the group consisting of: CA-125, glycoprotein (GP) IIb/IIIa receptor, TNF-alpha, CD52, TAG-72, Carcinoembryonic antigen (CEA), interleukin-6 receptor (IL-6R), IL-2, interleukin-2 receptor a-chain (CD25), CD22, CD23 (also known as CD23A, Fc epsilon RII, FcεRII, FCE2, CLEC4J, C-Type Lectin Domain Family 4 Member J, Immunoglobulin E-Binding Factor, Lymphocyte IgE Receptor, BLAST-2, or IGEBF), CD37 (also known as tetraspanin-26, TSPAN26, tspan-26, or GP52-40), Mucin-1, Prolatin Receptor (also known as PRL-R), SDC-1 (also known as CD138, Syndecan Proteoglycan 1, Syndecan 1, or Heparan Sulfate Proteoglycan Fibroblast Growth Factor Receptor), B-cell activating factor, interleukin-5 receptor (CD125), VEGF, VEGF-A, CD30, IL-1beta, prostate specific membrane antigen (PSMA), CD3, EpCAM, EGF receptor (EGFR), MUC1, human interleukin-2 receptor, Tac, RANK ligand, a complement protein, e.g., C5, EpCAM, CD11a, e.g., human CD11a, an integrin, e.g., alpha-v beta-3 integrin, vitronectin receptor alpha v beta 3 integrin , HER2, neu, CD3, CD15, CD20 (small and/or large loops), Interferon gamma, CD33, CA-IX, TNF alpha,, CTLA-4, carcinoembryonic antigen, IL-5, CD3 epsilon, CAM, Alpha-4-integrin, IgE, e.g., IgE Fc region, an RSV antigen, e.g., fusion protein of respiratory syncytial virus (RSV), TAG-72, NCA-90(granulocyte cell antigen), IL-6, GD2, GD3, IL-12, IL-23, IL-17, CTAA16.88, IL13, interleukin-1 beta, beta-amyloid, IGF-1 receptor (IGF-1R), delta-like ligand 4 (DLL4), alpha subunit of granulocyte macrophage colony stimulating factor receptor, hepatocyte growth factor, IFN-alpha, nerve growth factor, IL-13, PD-L1, CD326, CD47, and CD137. In some examples, the meditope-enabled antigen binds to another antigen identified as a target in a disease or condition of interest, such as cancer or other disease.

**CD19**

In one embodiment, the template antibody is an anti-CD19 antibody, for example, a human or humanized antibody or a mouse antibody for CD19, such as MOR208, or an antibody having a heavy chain, light chain, VH, or VL, of such an antibody, or a functional fragment thereof. In some embodiments, the meditope-enabled antibody specifically binds to CD19. CD19 (or cluster of differentiation-19) is, for example, identified by NCBI Accession No. NP_001171569 or UniProt Identifier P15391. In one aspect, CD19 is found on the surface of B cells from earliest recognizable B-lineage cells during development to B-cell blasts but is lost on maturation to plasma cells. In certain aspects, CD19 is a regulatory molecule that decreases the threshold for antigen receptor-dependent stimulation.

In some embodiments, the template antibody comprises the amino acid sequence of SEQ ID NO: 226 and/or of SEQ ID NO: 227 (with or without a leader sequence), which set forth light chain and heavy chain amino acid sequences, respectively, of MOR208 (XmAb®5574) (a monoclonal antibody that specifically binds to CD19, e.g., CD19 of immature, mature and malignant B cells), and/or comprises the amino acid sequence of SEQ ID NO: 245 and/or 246 (with or without a leader sequence), which set forth VL and VH amino acid sequences of MOR208, respectively.

In some aspects, the meditope-enabled antibody or fragment has a CDR (i.e., one or more CDR, e.g., CDR1, CDR2, and/or CDR3) of the light chain sequence set forth in SEQ ID NO: 226, or of the light chain or VL of MOR208, and/or a CDR (i.e., one or more CDR, e.g., CDR1, CDR2, and/or CDR3) of the heavy chain set forth in SEQ ID NO: 227, or of the heavy chain or VH of MOR208.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the amino acid sequence set forth as SEQ ID NO: 245, with or without the leader sequence, and/or the VH region of the meditope-enabled antibody contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the amino acid sequence of the VH region of the amino acid sequence set forth as SEQ ID NO: 246, with or without the leader sequence.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of MOR208, or of SEQ ID NO: 226; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of MOR208, or SEQ ID NO: 227; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of MOR208 or SEQ ID NO: 246; and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL region of MOR208 or SEQ ID NO: 245. In some embodiments, the antibody contains all or at least 95,

96, 97, 98, or 99 % identity within one or more, e.g., all, of the CDRs within the heavy and/or light chain, as compared to MOR208 (e.g., as compared to the CDR(s) of SEQ ID NO: 226 or 227).

In some aspects, the meditope-enabled antibody does not specifically bind to the epitope of a CD19 that is specifically bound by MOR208, but does specifically bind CD 19, or does not contain the CDRs of MOR208, and/or does not compete for antigen binding with MOR208, but does specifically bind to CD19.

**CD22**

In one embodiment, the template antibody is an anti-CD22 antibody, for example, a human or humanized antibody or a mouse antibody for CD22, such as epratuzumab, or moxetumomab, or an antibody having a heavy chain, light chain, VH, or VL, of such an antibody, or a functional fragment thereof. In some embodiments, the meditope-enabled antibody specifically binds to CD22. CD22 (or cluster of differentiation-22) is a molecule belonging to the SIGLEC family of lectins, for example, as identified by NCBI Accession No. NP_001762 or UniProt Identifier P20273. In one aspect, CD22 is found on the surface of mature B cells and to a lesser extent on some immature B cells. In certain aspects, CD22 is a regulatory molecule that prevents the over-activation of the immune system and the development of autoimmune diseases. In one embodiment, a template antibody for CD22 specifically binds to one or more epitopes on a polypeptide or protein having the amino acid sequence of SEQ ID NO: 175, or a polypeptide or protein having the amino acid sequence encoded by a polynucleotide comprising the nucleic acid sequence of SEQ ID NO: 176.

In some embodiments, the template antibody comprises the amino acid sequence of SEQ ID NO: 177 and/or of SEQ ID NO: 178 (with or without the leader sequence, which in each sequence is set forth in italicized text below), which set forth light chain and heavy chain amino acid sequences, respectively, of epratuzumab (a humanized monoclonal antibody that specifically binds to CD22, e.g., CD22 of mature and malignant B cells), and/or comprises the amino acid sequence of SEQ ID NO: 179 and/or 183 (with or without the leader sequence, which in each sequence is set forth in italicized text), which set forth VL and VH amino acid sequences of epratuzumab, respectively.

Epratuzumab light chain sequence (SEQ ID NO: 177):

MKLPVRLLVLLLFWIPASRGDVQVTQSPSSLSASVGDRVTITCRSSQSLANSY
GNTFLSWYLHKPGKAPQLLIYGISNRFSGVPDRFSGSGSGTDFTLTISSLQPED
FATYYCLQGTHQPYTFGQGTKVEIKRTVAAPSVFIFPPSDEQLKSGTASVVCL
LNNFYPREAKVQWKVDNALQSGNSQESVTEQDSKDSTYSLSSTLTLSKADYE
KHKVYACEVTHQGLSSPVTKSFNRGEC.

Epratuzumab heavy chain sequence (SEQ ID NO: 178):

MDFGFSLVFLALILKGVQCEVQLVQSGAEVKKPGASVKVSCKASGYRFTNY
WIHWVRQAPGQGLEWIGGINPGNNYATYRRKFQGRVTMTADTSTSTVYMEL
SSLRSEDTAVYYCTREGYGNYGAWFAYWGQGTLVTVSSASTKGPSVFPLAP
CSRSTSESTAALGCLVKDYFPEPVTVSWNSGALTSGVHTFPAVLQSSGLYSLS
SVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKP
REEQFNSTYRVVSVLTVLHQDWLNGKEYKCKVSNKGLPSSIEKTISKAKGQP
REPQVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLSLGK
.

Epratuzumab light chain variable (VL) region sequence (SEQ ID NO: 179):

*MKLPVRLLVLLLFWIPASRG*DVQVTQSPSSLSASVGDRVTITCRSSQSLANSYGNTFLSWYLHKPGKAPQLLIYGISNRFSGVPDRFSGSGSGTDFTLTISSLQPEDFATYYCLQGTHQPYTFGQGTKVEIKRTV.

Epratuzumab heavy chain variable (VH) region sequence (SEQ ID NO: 183):

MDFGFSLVFLALILKGVQCEVQLVQSGAEVKKPGASVKVSCKASGYRFTNYWIHWVRQAPGQGLEWIGGINPGNNYATYRRKFQGRVTMTADTSTSTVYMELSSLRSEDTAVYYCTREGYGNYGAWFAYWGQGTLVTVSS.

In some aspects, the meditope-enabled antibody or fragment has a CDR (i.e., one or more CDR, e.g., CDR1, CDR2, and/or CDR3) of the light chain or VL sequence set forth in SEQ ID NO: 177 or SEQ ID NO: 179, or of the light chain or VL of epratuzumab, and/or a CDR (i.e., one or more CDR, e.g., CDR1, CDR2, and/or CDR3) of the heavy chain or VH sequence set forth in SEQ ID NO: 178 or 183, or of the heavy chain or VH of epratuzumab.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the amino acid sequence set forth as SEQ ID NO: 179, with or without the leader sequence, and/or the VH region of the meditope-enabled antibody contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the amino acid sequence of SEQ ID NO: 183, with or without the leader sequence.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of epratuzumab, or of SEQ ID NO: 177 or of 179; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of epratuzumab, of SEQ ID NO: 178 or of 183; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of epratuzumab (e.g., to the amino acid sequence of SEQ ID NO: 183); and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL region of epratuzumab (e.g., to the amino acid sequence of SEQ ID NO: 179). In some embodiments, the antibody contains all or at least 95, 96, 97, 98, or 99 % identity within one or more, e.g., all, of the CDRs within the heavy and/or light chain, as compared to epratuzumab (e.g., as compared to the CDR(s) of SEQ ID NO: 177 or 179 or SEQ ID NO: 178 or 183).

In some aspects, the meditope-enabled antibody does not specifically bind to the epitope of a CD22 that is specifically bound by epratuzumab, but does specifically bind CD22, or does not contain the CDRs of epratuzumab, and/or does not compete for antigen binding with epratuzumab, but does specifically bind to CD22.

In some embodiments, the template antibody comprises the amino acid sequence of SEQ ID NO: 65 and/or of SEQ ID NO: 67 (with or without the leader sequence/residue), which set forth VL and VH amino acid sequences, respectively, of moxetumomab (an anti-CD22 mouse monoclonal antibody), and/or comprises the amino acid sequence of SEQ ID NO: 66 (with or without the leader sequence, which where applicable is set forth in italics), which sets forth a heavy chain amino acid sequence of moxetumomab.

Moxetumomab light chain variable (VL) region sequence (SEQ ID NO: 65):

DIQMTQTTSSLSASLGDRVTISCRASQDISNYLNWYQQKPDGTVKLLIYYTSILHSGVPSRFSGSGSGTDYSLTISNLEQEDFATYFCQQGNTLPWTFGCGTKLEIK.

Moxetumomab heavy chain sequence (SEQ ID NO: 66):

MEVQLVESGGGLVKPGGSLKLSCAASGFAFSIYDMSWVRQTPEKCLEWVAY
ISSGGGTTYYPDTVKGRFTISRDNAKNTLYLQMSSLKSEDTAMYYCARHSGY
GTHWGVLFAYWGQGTLVTVSAKASGGPEGGSLAALTAHQACHLPLETFTRH
RQPRGWEQLEQCGYPVQRLVALYLAARLSWNQVDQVIRNALASPGSGGDLG
EAIREQPEQARLALTLAAAESERFVRQGTGNDEAGAANGPADSGDALLERNY
PTGAEFLGDGGDVSFSTRGTQNWTVERLLQAHRQLEERGYVFVGYHGTFLE

AAQSIVFGGVRARSQDLDAIWRGFYIAGDPALAYGYAQDQEPDARGRIRNGA
LLRVYVPRSSLPGFYRTSLTLAAPEAAGEVERLIGHPLPLRLDAITGPEEEGGR
LETILGWPLAERTVVIPSAIPTDPRNVGGDLDPSSIPDKEQAISALPDYASQPGK
PPREDLK.

Moxetumomab heavy chain variable (VH) region sequence (SEQ ID NO: 67):

*M*EVQLVESGGGLVKPGGSLKLSCAASGFAFSIYDMSWVRQTPEKCLEWVAYI
SSGGGTTYYPDTVKGRFTISRDNAKNTLYLQMSSLKSEDTAMYYCARHSGY
GTHWGVLFAYWGQGTLVTVSA.

In some aspects, the meditope-enabled antibody or fragment has a CDR (i.e., one or more CDR, e.g., CDR1, CDR2, and/or CDR3) of the VL sequence set forth in SEQ ID NO: 65, or of the light chain or VL of moxetumomab, and/or a CDR (i.e., one or more CDR, e.g., CDR1, CDR2, and/or CDR3) of the heavy chain or VH sequence set forth in SEQ ID NO: 66 or 67, or of the heavy chain or VH of moxetumomab.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the amino acid sequence set forth as SEQ ID NO: 65 with or without the leader sequence, or VL of moxetumomab and/or the VH region of the meditope-enabled antibody contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the amino acid sequence of SEQ ID NO: 67, with or without the leader sequence, or VH of moxetumomab.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of moxetumomab, or of SEQ ID NO: 65; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of moxetumomab, of SEQ ID NO: 66 or 67; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of moxetumomab (e.g., to the amino acid sequence of SEQ ID NO: 67); and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL region of moxetumomab (e.g., to the amino acid sequence of SEQ ID NO: 65). In some embodiments, the antibody contains all or at least 95, 96, 97, 98, or 99 % identity within one or more, e.g., all, of the CDRs within the heavy and/or light chain, as compared to moxetumomab (e.g., as compared to the CDR(s) of SEQ ID NO: 65 or 66 or 67).

In some aspects, the meditope-enabled antibody does not specifically bind to the epitope of a CD22 that is specifically bound by moxetumomab, but does specifically bind CD22, or does not contain the CDRs of moxetumomab,

and/or does not compete for antigen binding with moxetumomab, but does specifically bind to CD22.

**CD23**

In one embodiment, the template antibody is an anti-CD23 antibody, for example, a human or humanized antibody or a mouse antibody for CD23, such as lumiliximab, or an antibody having a heavy chain, light chain, VH, or VL, of such an antibody, or a functional fragment thereof.

In some embodiments, the meditope-enabled antibody specifically binds to CD23. CD23 is also known as Fc epsilon RII, or FcεRII, is the "low-affinity" receptor for IgE, an antibody isotype involved in allergy and resistance to parasites, and is important in regulation of IgE levels. CD23 is a C-type lectin, for example, as identified by NCBI Accession No. NP_001193948.2 or UniProt Identifier P06734. CD23 antigen is a 45-kDa type II transmembrane glycoprotein that is expressed on several hematopoietic cell types. In some aspects, CD23 is expressed on mature B cells, activated macrophages, eosinophils, follicular dendritic cells, and platelets. CD23 is the only FcR that does not belong to the immunoglobulin gene superfamily. The functions of CD23 include modulation of IgE production by B cells and promoting the survival of germinal center-derived B cells. The expression of CD23 is highly upregulated in normally activated follicular B cells and in CLL cells.

In one embodiment, a template antibody for CD23 specifically binds to one or more epitopes on a polypeptide or protein having the amino acid sequence of SEQ ID NO: 172, or a polypeptide or protein having the amino acid sequence encoded by a polynucleotide comprising the nucleic acid sequence of SEQ ID NO: 184.

In some embodiments, the template antibody comprises the amino acid sequence of SEQ ID NO: 185 and/or of SEQ ID NO: 190 (with or without the leader sequence/residue, which where applicable is set forth in italics), which set forth VL and VH amino acid sequences, respectively, of lumiliximab.

Lumiliximab light chain variable (VL) region sequence plus leader (SEQ ID NO: 185):

*MDMRVPAQLLGLLLLWLPGARC*DIQMTQSPSSLSASVGDRVTITCRASQDIRYY
LNWYQQKPGKAPKLLIYVASSLQSGVPSRFSGSGSGTEFTLTVSSLQPEDFAT
YYCLQVYSTPRTFGQGTKVEIK.

Lumiliximab heavy chain variable (VH) region sequence plus leader (SEQ ID NO: 190):

MEFGLSWVFLVPLLKGVQCEVQLVESGGGLAKPGGSLRLSCAASGFRFTFNN
YYMDWVRQAPGQGLEWVSRISSSGDPTWYADSVKGRFTISRENANNTLFLQ
MNSLRAEDTAVYYCASLTTGSDSWGQGVLVTVSS.

Lumiliximab (also known as IDEC-152, P5E8, gomiliximab; Biogen Idec) is a primatized anti-CD23 mAb that contains cynomolgus macaque variable regions and human constant regions (IgG1-κ). It was originally developed to inhibit the production of IgE by activated human peripheral blood B cells; however, later on it gained an appreciation as a potent anti-CD23 mAb. Preclinical data suggest that lumiliximab mediates its antitumor effect on CLL cells and CD23-expressing B cells predominantly by the intrinsic pathway of apoptosis (i.e., inducing downregulation of anti-apoptotic proteins Bcl-2, Bcl-XI, and XIAP, activation of Bax and release of mitochondrial cytochrome c). Synergistic cytotoxicity has also been demonstrated on CD23-expressing B-cell lines and primary CLL cells when lumiliximab was combined with rituximab or fludarabine in xenograft models. In a Phase I clinical trial of 46 heavily pretreated CLL patients, lumiliximab showed modest clinical activity as a single agent. Although no objective Complete Responses (CRs) or Partial Responses (PRs) were observed, lumiliximab did reduce enlarged lymph node size in 52% of patients and decreased peripheral blood lymphocyte counts in 91% of patients. The safety profile of lumiliximab demonstrated that the majority of adverse events (AEs) are limited to grade 1/2. Lumiliximab was not immunosuppressive and the MTD (maximum tolerated dose) was not reached.

In some aspects, the meditope-enabled antibody or fragment has a CDR (i.e., one or more CDR, e.g., CDR1, CDR2, and/or CDR3) of the VL sequence set forth in SEQ ID NO: 185, or of the light chain or VL of lumiliximab, and/or a CDR (i.e., one or more CDR, e.g., CDR1, CDR2, and/or CDR3) of the VH sequence set forth in SEQ ID NO: 190, or of the heavy chain or VH of lumiliximab.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the amino acid sequence set forth as SEQ ID NO: 185 with or without the leader sequence, or VL of lumiliximab and/or the VH region of the meditope-enabled antibody contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the amino acid sequence of SEQ ID NO: 190, with or without the leader sequence, or VH of lumiliximab.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of lumiliximab, or of SEQ ID NO: 185; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of lumiliximab, of SEQ ID NO: 190; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of lumiliximab (e.g., to the amino acid sequence of SEQ ID NO: 190); and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL region of lumiliximab (e.g., to the amino acid sequence of SEQ ID NO: 185). In some embodiments, the antibody contains all or at least 95, 96, 97, 98, or 99 % identity within one or more, e.g., all, of the CDRs within the heavy and/or light chain, as compared to lumiliximab (e.g., as compared to the CDR(s) of SEQ ID NO: 185 or 190).

In some aspects, the meditope-enabled antibody does not specifically bind to the epitope of a CD23 that is specifically bound by lumiliximab, but does specifically bind CD23, or does not contain the CDRs of lumiliximab, and/or does not compete for antigen binding with lumiliximab, but does specifically bind to CD23.

**CD33**

In one embodiment, the template antibody is an anti-CD33 antibody, for example, a human or humanized antibody or a mouse antibody for CD33, such as gemtuzumab, or an antibody having a heavy chain, light chain, VH, or VL, of such an antibody, or a functional fragment thereof. In some embodiments, the meditope-enabled antibody specifically binds to CD33. CD33 (or cluster of differentiation-33) is, for example, identified by UniProt Identifier P20138. In one aspect, CD33 is found on the surface of myeloid cells.

In some embodiments, the template antibody comprises the amino acid sequence of SEQ ID NO: 224 and/or of SEQ ID NO: 225 (with or without a leader sequence), which set forth light chain and heavy chain amino acid sequences, respectively, of gemtuzumab (Mylotarg) (a monoclonal antibody that specifically binds to CD33, e.g., CD33 of immature, mature and malignant B cells), and/or comprises the amino acid sequence of SEQ ID NO: 243 and/or 245 (with or without a leader sequence), which set forth VL and VH amino acid sequences of gemtuzumab, respectively.

In some aspects, the meditope-enabled antibody or fragment has a CDR (i.e., one or more CDR, e.g., CDR1, CDR2, and/or CDR3) of the light chain sequence set forth in SEQ ID NO: 224, or of the light chain or VL of gemtuzumab, and/or a CDR (i.e., one or more CDR, e.g., CDR1, CDR2, and/or CDR3) of the heavy chain set forth in SEQ ID NO: 225, or of the heavy chain or VH of gemtuzumab.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the amino acid sequence set forth as SEQ ID NO: 243, with or without the leader sequence, and/or the VH region of the meditope-enabled antibody contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the amino acid sequence of the VH region of the amino acid sequence set forth as SEQ ID NO: 244, with or without the leader sequence.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of gemtuzumab, or of SEQ ID NO: 224; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of gemtuzumab, or SEQ ID NO: 225; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of gemtuzumab or SEQ ID NO: 244; and/or contains, within its VL region, at least at or about 75, 76, 77, 78, 79, 80, 81, 82, 83, 84, 85, 86, 87, 88, 89, 90, 91, 92, 93, 94, 95, 96, 97,

98, or 99 % identity to the VL region of gemtuzumab or SEQ ID NO: 243. In some embodiments, the antibody contains all or at least 95, 96, 97, 98, or 99 % identity within one or more, e.g., all, of the CDRs within the heavy and/or light chain, as compared to gemtuzumab (e.g., as compared to the CDR(s) of SEQ ID NO: 224 or 225).

In some aspects, the meditope-enabled antibody does not specifically bind to the epitope of a CD33 that is specifically bound by gemtuzumab, but does specifically bind CD33, or does not contain the CDRs of gemtuzumab, and/or does not compete for antigen binding with gemtuzumab, but does specifically bind to CD33.

**CD37**

In one embodiment, the template antibody is an anti-CD37 antibody, for example, a human or humanized antibody or a mouse antibody for CD37, such as otlertuzumab, or an antibody having a heavy chain, light chain, VH, or VL, of such an antibody, or a functional fragment thereof.

In some embodiments, the meditope-enabled antibody specifically binds to CD37. CD37, also known as GP52-40 or TSPAN26, is a member of the transmembrane 4 superfamily, also known as the tetraspanin family. WO 2011112978 discloses CD37-binding molecules and immunoconjugates thereof, the disclosure of which is incorporated herein by reference in its entirety for all purposes.

In one embodiment, a template antibody for CD37 specifically binds to one or more epitopes on a polypeptide or protein having the amino acid sequence of SEQ ID NO: 191, or a polypeptide or protein having the amino acid sequence encoded by a polynucleotide comprising the nucleic acid sequence of SEQ ID NO: 192.

In some embodiments, the template antibody comprises the amino acid sequence of SEQ ID NO: 193 and/or of SEQ ID NO: 194, which set forth VL and VH amino acid sequences, respectively, of otlertuzumab.

Otlertuzumab (also known as Betalutin or IMGN529) targets CD37, which has an expression profile similar to that of CD20 on NHL subtypes.

In some aspects, the meditope-enabled antibody or fragment has a CDR (i.e., one or more CDR, e.g., CDR1, CDR2, and/or CDR3) of the VL sequence set forth in SEQ ID NO: 193, or of the light chain or VL of otlertuzumab, and/or a CDR (i.e., one or more CDR, e.g., CDR1, CDR2, and/or CDR3) of the VH sequence set forth in SEQ ID NO: 194, or of the heavy chain or VH of otlertuzumab.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the amino acid sequence set forth as SEQ ID NO: 193, or VL of otlertuzumab and/or the VH region of the meditope-enabled antibody contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the amino acid sequence of SEQ ID NO: 194, or VH of otlertuzumab.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of otlertuzumab, or of SEQ ID NO: 193; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of otlertuzumab, of SEQ ID NO: 194; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of otlertuzumab (e.g., to the amino acid sequence of SEQ ID NO: 194); and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL region of otlertuzumab (e.g., to the amino acid sequence of SEQ ID NO: 193). In some embodiments, the antibody contains all or at least 95, 96, 97, 98, or 99 % identity within one or more, e.g., all, of the CDRs within the heavy and/or light chain, as compared to otlertuzumab (e.g., as compared to the CDR(s) of SEQ ID NO: 193 or 194).

In some aspects, the meditope-enabled antibody does not specifically bind to the epitope of a CD37 that is specifically bound by otlertuzumab, but does specifically bind CD37, or does not contain the CDRs of otlertuzumab, and/or does not compete for antigen binding with otlertuzumab, but does specifically bind to CD37.

**Mucin-1**

In one embodiment, the template antibody is an anti- Mucin-1 antibody, for example, a human or humanized antibody or a mouse antibody for Mucin-1, such as hPAM4-Cide (also known as clivatuzumab tetraxetan), or an antibody having a heavy chain, light chain, VH, or VL, of such an antibody, or a functional fragment thereof.

In some embodiments, the meditope-enabled antibody specifically binds to Mucin 1. Mucin-1 is also known as Mucin 1, cell surface associated (MUC1), or polymorphic epithelial mucin (PEM)) and is a mucin encoded by the MUC1

gene in humans, of example, as identified by NCBI Accession No. NP_001018021 or UniProt Identifier P15941. In one embodiment, a template antibody for Mucin-1 specifically binds to one or more epitopes on a polypeptide or protein having the amino acid sequence of SEQ ID NO: 195, or a polypeptide or protein having the amino acid sequence encoded by a polynucleotide comprising the nucleic acid sequence of SEQ ID NO: 196.

In some embodiments, the template antibody comprises the amino acid sequence of SEQ ID NO: 197 and/or of SEQ ID NO: 198, which set forth VL and VH amino acid sequences, respectively, of hPAM4-Cide (also known as clivatuzumab tetraxetan), respectively. WO 2010042562 discloses additional Mucin-1 antibodies, the disclosure of which is incorporated herein by reference for all purposes.

In some aspects, the meditope-enabled antibody or fragment has a CDR (i.e., one or more CDR, e.g., CDR1, CDR2, and/or CDR3) of the VL sequence set forth in SEQ ID NO: 197, or of the light chain or VL of hPAM4-Cide, and/or a CDR (i.e., one or more CDR, e.g., CDR1, CDR2, and/or CDR3) of the VH sequence set forth in SEQ ID NO: 198, or of the heavy chain or VH of hPAM4-Cide.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the amino acid sequence set forth as SEQ ID NO: 197 with or without the leader sequence, or VL of hPAM4-Cide and/or the VH region of the meditope-enabled antibody contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the amino acid sequence of SEQ ID NO: 198, with or without the leader sequence, or VH of hPAM4-Cide.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of hPAM4-Cide, or of SEQ ID NO: 197; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of hPAM4-Cide, of SEQ ID NO: 198; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of hPAM4-Cide (e.g., to the amino acid sequence of SEQ ID NO: 198); and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL region of hPAM4-Cide (e.g., to the amino acid sequence of SEQ ID NO: 197). In some embodiments, the antibody contains all or at least 95, 96, 97, 98, or 99 % identity within one or more, e.g., all, of the CDRs within the heavy and/or light chain, as compared to hPAM4-Cide (e.g., as compared to the CDR(s) of SEQ ID NO: 197 or 198). SEQ ID NO:197 shows an hPAM4-Cide light chain variable (VL) region sequence and SEQ ID NO:198 shows an hPAM4-Cide heavy chain variable (HL) region sequence.

In some aspects, the meditope-enabled antibody does not specifically bind to the epitope of a Mucin1 that is specifically bound by hPAM4-Cide, but does specifically bind Mucin1, or does not contain the CDRs of hPAM4-Cide, and/or does not compete for antigen binding with hPAM4-Cide, but does specifically bind to Mucin1.

**Prolactin Receptor**

In one embodiment, the template antibody is an anti- prolactin receptor (PRLR) antibody, for example, a human or humanized antibody or a mouse antibody for prolactin receptor, such as LFA-102 or 002-H08, or an antibody having a heavy chain, light chain, VH, or VL, of such an antibody, or a functional fragment thereof.

In some embodiments, the meditope-enabled antibody specifically binds to prolactin receptor. In one aspect, the prolactin receptor is encoded by a gene on Chromosome 5p13-14, and interacts with the prolactin molecule as a transmembrane receptor. The prolactin receptor contains an extracellular region that binds prolactin, a transmembrane region, and a cytoplasmic region.

In some embodiments, the template antibody comprises the amino acid sequence of a VL and/or VH amino acid sequence of LFA-102, which is a humanized monoclonal antibody of the IgG1 kappa subtype, and binds to the putative dimerization region of PRLR in a non-ligand competitive manner and inhibits PRL-induced signaling. LFA-102 is disclosed in Application Publication No. US 20110150760, the disclosure of which is incorporated herein by reference in its entirety for all purposes.

In some aspects, the meditope-enabled antibody or fragment has a CDR (i.e., one or more CDR, e.g., CDR1, CDR2, and/or CDR3) of the VL of LFA-102, and/or a CDR (i.e., one or more CDR, e.g., CDR1, CDR2, and/or CDR3) of the VH of LFA-102.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the VL of LFA-102and/or the VH region of the meditope-enabled antibody

contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the VH of LFA-102.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of LFA-102; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of LFA-102; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of LFA-102; and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL region of LFA-102. In some embodiments, the antibody contains all or at least 95, 96, 97, 98, or 99 % identity within one or more, e.g., all, of the CDRs within the heavy and/or light chain, as compared to LFA-102.

In some aspects, the meditope-enabled antibody does not specifically bind to the epitope of a prolactin receptor that is specifically bound by LFA-102, but does specifically bind prolactin receptor, or does not contain the CDRs of LFA-102, and/or does not compete for antigen binding with LFA-102, but does specifically bind to prolactin receptor.

In some embodiments, the template antibody comprises the amino acid sequence of SEQ ID NO: 208 and/or of SEQ ID NO: 209, which set forth VL and VH amino acid sequences, respectively, of 002-H08. Application Publication No. US 20120315276 discloses prolactin receptor antibodies including 002-H08, the disclosure of which is incorporated herein by reference for all purposes.

In some aspects, the meditope-enabled antibody or fragment has a CDR (i.e., one or more CDR, e.g., CDR1, CDR2, and/or CDR3) of the VL sequence set forth in SEQ ID NO: 208, or of the light chain or VL of 002-H08, and/or a CDR (i.e., one or more CDR, e.g., CDR1, CDR2, and/or CDR3) of the VH sequence set forth in SEQ ID NO: 209, or of the heavy chain or VH of 002-H08.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the amino acid sequence set forth as SEQ ID NO: 208, or VL of 002-H08 and/or the VH region of the meditope-enabled antibody contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the amino acid sequence of SEQ ID NO: 209, or VH of 002-H08.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of 002-H08, or of SEQ ID NO: 208; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of 002-H08, of SEQ ID NO: 209; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of 002-H08 (e.g., to the amino acid sequence of SEQ ID NO: 209); and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL region of 002-H08 (e.g., to the amino acid sequence of SEQ ID NO: 208). In some embodiments, the antibody contains all or at least 95, 96, 97, 98, or 99 % identity within one or more, e.g., all, of the CDRs within the heavy and/or light chain, as compared to 002-H08 (e.g., as compared to the CDR(s) of SEQ ID NO: 208 or 209).

In some aspects, the meditope-enabled antibody does not specifically bind to the epitope of a prolactin receptor that is specifically bound by 002-H08, but does specifically bind prolactin receptor, or does not contain the CDRs of 002-H08, and/or does not compete for antigen binding with 002-H08, but does specifically bind to prolactin receptor.

## SDC-1

In one embodiment, the template antibody is an anti-SDC-1 antibody, for example, a human or humanized antibody or a mouse antibody for SDC-1 (also known as Syndecan 1, or CD138), such as indatuximab ravtansine, or an antibody having a heavy chain, light chain, VH, or VL, of such an antibody, or a functional fragment thereof.

In some embodiments, the meditope-enabled antibody specifically binds to SDC-1. In one aspect, SDC-1 is a transmembrane (type I) heparan sulfate proteoglycan and is a member of the syndecan proteoglycan family. The syndecans mediate cell binding, cell signaling, and cytoskeletal organization and syndecan receptors are required for internalization of the HIV-1 tat protein. The syndecan-1 protein functions as an integral membrane protein and participates

in cell proliferation, cell migration and cell-matrix interactions via its receptor for extracellular matrix proteins. Altered syndecan-1 expression has been detected in several different tumor types. While several transcript variants may exist for this gene, the full-length natures of only two have been described to date. These two represent the major variants of this gene and encode the same protein. In one embodiment, a template antibody for SDC-1 specifically binds to one or more epitopes on a polypeptide or protein having the amino acid sequence of SEQ ID NO: 210.

In some embodiments, the template antibody comprises the amino acid sequence of SEQ ID NO: 211 and/or of SEQ ID NO: 212, which set forth VL and VH amino acid sequences, respectively, of indatuximab ravtansine. Indatuximab ravtansine (also known as BT-062, BT062, or B-B4) is a SDC-1 antibody.

In some aspects, the meditope-enabled antibody or fragment has a CDR (i.e., one or more CDR, e.g., CDR1, CDR2, and/or CDR3) of the VL sequence set forth in SEQ ID NO: 211, or of the light chain or VL of indatuximab ravtansine, and/or a CDR (i.e., one or more CDR, e.g., CDR1, CDR2, and/or CDR3) of the VH sequence set forth in SEQ ID NO: 212, or of the heavy chain or VH of indatuximab ravtansine.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the amino acid sequence set forth as SEQ ID NO: 211, or VL of indatuximab ravtansine and/or the VH region of the meditope-enabled antibody contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the amino acid sequence of SEQ ID NO: 212, with or without the leader sequence, or VH of indatuximab ravtansine.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of indatuximab ravtansine, or of SEQ ID NO: 211; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of indatuximab ravtansine, of SEQ ID NO: 212; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of indatuximab ravtansine (e.g., to the amino acid sequence of SEQ ID NO: 212); and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL region of indatuximab ravtansine (e.g., to the amino acid sequence of SEQ ID NO: 211). In some embodiments, the antibody contains all or at least 95, 96, 97, 98, or 99 % identity within one or more, e.g., all, of the CDRs within the heavy and/or light chain, as compared to indatuximab ravtansine (e.g., as compared to the CDR(s) of SEQ ID NO: 211 or 212).

In some aspects, the meditope-enabled antibody does not specifically bind to the epitope of a SDC-1 that is specifically bound by indatuximab ravtansine, but does specifically bind SDC-1, or does not contain the CDRs of indatuximab ravtansine, and/or does not compete for antigen binding with indatuximab ravtansine, but does specifically bind to SDC-1.

**EGFR**

In one embodiment, the template antibody is an anti-EGFR antibody, for example, a human or humanized antibody or a mouse antibody for EGFR, such as ABT-806, zatuximab, or an antibody having a heavy chain, light chain, VH, or VL, of such an antibody, or a functional fragment thereof. In some aspects, the anti-EGFR template antibody is not cetuximab, does not contain all or some CDRs of cetuximab, and/or does not compete for binding with cetuximab.

In one embodiment, a template antibody for EGFR specifically binds to one or more epitopes on a polypeptide or protein having the amino acid sequence of SEQ ID NO: 213, or a polypeptide or protein having the amino acid sequence encoded by a polynucleotide comprising the nucleic acid sequence of SEQ ID NO: 214.

In some embodiments, the template antibody comprises the amino acid sequence of SEQ ID NO: 215 and/or of SEQ ID NO: 216, which set forth VL and VH amino acid sequences, respectively, of ABT-806 (also known as mAb806/414), which is an EGFR antibody, and/or comprises the amino acid sequence of SEQ ID NO: 217 and SEQ ID NO: 218 which set forth light chain and heavy chain sequences of ABT-806, respectively.

In some aspects, the meditope-enabled antibody or fragment has a CDR (i.e., one or more CDR, e.g., CDR1, CDR2, and/or CDR3) of the VL sequence set forth in SEQ ID NO: 215, or of the light chain or VL of ABT-806, and/or a CDR (i.e., one or more CDR, e.g., CDR1, CDR2, and/or CDR3) of the VH sequence set forth in SEQ ID NO: 216, or of the heavy chain or VH of ABT-806.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the amino acid sequence set forth as SEQ ID NO: 215, or VL of ABT-806and/or the VH region of the meditope-enabled antibody contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the amino acid sequence of SEQ ID NO: 216, or VH of ABT-806.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of ABT-806, or of SEQ ID NO: 215; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of ABT-806, of SEQ ID NO: 216; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of ABT-806 (e.g., to the amino acid sequence of SEQ ID NO: 216); and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL region of ABT-806 (e.g., to the amino acid sequence of SEQ ID NO: 215). In some embodiments, the antibody contains all or at least 95, 96, 97, 98, or 99 % identity within one or more, e.g., all, of the CDRs within the heavy and/or light chain, as compared to ABT-806 (e.g., as compared to the CDR(s) of SEQ ID NO: 215 or 216).

In some aspects, the meditope-enabled antibody does not specifically bind to the epitope of a EGFR that is specifically bound by ABT-806, but does specifically bind EGFR, or does not contain the CDRs of ABT-806, and/or does not compete for antigen binding with ABT-806, but does specifically bind to EGFR.

In some embodiments, the template antibody comprises the amino acid sequence of SEQ ID NO: 219 and/or of SEQ ID NO: 220, which set forth VL and VH amino acid sequences, respectively, of zatuximab, and/or comprises the amino acid sequence of SEQ ID NO: 221 and/or 222, which set forth light and heavy chain sequences of zatuximab, an EGFR antibody.

In some aspects, the meditope-enabled antibody or fragment has a CDR (i.e., one or more CDR, e.g., CDR1, CDR2, and/or CDR3) of the VL sequence set forth in SEQ ID NO: 219, or of the light chain or VL of zatuximab, and/or a CDR (i.e., one or more CDR, e.g., CDR1, CDR2, and/or CDR3) of the VH sequence set forth in SEQ ID NO: 220, or of the heavy chain or VH of zatuximab.

In some aspects, the VL region of the meditope-enabled antibody contains one or more, generally a plurality of modifications (which generally are in the framework regions), such as 1, 2, 3, 4, 5, 6, 7, 8, 9, 10, 11, 12, 13, 14, 15, 16, 17, 18, 19, or 20 modifications, compared to the amino acid sequence set forth as SEQ ID NO: 219, or VL of zatuximab and/or the VH region of the meditope-enabled antibody contains one or more, e.g., a plurality of modifications (which generally are in the framework regions), such as 1, 2, 3, 4, 5, 6, 7, 8, 9, 10, 11, 12, 13, 14, 15, 16, 17, 18, 19, or 20 modifications) compared to the amino acid sequence of SEQ ID NO: 220, or VH of zatuximab.

In some embodiments, the meditope-enabled antibody contains, within its light chain framework regions (or within one or more such regions, e.g., within FR-L1, FR-L2, FR-L3, and/or FR-L4), at least at or about 75, 76, 77, 78, 79, 80, 81, 82, 83, 84, 85, 86, 87, 88, 89, 90, 91, 92, 93, 94, 95, 96, 97, 98, or 99 % identity to the light chain framework regions (or respective one or more FR-L(s)) of zatuximab, or of SEQ ID NO: 219; and/or contains, within its heavy chain framework regions (or within one or more such regions, e.g., within FR-H1, FR-H2, FR-H3, and/or FR-H4), at least at or about 75, 76, 77, 78, 79, 80, 81, 82, 83, 84, 85, 86, 87, 88, 89, 90, 91, 92, 93, 94, 95, 96, 97, 98, or 99 % identity to the heavy chain framework regions (or respective one or more FR-H) of zatuximab, of SEQ ID NO: 220; and/or contains, within its VH region, at least at or about 75, 76, 77, 78, 79, 80, 81, 82, 83, 84, 85, 86, 87, 88, 89, 90, 91, 92, 93, 94, 95, 96, 97, 98, or 99 % identity to the VH region of zatuximab (e.g., to the amino acid sequence of SEQ ID NO: 220); and/or contains, within its VL region, at least at or about 75, 76, 77, 78, 79, 80, 81, 82, 83, 84, 85, 86, 87, 88, 89, 90, 91, 92, 93, 94, 95, 96, 97, 98, or 99 % identity to the VL region of zatuximab (e.g., to the amino acid sequence of SEQ ID NO: 220). In some embodiments, the antibody contains all or at least 95, 96, 97, 98, or 99 % identity within one or more, e.g., all, of the CDRs within the heavy and/or light chain, as compared to zatuximab (e.g., as compared to the CDR(s) of SEQ ID NO: 219 or 220).

In some aspects, the meditope-enabled antibody does not specifically bind to the epitope of a EGFR that is specifically bound by zatuximab, but does specifically bind EGFR, or does not contain the CDRs of zatuximab, and/or does not compete for antigen binding with zatuximab, but does specifically bind to EGFR.

The meditope-enabled antibodies generally further include a constant region, typically a heavy and light chain constant region, which generally are human or partially human constant regions. In some aspects, the heavy chain constant region includes a CH1, or a portion thereof. In some aspects, the light chain constant region includes a CL, or a portion thereof. In some embodiments, the portion of the constant region is sufficient to render the antibody capable of binding to the meditope, e.g., with the requisite binding affinity. In some aspects, the constant regions are the constant regions of cetuximab or trastuzumab. Thus, in some aspects, the heavy chain constant regions are one or more human IgG1 constant regions; in some aspects, the light chain constant regions are kappa constant chain regions. In other

examples, the constant regions can include those of other isotypes, including human (or other organism, e.g., murine or chicken) IgG1, IgG2, IgG3, IgG4, IgEs, IgA1, IgA2, IgD, or IgM, and can include kappa or lambda constant regions. Thus, among the provided meditope-enabled antibodies are those generated by mutation of residues in other IgGs, such as human, murine, or chicken, or other immunoglobulins. In other words, the meditope-enabling methods provided herein may be used for any antibody, including IgA, IgE, IgD, and IgM and from any organism that produces antibodies including but not limited to chicken, murine, rat, bovine, rabbit, primates, and goat.

For example, the sequences of the first constant region (CH1) of a human IgG1, IgG2, IgG3, and IgG4 differ at residues that are not within the meditope-binding region of cetuximab, confirming that the meditope-enabling technology is applicable to isotypes other than the IgG1 of cetuximab. As another example, the sequence and structural alignment of an IgG1 and an IgE Fab domain indicates residues on the IgE near the meditope binding site.

The provided methods for meditope-site grafting of the Fab cavity within monoclonal antibodies can be used to create a unique handle for meditope binding and used with the technology previously disclosed and for newly-generated antibodies. In certain embodiments, the meditope binding site can be created on pre-existing and all future monoclonal antibodies.

Also provided are methods for modifying the meditope-enabled antibodies, for example, to alter various properties of the meditope-enabled antibodies, including aspects of the interaction with the meditopes, including affinity, avidity, pH-dependence, as well as other aspects, including pharmacokinetics (PK) and pharmacodynamics (PD) of the antibodies. Thus, also among the provided meditope-enabled antibodies are those modified antibodies generated according to those methods, e.g., by generating a pharmacophore binding model, including antibodies having any one or more of the modifications described in section F, below.

Also among the antibodies used as template antibodies to generate the meditope-enabled antibodies are modified antibodies and portions thereof, such as a CovX-Body™. Thus, among the provided meditope-enabled antibodies are CovX-bodies, modified to allow their binding to one or more of the provided meditopes, e.g., with an affinity as described herein.

Also provided are complexes containing one or more meditope bound to a meditope-enabled antibody, such as any of the antibodies described herein.

Also provided are nucleic acids, such as cDNA and RNA molecules, encoding the meditope-enabled antibodies, and vectors and libraries containing the same, as well as methods for their use, including selection and expression methods and methods for generating transgenic animals using such constructs.

### D. Methods of use and compositions

Also provided are methods and uses of the meditopes, including multivalent meditopes. Among the methods are methods of delivering meditopes, antibodies, and/or agents conjugated thereto to cells and tissues expressing antigens recognized by the meditope-enabled antibodies.

In some embodiments, the provided methods utilize properties of tumor cells and alter the internalization and sorting pathway of conjugated biologics to efficiently release the agent. In some embodiments, the methods effect co-localization, clustering, aggregation, internalization, and/or intracellular trafficking or sorting of the antigen and/or meditope-enabled antibodies, and/or recapitulation or enhancement or alteration thereof, for example, by administering multivalent meditope in connection with, e.g., in conjunction with, before, or following, administration of meditope-enabled antibodies. Observed herein are differences in clustering and/or internalization of such surface receptors following administration to cells of meditope-enabled antibodies recognizing such receptor antigens and multivalent meditopes, in comparison to administration of antibodies alone. Among the provided embodiments are methods comprising administering multivalent meditopes, which promote co-localization and clustering of surface antigens recognized by meditope-enabled antibodies (meditope enabled antibodies), and/or internalization/trafficking/sorting of such antigens, such as cell-surface antigens such as receptors.

Also provided are methods and uses of the multivalent meditopes in delivering meditopes and/or coupled agents to cells, for example, in imaging and therapeutic methods. Exemplary methods are those effecting delivery of a cytotoxin or radiolabeled agent coupled to the multivalent meditope. In some embodiments, the methods provide an increased degree and/or speed of internalization of a cell-surface antigen. Increased or more rapid internalization can be advantageous, for example, in the context of ADC or other uses in which the antibody is used to deliver an agent to a cell or in which the antibody is delivered to promote downregulation of a surface receptor or other surface antigen associated with or causative in the progression of disease, such as cancer, for example, by targeting it for degradation. A slower degree or less rapid internalization may be desirable in some contexts, such as those in which the antibody is delivered to promote effector functions such as ADCC and/or complement-mediated lysis, and/or to engage and promote signaling via the cell-surface receptor antigens.

Provided are methods for delivering meditopes and/or for enhancing or increasing co-localization or clustering of cell-surface antigens on target cells. In some embodiments, the methods are carried out by administering to the cells

one or more meditope-enabled antibody that specifically binds to the cell surface antigen(s) and a multivalent meditope. In some embodiments, co-localization, clustering, and/or aggregation of the antigen is enhanced or increased. In some aspects, the increase is an increase in degree, speed, and/or duration in comparison to that observed prior to or in the absence of the addition of the meditope-enabled antibody and/or the multivalent meditope, or in the presence of the antibody without the meditope. In some embodiments, the method promotes or enhances the degree of a signal via the cell surface antigen and/or an effector function mediated via the bound antibody.

Provided are methods for delivering meditopes, agents coupled thereto, and/or for enhancing or increasing internalization and/or degradation of cell-surface antigens on target cells. In some embodiments, the methods are carried out by administering to the cells one or more meditope-enabled antibody that specifically binds to the cell surface antigen(s) and a multivalent meditope. In some embodiments, internalization and/or degradation of the antigen is promoted, enhanced or increased. In some aspects, the increase is an increase in degree, speed, and/or duration in comparison to that observed prior to or in the absence of the addition of the meditope-enabled antibody and/or the multivalent meditope, or in the presence of the antibody without the meditope. In some embodiments, the meditope is coupled to a therapeutic or diagnostic agent, such as a chemotherapeutic, cytotoxin, or radiolabeled agent, such as for use in ADC or RIT. In some embodiments, the methods alter internalization or intracellular trafficking.

In some aspects, the multivalent meditope is used instead of a second antibody or bispecific antibody, e.g., to achieve synergy in combination with a meditope-enabled antibody. In some aspects, the multivalent meditopes provide advantages compared to use of a second antibody recognizing a separate epitope on the same antigen. For example, production of the multivalent meditopes can be more efficient, and cost-effective when compared to a second antibody. For example, although a number of preclinical/clinical trials are investigating the co-administration of two monoclonal antibodies, the costs of producing and marketing such a therapeutic is likely to be prohibitive. In some aspects, the multivalent meditopes also are comparatively more easily targeted to disease sites, such as tumors. Given the nature of the meditope binding site (within the meditope binding site, separate from the antibody CDRs), and the broadly applicable methods provided herein for meditope-enabling any antibody of choice, the multivalent meditopes also have the advantage of being readily applicable to a large range of therapeutic antibodies, without the need to identify a second antibody or epitope with therapeutically acceptable characteristics. Thus, in some aspects, use of a multivalent meditope avoids the need to identify a second mAb with acceptable characteristics, and the associated and significant cost of its development thereof.

In some embodiments, the multivalent meditopes provide advantages over and/or improvements in bivalent antibodies. In some embodiments, the multivalent meditope is used with a bispecific meditope-enabled antibody, e.g., to enhance aggregation of two antigens, e.g., cell-surface antigens, bound by the different arms of the bispecific antibody, for example, to enhance affinity, signaling induced by the antibody, effector functions, and/or internalization, as compared to use of the bispecific antibody alone. Thus, in some aspects, the multivalent meditope is used in connection with a bispecific meditope-enabled antibody and provides an increased level of efficacy, for example, by causing an additional degree of clustering as compared to the bispecific antibody alone.

In some embodiments, two meditope-enabled antibodies, each of which specifically binds to a different antigen expressed on the surface of the same cell, together with a multivalent meditope, are used in place of a bivalent antibody, providing enhanced antigen (e.g., receptor) aggregation, and/or a certain level aggregation regardless of the ratio of the density or presence of the two antigens relative to one another on the cell surface. In some aspects, such embodiments enhance cell-surface clustering of antigens and/or co-localization. In one embodiment, a multivalent meditope is used to achieve such clustering together with multiple meditope-enabled antibodies recognizing multiple different antigens. Thus, in some embodiments, as compared to a bispecific antibody, in which clustering would occur only where each arm of the antibody bound its respective antigen, clustering in such an embodiment may be achieved even where the antigen bound by one of the antibodies is present at a much higher density on the cell surface compared to the antigen bound by another.

In some embodiments, the methods enhance delivery of an agent, such as in ADC or RIT or other methods for antibody-mediated delivery to a cell or tissue expressing an antigen targeted by the antibody. In some embodiments, the methods are advantageous in that they promoter clustering and therefore internalization of antigens, antibodies bound to the antigens, and agents associated with the antibodies, in the context of non-internalizing antigens, such as carcinoembryonic antigen (CEA), tumor-associated glycoprotein (TAG-72), KS1/4 (*see* Schmitd et al., Cancer Immunol Immunother. Dec 2008; 57(12): 1879-1890; Myer et al. Cancer Res 1993;53:3956-3963), and CD20.

The methods include those involving administration of meditopes, e.g., multivalent meditopes, and the meditope-enabled antibodies, and complexes containing the same, including therapeutic and diagnostic uses, as well as other uses, including antibody purification. Also provided are pharmaceutical compositions containing the peptidyl central cavity binding-kappa light chain binding fusion proteins, meditopes (including variant and multivalent meditopes and meditope fusion proteins) for use in such diagnostic and therapeutic methods.

Antibodies and binding compounds (e.g., meditopes) provided have practical utility and in some embodiments can overcome hurdles associated with available mAb-based technologies. In some embodiments, the provided compounds and methods address the problem of antigenicity that can arise with non-native reagents, which for example, expose new surfaces, which can be antigenic, and/or concerns with stability and the scalability in the manufacturing of such molecules (Nelson AL (2010) Antibody fragments: hope and hype. MAbs 2(1):77-83). Cetuximab is used successfully in the clinic and is stable and produced at scale. In some embodiments, the unique combination of residues that line the meditope binding site is distant in sequence (i.e., unlikely to be a peptide T-cell epitope) and located within a deep cavity (i.e., unlikely to be a surface exposed B-cell epitope)). Thus, in some embodiments, mAbs containing the meditope binding site are not immunogenic.

Data herein demonstrate stable interaction of bivalent meditope-Fc compounds with meditope enabled antibody-treated cells. The provided compounds and methods in some embodiments are useful in pre-targeted imaging and drug delivery methods, such as those described by Goldenberg DM, et al. (2012) Pretargeted molecular imaging and radioimmunotherapy. Theranostics 2(5):523-540; Pagel JM, et al. (2006) Comparison of a tetravalent single-chain antibody-streptavidin fusion protein and an antibody-streptavidin chemical conjugate for pretargeted anti-CD20 radioimmunotherapy of B-cell lymphomas. Blood 108(1):328-336.

In some embodiments, the compounds and methods are useful in deriving synergistic tumor inhibition (Kamat, V., et al., Cancer Biol Ther 7, 726-733 (2008); Koefoed K, et al. (2011) Rational identification of an optimal antibody mixture for targeting the epidermal growth factor receptor. MAbs 3(6):584-595; Spangler JB, et al. (2010) Combination antibody treatment down-regulates epidermal growth factor receptor by inhibiting endosomal recycling. Proc Natl Acad Sci USA 107(30):13252-13257), as observed in the trastuzumab-pertuzumab combination (Baselga J, et al. (2012) Pertuzumab plus trastuzumab plus docetaxel for metastatic breast cancer. N Engl J Med 366(2):109-119). In some examples, bivalent meditopes, such as the meditope-Fc, function as second "mAb"-like molecules, but with the flexibility of being able to pair up with any meditope enabled antibody. In some aspects, this reduces the cost of identifying and developing a second therapeutic mAb. In some embodiments, multivalent meditope variants (e.g., generated by altering valency and/or linker geometry) further enhance specificity and synergistic effects.

In some embodiments, the meditope binding site is used as a specific point of conjugation, useful in avoiding issues of heterogeneity and conjugation efficiency that typically affect the development of effective ADCs by available methods. A toxin-bearing meditope in some embodiments can direct a reactive chemical to the Fab cavity, favoring specificity and unit stoichiometry.

In some aspects, moderate affinity of meditopes (e.g., cQFD and cQYN meditopes) is advantageous, such as when considering a multivalent approach ((Mammen M, Choi S, & Whitesides G (1998) Polyvalent interactions in biological systems: Implications for design and use of multivalent ligands and inhibitor. Angew. Chem., Int. Ed. Eng 37:2755)), in some embodiments, different un-natural amino acids, cyclization strategies, and/or screening methods are used to improve affinity and/or pharmacokinetics / pharmacodynamics / toxicity. As demonstrated herein, in some embodiments, it is equally possible to tailor the meditope binding site to accommodate changes in the meditope.

## I. Therapeutic and diagnostic uses and pharmaceutical compositions

In one embodiment, the specificity and binding of the meditopes, meditope variants, multivalent meditopes, MPLs, multivalent meditope tethering agents and multivalent meditope variant tethering agents, are used to deliver therapeutic agents, diagnostic agents (e.g., imaging agents), or a combination thereof for treatment, diagnosis (e.g., imaging) a disease or condition, typically when administered in combination with (either simultaneously or separately) one or more meditope-enabled monoclonal antibodies.

In one example, meditopes, e.g., multivalent meditopes, are used for pre-targeted therapy or pre-targeted imaging, as described further below, by administering a meditope-enabled monoclonal antibody before administering the meditopes, meditope variants, multivalent meditope tethering agents or multivalent meditope variant tethering agents. Further, the use of multivalent meditopes can enhance selectivity and improve tumor detection as has been demonstrated for engineered scFvs or chemically conjugated mAbs, but avoids potential immunogenicity inherent in these non-human constructs.

Thus, the platform technology described herein has a broad impact on the mAb delivery field and provides useful methods for the treatment and diagnosis of various diseases and conditions, including cancers, such as those against which use of an antibody is indicated. For example, meditope-enabled antibodies directed against EGFR-positive cancers, including colorectal and squamous cell carcinoma head and neck cancers, would benefit from use of where cetuximab and a meditope. Additionally, modifying other therapeutic antibodies to generate meditope-enabled versions of such antibodies allows for the platform technology to be utilized in methods for the treatment and diagnosis of several other cancers, diseases and other conditions.

Use of the meditopes in such methods is advantageous, for example, by way of specificity, e.g., as compared to other means for binding or conjugation to antibodies. For example, PpL (Protein L) and SpA (Protein A) are not murine-specific or chimeric antibody-specific. PpL binds to -66% of murine and -50% of human IgG Kappa light chains and SpA binds to 12% of murine and 50% of human variable heavy chains (Graille et al., 2002). In contrast, as demonstrated

herein, the interaction of meditopes with meditope-enabled antibodies is highly specific, which can avoid adverse effects, including off-target effects, e.g., avoiding binding of the therapeutic compound to immunoglobulins endogenous to the subject.

In some embodiments, a meditope administered in combination with a meditope enabled antibody, an antibody-meditope complex, a meditope administered in combination with a meditope enabled antibody, or a combination thereof may be conjugated to one or more imaging agent. In one aspect, an imaging agent may include, but is not limited to a fluorescent, luminescent, magnetic protein, or radionuclide protein, peptide or derivatives thereof (e.g., genetically engineered variants). Fluorescent proteins that may be expressed by the mRNA component include green fluorescent protein (GFP), enhanced GFP (EGFP), red, blue, yellow, cyan, and sapphire fluorescent proteins, and reef coral fluorescent protein. Luminescent proteins that may be expressed by the mRNA component include luciferase, aequorin and derivatives thereof. Numerous fluorescent and luminescent dyes and proteins are known in the art (see, e.g., U.S. Patent Application Publication 2004/0067503; Valeur, B., "Molecular Fluorescence: Principles and Applications," John Wiley and Sons, 2002; Handbook of Fluorescent Probes and Research Products, Molecular Probes, 9.sup.th edition, 2002; and The Handbook--A Guide to Fluorescent Probes and Labeling Technologies, Invitrogen, 10th edition, available at the Invitrogen web site; both of which are hereby incorporated by reference as if fully set forth herein.)

In other aspects, the meditope administered in combination with a meditope enabled antibody, the antibody-meditope complex, the meditope administered in combination with a meditope enabled antibody, or a combination thereof may be further conjugated to or otherwise associated with a non-protein imaging agent or a delivery vehicle such as a nanoparticle, radioactive substances (e.g., radioisotopes, radionuclides, radiolabels or radiotracers), dyes, contrast agents, fluorescent compounds or molecules, bioluminescent compounds or molecules, enzymes and enhancing agents (e.g., paramagnetic ions). In addition, it should be noted that some nanoparticles, for example quantum dots and metal nanoparticles (described below) may also be suitable for use as an imaging agent or a therapeutic agent (e.g., using hyperthermal and photodynamic therapies as well as imaging agents through fluorescence and or MRI contrast).

The meditope-mAb technology allows for a system that may be used to generate an antibody-meditope complex that may be further conjugated to one or more meditope-enabled antibody, a therapeutic agent, an imaging agent or a combination thereof. Thus, a set of meditopes or high affinity meditope variants, each conjugated to a unique cytotoxic or imaging agent, would allow for the co-administration of a desired meditope conjugate and meditope-enabled mAb for treatment. The meditope conjugates are an improvement over the current antibody-drug conjugates, which have drawbacks such as a reduced specificity due to chemical modification, release of payload, and inconsistent payload conjugation. A method for enhancing the binding affinity of a therapeutic antibody or functional fragment thereof is provided herein. Such a method may include administering to a subject a therapeutically effective amount of a pharmaceutical composition via any suitable route of administration. The pharmaceutical composition may include a meditope or meditope variant in combination with a meditope enabled antibody, a multivalent meditope or meditope variant tethering entity in combination with a meditope enabled antibody, a meditope-enabled therapeutic antibody or functional fragment thereof, a pharmaceutically acceptable carrier, and any combination thereof. The enhanced binding affinity of the multivalent meditope may be attributed to the multivalent cross-linking of IgGs bound to the cell surface. Crosslinking IgGs (through parental murine M425 antibody or using anti-IgG IgM) significantly affects signaling, receptor endocytosis and recycling, and cell death. Thus, multivalent peptides may act synergistically with a therapeutic monoclonal antibody to enhance its therapeutic efficacy.

In some embodiments, the meditope, alone or as part of the tethering entity, may contain a cysteine or other suitable alkylating agent that binds to a Fab cysteine at the binding site, thus creating a cysteine-cysteine interaction. Alternatively, the meditope may bind to the Fab at an unnatural amino acid (e.g., p-acetylphenylalanine). The Cys meditope is conjugated to any substance and directs the conjugate to the IgG.

An antibody-meditope complex may also be used in a method for directing treatment to a particular type of cell or population of cells in a disease or condition that can be targeted by a therapeutic antibody. Such a method of treatment may include administering a therapeutically effective amount of a pharmaceutical composition to a subject having the disease or condition via any suitable route of administration. The pharmaceutical composition may include a meditope or meditope variant in combination with a meditope enabled antibody, a multivalent meditope or meditope variant tethering entity in combination with a meditope enabled antibody, a meditope-enabled therapeutic antibody or functional fragment thereof.

In other embodiments, a method for imaging tumors or other tissues is provided. In such methods, an unmodified therapeutic antibody may be administered to target a tumor or other tissue that overexpress the corresponding antigen. Subsequently, a multivalent meditope tethering entity that is labeled with an imaging agent is administered via any suitable route of administration and will bind to the therapeutic antibodies that are bound to the target tumor or tissue. See Figure 1. Examples of imaging agents include but are not limited to radiolabels (e.g., $^{3}H$, $^{14}C$, $^{35}S$, $^{90}Y$, $^{99m}Tc$, $^{125}I$, $^{131}I$, $^{177}Lu$, $^{166}Ho$, and $^{153}Sm$), metal or magnetic labels (e.g., gold, iron, gadolinium), biotin, chelating agents (e.g., 1,4,7,10-tetraazacyclododecane-N,N', N",N"'-tetraacetic acid ("DOTA")) or any agent described above. In one embodiment, the imaging agent used with the method described herein is DOTA.

There are several advantages over known methods to the imaging or treatment methods described above. First, the bivalent character of a therapeutic monoclonal antibody enhances the selectivity to the tumor. As discussed further below, this enhanced selectivity is lost with the use of scFvs and is not entirely compensated for by enhanced affinity. Second, the mass of the labeled multivalent meditope tethering entity will be below the renal threshold for filtering (less than 60 kDa, may be as low as ~10 kDa), allowing it to be easily filtered out of the body. In contrast, direct labeling of a therapeutic antibody with an imaging agent or other therapeutic agent is typically avoided because it will circulate for extended periods (days to weeks) in the body. Thus, imaging of tumors or other diseased organs is often accomplished using less selective scFvs.

In further embodiments, the meditopes may be used to join two or more therapeutic molecules to form a pharmaceutical compound that may be administered, as part of a pharmaceutical composition, in a therapeutically effective amount to a subject for treatment of cancer, autoimmune disease or other conditions. The two or more therapeutic molecules may include, but are not limited to, functional antibody fragments (e.g., $F(ab')_2$ or Fab fragments), peptides or other small molecules that can target tumor or disease-specific receptors such as those described above. The therapeutic molecules may be two or more of the same therapeutic molecule or alternatively, may be two or more different molecules that target the same tumor or diseased tissue.

In some embodiments, the pharmaceutical compound or composition may include a proprietary antibody, or portion thereof, such as a CovX-Body™. In one example, the meditopes are used as linkers to join two or more therapeutic molecules to a specially designed CovX antibody. In one example, a small molecule, peptide or scFv associated with such a meditope is recognized by the meditope-binding site (e.g., framework binding interface) of the CovX antibody. When these components are combined, the resulting bivalent CovX-Body™ possesses the biologic actions of the small molecule, peptide or scFv while also retaining an extended half-life of the antibody.

In some embodiments, the provided meditopes, meditope-enabled antibodies, and complexes thereof, are used in treatment, diagnosis or imaging of a disease or condition, including any cancer, disease or other condition that may be treated or targeted using a therapeutic antibody. Cancers avoid immune surveillance by actively suppressing the immune system. One method envisioned for counteracting this immunosuppression is through vaccination using epitopes of antigens that are either uniquely expressed or over-expressed by the tumor cells. For example, monoclonal antibodies (mAbs) that block signaling pathways, sequester growth factor and/or induce an immune response have been successfully implemented in the clinic to treat cancer and other diseases.

Thus, the diseases and conditions include any cancer, as well as other diseases and conditions, such as those targeted using therapeutic mAbs, including, but not limited to, leukemia and lymphomas (which can be treated or imaged using, e.g., meditope-enabled versions of alemtuzumab, bectumomab, gemtuzumab, FBTA05, ibritumomab tiuzetan, ofatumumab, rituximab, tositumomab), breast cancer (which can be treated or imaged using, e.g., meditope-enabled versions of trastuzumab, adecatumumab, ertumaxomab) prostate cancer (which can be treated or imaged using, e.g., meditope-enabled versions of adecatumumab, capromab pendetide, etaracizumab), colorectal cancer (which can be treated or imaged using, e.g., meditope-enabled versions of labetuzumab, panitumumab, altumumab pentetate, votumumab), gastrointestinal cancers (which can be treated or imaged using, e.g., meditope-enabled versions of arcitumumab, catumaxomab), ovarian cancer (which can be treated or imaged using, e.g., meditope-enabled versions of abagovomab, catumaxomab, etaracizumab, igovomab, oregovomab), lung cancer (which can be treated or imaged using, e.g., meditope-enabled versions of anatumumab mafenatox), pancreatic cancer (which can be treated or imaged using, e.g., meditope-enabled versions of clivatuzumab tetraxetan), renal cancer (which can be treated or imaged using, e.g., meditope-enabled versions of girentuximab), melanoma cancer (which can be treated or imaged using, e.g., meditope-enabled versions of etaracizumab, ipilimumab, TRBS07), glioma (which can be treated or imaged using, e.g., meditope-enabled versions of nimotuzumab), bone metastases (which can be treated or imaged using, e.g., meditope-enabled versions of denosumab), head and neck cancer (which can be treated or imaged using, e.g., meditope-enabled versions of zalutumumab), cardiovascular disease (which can be treated or imaged using, e.g., meditope-enabled versions of abciximab), autoimmune disorders (which can be treated or imaged using, e.g., meditope-enabled versions of adalimumab, infliximab), rheumatoid arthritis (which can be treated or imaged using, e.g., meditope-enabled versions of atlizumab, golimumab, infliximab), transplant rejection (which can be treated or imaged using, e.g., meditope-enabled versions of basiliximab, daclizumab, muromonab-CD3), Crohn's disease (which can be treated or imaged using, e.g., meditope-enabled versions of certolizumab, fontolizumab, natalizumab, infliximab, visilizumab), hemoglobinuria (which can be treated or imaged using, meditope-enabled versions of eculizumab), psoriasis (which can be treated or imaged using, e.g., meditope-enabled versions of efalizumab, infliximab, ustekinumab), multiple sclerosis (which can be treated or imaged using, e.g., meditope-enabled versions of natalizumab, ustekinumab), asthma (which can be treated or imaged using, e.g., meditope-enabled versions of benralizumab, mepolizumab, omalizumab), respiratory syncytial virus (RSV) (which can be treated or imaged using, e.g., meditope-enabled versions of palivizumab), macular degeneration (which can be treated or imaged using, e.g., meditope-enabled versions of ranibizumab), appendicitis (which can be treated or imaged using, e.g., meditope-enabled versions of fanolesomab) and any other condition that may be targeted or treated with an antibody. The above-listed antibodies and related diseases or disorders are examples only and do not limit the

platform.

In certain embodiments, one or more meditopes, meditope variants, multivalent meditope tethering agents or multivalent meditope variant tethering agents may be conjugated to one or more imaging agents, therapeutically effective agents or compounds in therapeutically effective amounts or both, such that the binding of the meditopes or variants thereof to one or more meditope-enabled antibody with the therapeutically effective compound may treat, prevent, diagnose or monitor a disease or condition. Such conjugation of a high affinity and/or multivalent meditope coupled to meditope-enabled mAbs provides a highly versatile platform technology that will significantly improve mAb based therapeutics and imaging methods to treat and detect disease (see Figure 1).

In some embodiments, the provided methods and compounds are useful in antibody engineering, antibody-drug conjugates (ADCs), antibody-directed enzyme prodrug therapies (ADEPT), immune system engagement (e.g., Fc modifications (Desjarlais JR & Lazar GA (2011) Modulation of antibody effector function. Exp Cell Res 317(9):1278-1285), chemokine fusion, bi-specific T-cell engagers(Wolf E, Hofmeister R, Kufer P, Schlereth B, & Baeuerle PA (2005) BiTEs: bispecific antibody constructs with unique anti-tumor activity. Drug Discov Today 10(18):1237-1244) and disease imaging (e.g., immunoPET and pre-targeted radionuclide imaging (Goldenberg DM, et al. (2012) Pretargeted molecular imaging and radioimmunotherapy. Theranostics 2(5):523-540; Pagel JM, et al. (2006) Comparison of a tetravalent single-chain antibody-streptavidin fusion protein and an antibody-streptavidin chemical conjugate for pretargeted anti-CD20 radioimmunotherapy of B-cell lymphomas. Blood 108(1):328-336).

In some aspects, the provided methods and compounds overcome issues observed in available such methods, for example, those using post-translational chemical modifications or manipulation of the gene sequence, which can for example lead to undesirable consequences that are detrimental to therapeutic and imaging applications. For example, with ADCs targeting cytotoxins to disease sites using available methods, chemical conjugation of the toxin (typically involves lysines, reduced cysteines, or sugars on the mAb) can produce a heterogeneous mixture, which can adversely affect the specificity and stability of the mAb and alter its biodistribution.

As another example, reducing the size of the mAb to Fab fragments (e.g., single chain Fab variable domain (scFvs)) to facilitate tumor penetration and enhance imaging using available methods can reduce the valency and thus affect both the affinity and the tissue specificity compared to the original mAb. In other cases, with available methods, as part of a pre-targeted imaging protocol to enhance detection, mAbs conjugated with streptavidin or multiple Fabs/scFvs stitched together through a scaffold (e.g., "lock-and-dock") can be immunogenic, unstable in serum, and technically difficult and expensive to produce, especially at scales necessary for clinical use. The provided compounds and methods in some embodiments are useful in overcoming such difficulties, for example, by providing an alternative mAb platform and improved mAb-based delivery systems for cancer and other diseases and conditions.

Having described the invention with reference to the embodiments and illustrative examples, those in the art may appreciate modifications to the invention as described and illustrated that do not depart from the spirit and scope of the invention as disclosed in the specification. The Examples are set forth to aid in understanding the invention but are not intended to, and should not be construed to limit its scope in any way. The examples do not include detailed descriptions of conventional methods. Such methods are well known to those of ordinary skill in the art and are described in numerous publications. Further, all references cited above and in the examples below are hereby incorporated by reference in their entirety, as if fully set forth herein.

The following documents are hereby incorporated by reference in their entirety for all purposes: United States Application Serial Number 61/597,708, filed February 10, 2012; United States Application Serial Number 13/270,207, filed October 10, 2011, now U.S. Patent No. 8,658,774; International Application Number PCT/US2011/055656 filed October 10, 2011; International Application Number PCT/US12/32938 filed April 10, 2012; United States Application Serial Number 13/443,804, filed April 10, 2012, now U.S. Patent No. 8,962,804; United States Application Serial Number 61/749,830, filed February 10, 2012, and United States Application Serial Number 13/764,762, filed February 11, 2013.

## E. Definitions

An "antibody," as used herein, refers to an immunoglobulin molecule that specifically binds to, or is immunologically reactive with an antigen or epitope, and includes both polyclonal and monoclonal antibodies, as well as functional antibody fragments , including but not limited to fragment antigen binding (Fab) fragments, $F(ab')_2$ fragments, Fab' fragments, Fv fragments, recombinant IgG (rIgG) fragments, single chain variable fragments (scFv) and single domain antibodies (e.g., sdAb, sdFv, nanobody) fragments. The term "antibody" includes genetically engineered or otherwise modified forms of immunoglobulins, such as intrabodies, peptibodies, chimeric antibodies, fully human antibodies, humanized antibodies, meditope-enabled antibodies and heteroconjugate antibodies (e.g., bispecific antibodies, diabodies, triabodies, tetrabodies, tandem di-scFv, tandem tri-scFv). Unless otherwise stated, the term "antibody" should be understood to encompass functional antibody fragments thereof.

The terms "complementarity determining region," and "CDR," are known in the art to refer to non-contiguous sequences of amino acids within antibody variable regions, which confer antigen specificity and binding affinity. In

general, there are three CDRs in each heavy chain variable region (CDR-H1, CDR-H2, CDR-H3) and three CDRs in each light chain variable region (CDR-L1, CDR-L2, CDR-L3). "Framework regions" and "FR" are known in the art to refer to the non-CDR portions of the variable regions of the heavy and light chains. In general, there are four FRs in each heavy chain variable region (FR-H1, FR-H2, FR-H3, and FR-H4), and four FRs in each light chain variable region (FR-L1, FR-L2, FR-L3, and FR-L4).

The precise amino acid sequence boundaries of a given CDR or FR can be readily determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), MacCallum et al., J. Mol. Biol. 262:732-745 (1996), "Antibody-antigen interactions: Contact analysis and binding site topography," J. Mol. Biol. 262, 732-745." (Contact" numbering scheme), Lefranc MP et al., "IMGT unique numbering for immunoglobulin and T cell receptor variable domains and Ig superfamily V-like domains," Dev Comp Immunol, 2003 Jan;27(1):55-77 ("IMGT" numbering scheme), and Honegger A and Plückthun A, "Yet another numbering scheme for immunoglobulin variable domains: an automatic modeling and analysis tool," J Mol Biol, 2001 Jun 8;309(3):657-70, (AHo numbering scheme).

The boundaries of a given CDR or FR may vary depending on the scheme used for identification. For example, the Kabat scheme is based structural alignments, while the Chothia scheme is based on structural information. Numbering for both the Kabat and Chothia schemes is based upon the most common antibody region sequence lengths, with insertions accommodated by insertion letters, for example, "30a," and deletions appearing in some antibodies. The two schemes place certain insertions and deletions ("indels") at different positions, resulting in differential numbering. The Contact scheme is based on analysis of complex crystal structures and is similar in many respects to the Chothia numbering scheme.

Table 4, below, lists the positions of CDR-L1, CDR-L2, CDR-L3 and CDR-H1, CDR-H2, CDR-H3 as identified by the Kabat, Chothia, and Contact schemes, respectively. For CDR-H1, residue numbering is given listed using both the Kabat and Chothia numbering schemes. It is noted that because the Kabat numbering scheme places insertions at H35A and H35B, the end of the Chothia CDR-H1 loop when numbered using the Kabat numbering convention varies between H32 and H34, depending on the length of the loop.

**Table 4.**

| CDR | Kabat | Chothia | Contact |
|---|---|---|---|
| CDR-L1 | L24--L34 | L24--L34 | L30--L36 |
| CDR-L2 | L50--L56 | L50--L56 | L46--L55 |
| CDR-L3 | L89--L97 | L89--L97 | L89--L96 |
| CDR-H1 (Kabat Numbering[1]) | H31--H35B | H26-H32..34 | H30-H35B |
| CDR-H1 (Chothia Numbering[2]) | H31--H35 | H26--H32 | H30--H35 |
| CDR-H2 | H50--H65 | H52--H56 | H47--H58 |
| CDR-H3 | H95--H102 | H95--H102 | H93-H101 |
| 1 - Kabat et al. (1991), "Sequences of Proteins of Immunological Interest," 5th Ed. Public Health Service, National Institutes of Health, Bethesda, MD<br>2 - Al-Lazikani et al., (1997) JMB 273,927-948 | | | |

Thus, unless otherwise specified, a "CDR" or "complementary determining region," or individual specified CDRs (e.g., "CDR-H1, CDR-H2), of a given antibody or region thereof, such as a variable region thereof, should be understood to encompass a (or the specific) complementary determining region as defined by any of the known schemes. Likewise, unless otherwise specified, a "FR" or "framework region," or individual specified FRs (e.g., "FR-H1, FR-H2), of a given antibody or region thereof, such as a variable region thereof, should be understood to encompass a (or the specific) framework region as defined by any of the known schemes. In some instances, the scheme for identification of a particular CDR, FR, or FRs or CDRs is specified, such as the CDR as defined by the Kabat, Chothia, or Contact method. In other cases, the particular amino acid sequence of a CDR or FR is given.

The term "meditope-enabled" antibody and "meditope enabled antibody" and "meditope enabled antibody" refer to an antibody or functional fragment thereof that is able to bind to a meditope, via a meditope binding site. Examples of meditope-enabled antibodies include, but are not limited to, cetuximab and others described herein. A "meditope binding site" is a region of the meditope-enabled antibody containing the amino acid residues that interact with a bound meditope, which residues include framework region (FR) residues of the heavy and light chains. With reference to a Fab

fragment or a Fab portion of an antibody, the meditope binding site is located within the central cavity of the Fab fragment or portion.

The "central cavity," with respect to the three-dimensional structure of a Fab, refers to the internal cavity of the Fab, lined by portions of the heavy and light chain variable and constant regions. The central cavity thus is lined by residues of the VH, VL, CH1, and CL regions and does not include the antigen binding site.

In some embodiments, the meditope binding site includes residues 40, 41, 83, and 85 of the light chain of a meditope-enabled antibody, according to Kabat numbering, and/or residues 39, 89, 105, and 108 of the heavy chain of the meditope-enabled antibody, according to Kabat numbering.

In some embodiments, the meditope binding site is located within a cavity formed by residues 8, 9, 10, 38, 39, 40, 41 42, 43, 44, 45, 82, 83, 84, 85, 86, 87, 99, 100, 101, 102, 103, 104, 105, 142, 162, 163, 164, 165, 166, 167, 168, and 173 of the light chain and 6, 9, 38, 39, 40, 41, 42, 43, 44, 45, 84, 86, 87, 88, 89, 90, 91, 103, 104, 105, 106, 107, 108, 111, 110, 147, 150, 151, 152, 173, 174, 175, 176, 177, 185, 186, and 187 of the heavy chain of the antibody, according to Kabat numbering.

With respect to a Fab portion of a meditope-enabled antibody, the meditope binding site includes residues within the central cavity. The meditope-binding site typically further includes constant region residues.

A "therapeutic agent," as used herein, is an atom, molecule, or compound that is useful in treatment of a disease or condition.

A "therapeutically effective amount," "therapeutically effective concentration" or "therapeutically effective dose" is the amount of a compound that produces a desired therapeutic effect in a subject, such as preventing or treating a target condition, alleviating symptoms associated with the condition, producing a desired physiological effect, or allowing imaging or diagnosis of a condition that leads to treatment of the disease or condition. The precise therapeutically effective amount is the amount of the composition that will yield the most effective results in terms of efficacy of treatment in a given subject. This amount will vary depending upon a variety of factors, including, but not limited to, the characteristics of the therapeutic compound (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and the route of administration. One skilled in the clinical and pharmacological arts will be able to determine a therapeutically effective amount through routine experimentation, namely by monitoring a subject's response to administration of a compound and adjusting the dosage accordingly. For additional guidance, see Remington: The Science and Practice of Pharmacy 21st Edition, Univ. of Sciences in Philadelphia (USIP), Lippincott Williams & Wilkins, Philadelphia, PA, 2005.

A "pharmaceutically acceptable carrier" refers to a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or some combination thereof. Each component of the carrier must be "pharmaceutically acceptable" in that it must be compatible with the other ingredients of the formulation. It also must be suitable for contact with any tissue, organ, or portion of the body that it may encounter, meaning that it must not carry a risk of toxicity, irritation, allergic response, immunogenicity, or any other complication that outweighs its therapeutic benefits.

A "route of administration" may refer to any administration pathway known in the art, including but not limited to aerosol, enteral, nasal, ophthalmic, oral, parenteral, rectal, transdermal, or vaginal. "Transdermal" administration may be accomplished using a topical cream or ointment or by means of a transdermal patch. "Parenteral" refers to a route of administration that is generally associated with injection, including infraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal.

"In combination" or "in combination with," when used herein in the context of multiple agents, therapeutics, or treatments, means in the course of treating the same disease or condition in a subject administering two or more agents, drugs, treatment regimens, treatment modalities or a combination thereof (e.g., an antibody in combination with a meditope or a meditope), in any order. This includes simultaneous administration (or "coadministration"), administration of a first agent prior to or after administration of a second agent, as well as in a temporally spaced order of up to several days apart. Such combination treatment may also include more than a single administration of any one or more of the agents, drugs, treatment regimens or treatment modalities. Further, the administration of the two or more agents, drugs, treatment regimens, treatment modalities or a combination thereof may be by the same or different routes of administration.

A "therapeutic antibody" may refer to any antibody or functional fragment thereof that is used to treat cancer, autoimmune diseases, transplant rejection, cardiovascular disease or other diseases or conditions such as those described herein. Examples of therapeutic antibodies that may be used according to the embodiments described herein include, but are not limited to murine antibodies, murinized or humanized chimera antibodies or human antibodies including, but not limited to, Erbitux (cetuximab), ReoPro (abciximab), Simulect (basiliximab), Remicade (infliximab);

Orthoclone OKT3 (muromonab-CD3); Rituxan (rituximab), Bexxar (tositumomab) Humira (adalimumab), Campath (alemtuzumab), Simulect (basiliximab), Avastin (bevacizumab), Cimzia (certolizumab pegol), Zenapax (daclizumab), Soliris (eculizumab), Raptiva (efalizumab), Mylotarg (gemtuzumab), Zevalin (ibritumomab tiuxetan), Tysabri (natalizumab), Xolair (omalizumab), Synagis (palivizumab), Vectibix (panitumumab), Lucentis (ranibizumab), and Herceptin (trastuzumab).

"Treating" or "treatment" of a condition may refer to preventing the condition, slowing the onset or rate of development of the condition, reducing the risk of developing the condition, preventing or delaying the development of symptoms associated with the condition, reducing or ending symptoms associated with the condition, generating a complete or partial regression of the condition, or some combination thereof.

As used herein, the terms "including," "containing," and "comprising" are used in their open, non-limiting sense.

As used herein, the singular forms "a," "an," and "the" include plural referents unless the context clearly dictates otherwise.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

As used herein, "alkyl" refers to a saturated, straight- or branched-chain hydrocarbon group having from 1 to 12 carbon atoms. Representative alkyl groups include, but are not limited to, methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, n-pentyl, isopentyl, neopentyl, n-hexyl, and the like, and longer alkyl groups, such as heptyl, octyl, and the like. The term "$C_{x-y}$alkyl," where x and y are integers, refers to an alkyl with x-y carbon atoms.

As used herein, an "alkenyl" refers to a straight- or branched-chain hydrocarbon group having one or more double bonds therein and having from 2 to 12 carbon atoms. Illustrative alkenyl groups include, but are not limited to, ethylenyl, vinyl, allyl, butenyl, pentenyl, hexenyl, butadienyl, pentadienyl, hexadienyl, 2-ethylhexenyl, 2-propyl-2-butenyl, 4-(2-methyl-3-butene)-pentenyl, and the like. The term "$C_{x-y}$alkenyl," where x and y are integers, refers to an alkenyl with x-y carbon atoms.

The term "alkylenyl" or "alkylene" refers to a divalent alkyl group. The term "alkenylene" or "alkenylene" refers to a divalent alkenyl group.

As used herein, "alkynyl" refers to a straight- or branched-chain hydrocarbon group having one or more triple bonds therein and having from 2 to 10 carbon atoms. Exemplary alkynyl groups include, but are not limited to, ethynyl, propynyl, butynyl, pentynyl, hexynyl, methylpropynyl, 4-methyl-1-butynyl, 4-propyl-2-pentynyl, 4-butyl-2-hexynyl, and the like.

The term "heteroalkyl," by itself or in combination with another term, means, unless otherwise stated, a stable straight or branched chain, or combinations thereof, including at least one carbon atom and at least one heteroatom selected from the group consisting of O, N, P, Si, and S, and wherein the nitrogen and sulfur atoms may optionally be oxidized, and the nitrogen heteroatom may optionally be quaternized. The heteroatom(s) O, N, P, S, and Si may be placed at any interior position of the heteroalkyl group or at the position at which the alkyl group is attached to the remainder of the molecule. Examples include, but are not limited to: $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-CH_2$, $-S(O)-CH_3$, $-CH_2-CH_2-S(O)_2-CH_3$, $-CH=CH-O-CH_3$, $-Si(CH_3)_3$, $-CH_2-CH=N-OCH_3$, $-CH=CH-N(CH_3)-CH_3$, $-O-CH_3$, $-O-CH_2-CH_3$, and -CN. Up to two or three heteroatoms may be consecutive, such as, for example, $-CH_2-NH-OCH_3$ and - $CH_2-O-Si(CH_3)_3$.

Similarly, the term "heteroalkylene," by itself or as part of another substituent, means, unless otherwise stated, a divalent radical derived from heteroalkyl, as exemplified, but not limited by, $-CH_2-CH_2-S-CH_2-CH_2-$ and $-CH_2-S-CH_2-CH_2-NH-CH_2-$. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula $-C(O)_2R'-$ represents both $-C(O)_2R'-$ and $-R'C(O)_2-$. As described above, heteroalkyl groups, as used herein, include those groups that are attached to the remainder of the molecule through a heteroatom, such as -C(O)R', -C(O)NR', -NR'R", -OR', -SR', and/or $-SO_2R'$. Where "heteroalkyl" is recited, followed by recitations of specific heteroalkyl groups, such as -NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R" or the like.

The terms "cycloalkyl" and "heterocycloalkyl," by themselves or in combination with other terms, mean, unless otherwise stated, cyclic versions of "alkyl" and "heteroalkyl," respectively. Additionally, for heterocycloalkyl, a heteroatom

can occupy the position at which the heterocycle is attached to the remainder of the molecule. Examples of cycloalkyl include, but are not limited to, cyclopropyl, cyclobutyl, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like. A "cycloalkylene" and a "heterocycloalkylene," alone or as part of another substituent, means a divalent radical derived from a cycloalkyl and heterocycloalkyl, respectively.

The term "aryl" means, unless otherwise stated, a polyunsaturated, aromatic, hydrocarbon substituent, which can be a single ring or multiple rings (preferably from 1 to 3 rings) that are fused together (i.e., a fused ring aryl) or linked covalently. A fused ring aryl refers to multiple rings fused together wherein at least one of the fused rings is an aryl ring. The term "heteroaryl" refers to aryl groups (or rings) that contain at least one heteroatom such as N, O, or S, wherein the nitrogen and sulfur atoms are optionally oxidized, and the nitrogen atom(s) are optionally quaternized. Thus, the term "heteroaryl" includes fused ring heteroaryl groups (i.e., multiple rings fused together wherein at least one of the fused rings is a heteroaromatic ring). A 5,6-fused ring heteroarylene refers to two rings fused together, wherein one ring has 5 members and the other ring has 6 members, and wherein at least one ring is a heteroaryl ring. Likewise, a 6,6-fused ring heteroarylene refers to two rings fused together, wherein one ring has 6 members and the other ring has 6 members, and wherein at least one ring is a heteroaryl ring. And a 6,5-fused ring heteroarylene refers to two rings fused together, wherein one ring has 6 members and the other ring has 5 members, and wherein at least one ring is a heteroaryl ring. A heteroaryl group can be attached to the remainder of the molecule through a carbon or heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable substituents described below. An "arylene" and a "heteroarylene," alone or as part of another substituent, mean a divalent radical derived from an aryl and heteroaryl, respectively. Non-limiting examples of heteroaryl groups include pyridinyl, pyrimidinyl, thiophenyl, furanyl, indolyl, benzoxadiazolyl, benzodioxolyl, benzodioxanyl, thianaphthanyl, pyrrolopyridinyl, indazolyl, quinolinyl, quinoxalinyl, pyridopyrazinyl, quinazolinonyl, benzoisoxazolyl, imidazopyridinyl, benzofuranyl, benzothiophenyl, phenyl, naphthyl, biphenyl, pyrrolyl, pyrazolyl, imidazolyl, pyrazinyl, oxazolyl, isoxazolyl, thiazolyl, furylthienyl, pyridyl, pyrimidyl, benzothiazolyl, purinyl, benzimidazolyl, isoquinolyl, thiadiazolyl, oxadiazolyl, pyrrolyl, diazolyl, triazolyl, tetrazolyl, benzothiadiazolyl, isothiazolyl, pyrazolopyrimidinyl, pyrrolopyrimidinyl, benzotriazolyl, benzoxazolyl, or quinolyl. The examples above may be substituted or unsubstituted and divalent radicals of each heteroaryl example above are non-limiting examples of heteroarylene.

The term "boronic ester" refers to a substituent $-B(OR)_2$, wherein each R group is independently a $C_{1-4}$alkyl, or the two R groups taken together form a $C_{2-6}$alkylene.

The term "acetal" refers to a $-CH(OR)_2$ group, wherein wherein each R group is independently a $C_{1-4}$alkyl, or the two R groups taken together form a $C_{2-6}$alkylene. Exemplary acetal groups include dimethylacetal or diethylacetal, or a cyclic acetal. The term "ketal" refers to a $-C(OR)_2-$ group, wherein each R group is independently a $C_{1-4}$alkyl, or the two R groups taken together form a $C_{2-6}$alkylene. Exemplary ketals include dimethylketal or diethylketal, or a cyclic ketal.

The term "halo" represents chloro, fluoro, bromo, or iodo. In some embodiments, halo is chloro, fluoro, or bromo. The term "halogen" as used herein refers to fluorine, chlorine, bromine, or iodine. The term "ortho ester" refers to a $-C(OR)_3$ group, wherein each R group is independently a $C_{1-4}$alkyl, or two of the R groups taken together form a $C_{2-6}$alkylene. The term "oxo" means an =O group and may be attached to a carbon atom or a sulfur atom. The term "phosphonate ester" refers to a $-P(O)(OR)_2$ group, wherein each R group is independently a $C_{1-4}$alkyl, or the two R groups taken together form a $C_{2-6}$alkylene

As used herein, the term "cycloalkyl" refers to a saturated or partially saturated, monocyclic, fused polycyclic, bridged polycyclic, or spiro polycyclic carbocycle having from 3 to 15 ring carbon atoms. A non limiting category of cycloalkyl groups are saturated or partially saturated, monocyclic carbocycles having from 3 to 6 carbon atoms. Illustrative examples of cycloalkyl groups include, but are not limited to, the following moieties:

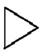, 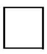, 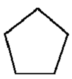, 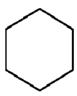, 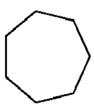, 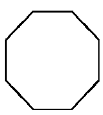, 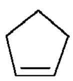, 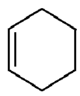, 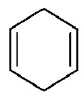, 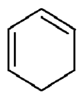,

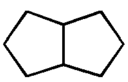, 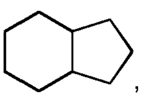, 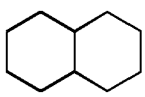, 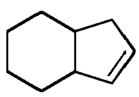, 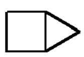,

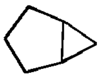, 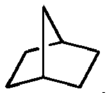, 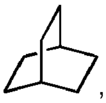, 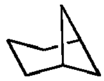, and 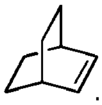.

As used herein, the term "5-membered heteroaryl" refers to a monocyclic, aromatic heterocycle having five ring atoms that are selected from carbon, oxygen, nitrogen, and sulfur. Examples of 5-membered heteroaryl groups include, but are not limited to, imidazolyl, pyrazolyl, triazolyl, tetrazolyl, furanyl, thienyl, isoxazolyl, thiazolyl, oxazolyl, isothiazolyl, pyrrolyl, and thiadiazolyl. Particular examples of 5-membered heteraryls include those that may be formed by 1,3-cycloaddition reactions such as a Huisgen reaction between an azide and a propargyl group.

As used herein, the term "substituted" means that the specified group or moiety bears one or more suitable substituents. As used herein, the term "unsubstituted" means that the specified group bears no substituents. As used herein, the term "optionally substituted" means that the specified group is unsubstituted or substituted by the specified number of substituents. Where the term "substituted" is used to describe a structural system, the substitution is meant to occur at any valency-allowed position on the system. As used herein, the expression "one or more substituents" denotes one to maximum possible number of substitution(s) that can occur at any valency-allowed position on the system. In a certain embodiment, "one or more substituents" means 1, 2, 3, 4, or 5 substituents. In another embodiment, one or more substituent means 1, 2, or 3 substituents.

A "substituent group" or "substituent" as used herein, means a group selected from the following moieties:

- (A) oxo,
  halogen, $-CF_3$, -CN, -OH, $-NH_2$, -COOH, $-CONH_2$, $-NO_2$, -SH, - $SO_2Cl$, $-SO_3H$, $-SO_4H$, $-SO_2NH_2$, $-NHNH_2$, $-ONH_2$, $-NHC=(O)NHNH_2$, -NHC=(O) $NH_2$, $-NHSO_2H$, -NHC= (O)H, - NHC(O)-OH, -NHOH, $-OCF_3$, $-OCHF_2$, unsubstituted alkyl, unsubstituted heteroalkyl, unsubstituted cycloalkyl, unsubstituted heterocycloalkyl, unsubstituted aryl, unsubstituted heteroaryl, and

- (B) alkyl, heteroalkyl, cycloalkyl, heterocycloalkyl, aryl, and heteroaryl, substituted with at least one substituent selected from:

  i. oxo,
  halogen, $-CF_3$, -CN, -OH, $-NH_2$, -COOH, $-CONH_2$, $-NO_2$, -SH, $-SO_2Cl$, $-SO_3H$, $-SO_4H$, $-SO_2NH_2$, $-NHNH_2$, $-ONH_2$, $-NHC=(O)NHNH_2$, -NHC=(O) $NH_2$, $-NHSO_2H$, -NHC= (O)H, -NHC(O)-OH, -NHOH, $-OCF_3$, $-OCHF_2$, unsubstituted alkyl, unsubstituted heteroalkyl, unsubstituted cycloalkyl, unsubstituted heterocycloalkyl, unsubstituted aryl, unsubstituted heteroaryl, and

  ii. alkyl, heteroalkyl, cycloalkyl, heterocycloalkyl, aryl, and heteroaryl, substituted with at least one substituent selected from:

    1. oxo, halogen, $-CF_3$, -CN, -OH, $-NH_2$, -COOH, $-CONH_2$, $-NO_2$, -SH, $-SO_2Cl$, $-SO_3H$, $-SO_4H$, $-SO_2NH_2$, $-NHNH_2$, $-ONH_2$, $-NHC=(O)NHNH_2$, $-NHC=(O)NH_2$, - $NHSO_2H$, -NHC= (O)H, -NHC(O)-OH, -NHOH, $-OCF_3$, $-OCHF_2$, unsubstituted alkyl, unsubstituted heteroalkyl, unsubstituted cycloalkyl, unsubstituted heterocycloalkyl, unsubstituted aryl, unsubstituted heteroaryl, and

    2. alkyl, heteroalkyl, cycloalkyl, heterocycloalkyl, aryl, or heteroaryl, substituted with at least one substituent selected from: oxo,
    halogen, $-CF_3$, -CN, -OH, $-NH_2$, -COOH, $-CONH_2$, $-NO_2$, -SH, $-SO_2Cl$, $-SO_3H$, $-SO_4H$, $-SO_2NH_2$, $-NHNH_2$, $-ONH_2$, $-NHC=(O)NHNH_2$, $-NHC=(O)NH_2$, - $NHSO_2H$, -NHC= (O)H, -NHC(O)-OH, -NHOH, $-OCF_3$, $-OCHF_2$, unsubstituted alkyl, unsubstituted heteroalkyl, unsubstituted cycloalkyl, unsubstituted heterocycloalkyl, unsubstituted aryl, and unsubstituted heteroaryl.

A "size-limited substituent" or " size-limited substituent group," as used herein, means a group selected from all of the substituents described above for a "substituent group" wherein each substituted or unsubstituted alkyl is a substituted or unsubstituted $C_1$-$C_{20}$ alkyl, each substituted or unsubstituted heteroalkyl is a substituted or unsubstituted 2 to 20 membered heteroalkyl, each substituted or unsubstituted cycloalkyl is a substituted or unsubstituted $C_3$-$C_8$ cycloalkyl, each substituted or unsubstituted heterocycloalkyl is a substituted or unsubstituted 3 to 8 membered heterocycloalkyl, each substituted or unsubstituted aryl is a substituted or unsubstituted $C_6$-$C_{10}$ aryl, and each substituted or unsubstituted heteroaryl is a substituted or unsubstituted 5 to 10 membered heteroaryl. A "lower substituent" or " lower substituent group," as used herein, means a group selected from all of the substituents described above for a "substituent

group," wherein each substituted or unsubstituted alkyl is a substituted or unsubstituted $C_1$-$C_8$ alkyl, each substituted or unsubstituted heteroalkyl is a substituted or unsubstituted 2 to 8 membered heteroalkyl, each substituted or unsubstituted cycloalkyl is a substituted or unsubstituted $C_3$-$C_7$ cycloalkyl, each substituted or unsubstituted heterocycloalkyl is a substituted or unsubstituted 3 to 7 membered heterocycloalkyl, each substituted or unsubstituted aryl is a substituted or unsubstituted $C_6$-$C_{10}$ aryl, and each substituted or unsubstituted heteroaryl is a substituted or unsubstituted 5 to 9 membered heteroaryl.

In some embodiments, each substituted group described in the compounds herein is substituted with at least one substituent group. More specifically, in some embodiments, each substituted alkyl, substituted heteroalkyl, substituted cycloalkyl, substituted heterocycloalkyl, substituted aryl, substituted heteroaryl, substituted alkylene, substituted heteroalkylene, substituted cycloalkylene, substituted heterocycloalkylene, substituted arylene, and/or substituted heteroarylene described in the compounds herein are substituted with at least one substituent group. In other embodiments, at least one or all of these groups are substituted with at least one size-limited substituent group. In other embodiments, at least one or all of these groups are substituted with at least one lower substituent group.

In other embodiments herein, each substituted or unsubstituted alkyl may be a substituted or unsubstituted $C_1$-$C_{20}$ alkyl, each substituted or unsubstituted heteroalkyl is a substituted or unsubstituted 2 to 20 membered heteroalkyl, each substituted or unsubstituted cycloalkyl is a substituted or unsubstituted $C_3$-$C_8$ cycloalkyl, each substituted or unsubstituted heterocycloalkyl is a substituted or unsubstituted 3 to 8 membered heterocycloalkyl, each substituted or unsubstituted aryl is a substituted or unsubstituted $C_6$-$C_{10}$ aryl, and/or each substituted or unsubstituted heteroaryl is a substituted or unsubstituted 5 to 10 membered heteroaryl. In some embodiments of the compounds herein, each substituted or unsubstituted alkylene is a substituted or unsubstituted $C_1$-$C_{20}$ alkylene, each substituted or unsubstituted heteroalkylene is a substituted or unsubstituted 2 to 20 membered heteroalkylene, each substituted or unsubstituted cycloalkylene is a substituted or unsubstituted $C_3$-$C_8$ cycloalkylene, each substituted or unsubstituted heterocycloalkylene is a substituted or unsubstituted 3 to 8 membered heterocycloalkylene, each substituted or unsubstituted arylene is a substituted or unsubstituted $C_6$-$C_{10}$ arylene, and/or each substituted or unsubstituted heteroarylene is a substituted or unsubstituted 5 to 10 membered heteroarylene.

In some embodiments, each substituted or unsubstituted alkyl is a substituted or unsubstituted $C_1$-$C_8$ alkyl, each substituted or unsubstituted heteroalkyl is a substituted or unsubstituted 2 to 8 membered heteroalkyl, each substituted or unsubstituted cycloalkyl is a substituted or unsubstituted $C_3$-$C_7$ cycloalkyl, each substituted or unsubstituted heterocycloalkyl is a substituted or unsubstituted 3 to 7 membered heterocycloalkyl, each substituted or unsubstituted aryl is a substituted or unsubstituted $C_6$-$C_{10}$ aryl, and/or each substituted or unsubstituted heteroaryl is a substituted or unsubstituted 5 to 9 membered heteroaryl. In some embodiments, each substituted or unsubstituted alkylene is a substituted or unsubstituted $C_1$-$C_8$ alkylene, each substituted or unsubstituted heteroalkylene is a substituted or unsubstituted 2 to 8 membered heteroalkylene, each substituted or unsubstituted cycloalkylene is a substituted or unsubstituted $C_3$-$C_7$ cycloalkylene, each substituted or unsubstituted heterocycloalkylene is a substituted or unsubstituted 3 to 7 membered heterocycloalkylene, each substituted or unsubstituted arylene is a substituted or unsubstituted $C_6$-$C_{10}$ arylene, and/or each substituted or unsubstituted heteroarylene is a substituted or unsubstituted 5 to 9 membered heteroarylene.

Any atom that is represented herein with an unsatisfied valence is assumed to have the sufficient number of hydrogen atoms to satisfy the atom's valence. When any variable, such as alkyl, $R^3$, or $R^5$, appears in more than one place in any formula or description provided herein, the definition of that variable on each occurrence is independent of its definition at every other occurrence. Numerical ranges, as used herein, are intended to include sequential whole numbers. For example, a range expressed as "from 0 to 4" or "0-4" includes 0, 1, 2, 3 and 4. When a multifunctional moiety is shown, the point of attachment to the core is indicated by a line or hyphen. For example, -OH refers to a moiety in which an oxygen atom is the point of attachment of the hydroxyl group to the remainder of the molecule.

Any formula given herein is intended to represent compounds having structures depicted by the structural formula as well as certain variations or forms. E.g., compounds of any formula herein may have asymmetric or chiral centers and therefore exist in different stereoisomeric forms. All stereoisomers, including optical isomers, enantiomers, and diastereomers, of the compounds of the general formula, and mixtures thereof, are considered to fall within the scope of the formula. Furthermore, certain structures may exist as geometric isomers (i.e., *cis* and *trans* isomers), as tautomers, or as atropisomers. All such isomeric forms, and mixtures thereof, are contemplated herein as part of the present invention. Thus, any formula given herein is intended to represent a racemate, one or more enantiomeric forms, one or more diastereomeric forms, one or more tautomeric or atropisomeric forms, and mixtures thereof.

Diastereomeric mixtures may be separated into their individual diastereomers on the basis of their physical chemical differences by methods well known to those skilled in the art, such as, for example, by chromatography and/or fractional crystallization. Enantiomers may be separated by converting the enantiomeric mixture into a diastereomeric mixture by reaction with an appropriate optically active compound (e.g., chiral auxiliary such as a chiral alcohol or Mosher's acid chloride, or formation of a mixture of diastereomeric salts), separating the diastereomers and converting (e.g., hydrolyzing or de-salting) the individual diastereomers to the corresponding pure enantiomers. Enantiomers may also be separated by use of chiral HPLC column. The chiral centers of compounds of the present invention may be

designated as "R" or "S" as defined by the IUPAC 1974 Recommendations, or by "D" or "L" designations consistent with the peptide literature.

As used herein, a box around a portion of a structure, drawn with a subscript, indicates that the structural fragment that appears within the box is repeated according to the subscript. For example, the substructure:

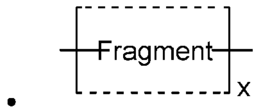

where x is 0, 1, or 2, indicates the fragment is absent from the structure, or is -Fragment-, or is -Fragment-Fragment-. For example, within Formula VII, the following substructure:

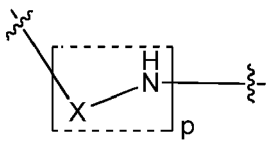

where p is 0 or 1, means that the -X-NH- group within the box is absent from the structure (p is 0), or is present once (p is 1).

The compounds of the invention can form pharmaceutically acceptable salts, which are also within the scope of this invention. A "pharmaceutically acceptable salt" refers to a salt of a free acid or base of a compound described herein that is non-toxic, is physiologically tolerable, is compatible with the pharmaceutical composition in which it is formulated, and is otherwise suitable for formulation and/or administration to a subject. Reference to a compound herein is understood to include reference to a pharmaceutically acceptable salt of said compound unless otherwise indicated.

Compound salts include acidic salts formed with inorganic and/or organic acids, as well as basic salts formed with inorganic and/or organic bases. In addition, where a given compound contains both a basic moiety, such as, but not limited to, a pyridine or imidazole, and an acidic moiety, such as, but not limited to, a carboxylic acid, one of skill in the art will recognize that the compound may exist as a zwitterion ("inner salt"); such salts are included within the term "salt" as used herein. Salts of the compounds of the invention may be prepared, for example, by reacting a compound with an amount of a suitable acid or base, such as an equivalent amount, in a medium such as one in which the salt precipitates or in an aqueous medium followed by lyophilization.

Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate ("mesylate"), ethanesulfonate, benzenesulfonate, *p*-toluenesulfonate, and pamoate (i.e., 1,1'-methylene-bis(2-hydroxy-3-naphthoate)) salts. A pharmaceutically acceptable salt may involve the inclusion of another molecule such as an acetate ion, a succinate ion or other counterion. The counterion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counterions. Hence, a pharmaceutically acceptable salt can have one or more charged atoms and/or one or more counter ion.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates,) and the like.

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, t-butyl amines, and salts with amino acids such as arginine, lysine and the like. Basic nitrogen-containing groups may be quarternized with agents such as lower alkyl halides (e.g. methyl, ethyl, and butyl chlorides, bromides and iodides), dialkyl sulfates (e.g. dimethyl, diethyl, and dibutyl sulfates), long chain halides (e.g. decyl, lauryl, and stearyl chlorides, bromides and iodides), aralkyl halides (e.g. benzyl and phenethyl bromides), and others.

Acids and bases generally considered suitable for the formation of pharmaceutically useful salts from pharmaceutical compounds are discussed, e.g., by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19;

P. Gould, International J. of Pharmaceutics (1986) 33 201-217; Anderson et al, The Practice of Medicinal Chemistry (1996), Academic Press, New York; and in The Orange Book (Food & Drug Administration, MD, available from FDA). These disclosures are incorporated herein by reference thereto.

Any compound described herein is intended to refer also to any unsolvated form, or a hydrate, solvate, or polymorph of such a compound, and mixtures thereof, even if such forms are not listed explicitly. "Solvate" means a physical association of a compound of the invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of a crystalline solid. "Solvate" encompasses both solution-phase and isolatable solvates. Suitable solvates include those formed with pharmaceutically acceptable solvents such as water, ethanol, and the like. In some embodiments, the solvent is water and the solvates are hydrates.

Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, phosphorous, fluorine, chlorine, and iodine, such as $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{36}Cl$, and $^{125}I$, respectively. Such isotopically labelled compounds are useful in metabolic studies (for example with $^{14}C$), reaction kinetic studies (with, for example $^{2}H$ or $^{3}H$), detection or imaging techniques [such as positron emission tomography (PET) or single-photon emission computed tomography (SPECT)] including drug or substrate tissue distribution assays, or in radioactive treatment of patients. In particular, an $^{18}F$ or $^{11}C$ labeled compound may be particularly suitable for PET or SPECT studies. Further, substitution with heavier isotopes such as deuterium (i.e., $^{2}H$) may afford certain therapeutic advantages resulting from greater metabolic stability, for example increased *in vivo* half-life or reduced dosage requirements. Isotopically labeled compounds of this invention and prodrugs thereof can generally be prepared by carrying out the procedures disclosed in the schemes or in the examples and preparations described below by substituting a readily available isotopically labeled reagent for a non-isotopically labeled reagent.

## EXAMPLES

### EXAMPLE 1: MEDITOPE Fc BINDING

A meditope-Fc construct was generated by fusing a meditope to the N-terminus of an Fc domain via a poly-glycine-serine linker. The gene for this meditope-Fc construct was constructed by DNA2.0 (cQFD meditope connected to human IgG gamma heavy chain 3 (accession# AAW65947) residues 22-241 via a 37-residue linker, cloned into pAcGP67A vector (BD Biosciences) and produced in Sf9 cells. The protein was purified using Protein A and size exclusion chromatography. The meditope-Fc included a polypeptide having an amino acid sequence as set forth in SEQ ID NO: 57 (shown below with a leader ("G") in position 1, meditope bolded, linker italicized, Fc portion in plain text):

G**CQFDLSTRRLRC***GGSRSGGTSGGGSVPGSGSSGSTSGSGKSSEG*
*SGQAS*THTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSV
MHEALHNHYTQKSLSLSPGK (SEQ ID NO: 57).

Using this construct, it was demonstrated by size exclusion chromatography (SEC) that cetuximab Fab, EGFRdIII and meditope form a complex. The meditope-Fc construct was added to a stoichiometric complex of EGFRdIII and cetuximab Fab and observed a new peak, which eluted much earlier than the Fab-EGFRdIII complex, consistent with an increase in the hydrodynamic mass. SDS-PAGE of this new peak showed the presence of all three proteins. These studies further confirmed that the meditope did not significantly interfere with antigen recognition.

### EXAMPLE 2: GENERATION OF A MEDITOPE-ENABLED TRASTUZUMAB

A meditope binding site was grafted onto a human mAb framework to generate a meditope-enabled antibody

(meditope enabled antibody), specifically, a meditope enabled antibody trastuzumab. The sequences of cetuximab and trastuzumab were aligned and sequence identity mapped onto the atomic model of trastuzumab (1N8Z, see Cho et al., Nature 421, 756-760(2003)). Based on this mapping and the superposition of cQFD-cetuximab and trastuzumab structures, thirteen (13) residues were identified that differ between cetuximab and trastuzumab and whose side chains either make direct contact with the meditope or may indirectly affect meditope binding. These residues were: Thr40, Asn41, Asp85 in the light chain (see above), Arg39, and Arg45 in the light chain, which coordinate a non-bonded phosphate group and in some examples stabilize the loop containing Thr40 and Asn41 in the light chain, Val9 and Ile10 which participate in forming a shallow hydrophobic surface near the side chain of Leu10 in the meditope, and Gly42, Ser43, Ile83 and Ala100 of the light chain, as well as Ser40 and Ile89 in the heavy chain.

Equivalent positions in the trastuzumab sequence were mutated (to Thr40, Asn41, Asp85, Arg39, Arg45, Ile9, Leu10, Gly42, Ser43, Ile83 and Ala100 in the light chain and Ser40 and Ile89 in the heavy chain, based on Kabat numbering), and the protein produced and purified. Using SPR, it was shown that the cQFD meditope bound to the grafted trastuzumab Fab with similar affinity as cetuximab ($K_D$ = 1.2 $\mu$M). It was also observed that the meditope-enabled trastuzumab Fab bound to soluble HER2 with similar affinity as the Fab isolated from the commercial source, confirming that the mutations had no deleterious effect on antigen binding. It was demonstrated by size exclusion chromatography that the meditope-enabled trastuzumab, sHER2, and meditope-Fc co-eluted.

To further confirm that the point mutations did not significantly perturb the overall structure of the Fab, the Fab of the trastuzumab meditope enabled antibody was crystalized with and without the cQFD meditope. Well diffracting crystals were obtained in the presence of Protein A and Protein L to aid crystallization. The structures of the apo-meditope enabled antibody trastuzumab and the meditope-containing complex were solved at 1.95 and 2.0 Å, respectively. The parental trastuzumab Fab bound to Protein A and Protein L was also crystallized and solved to 2.08 Å resolution. Superposition of either the apo-parental trastuzumab Fab or HER2-bound parental trastuzumab Fab (22) onto "apo"-meditope enabled antibody trastuzumab revealed only minor changes in the overall structure (RMSD = 0.22 Å and 0.56 Å over 433 and 431 C$\alpha$ atoms, respectively). Superposition of the "apo"- and meditope-liganded meditope enabled antibody trastuzumab showed that meditope occupancy stabilized the heavy chain loop (residues 39-44) in an open conformation, similar to what was observed for cetuximab, and accounted in part for the slightly higher RMSD. More importantly, residues (Thr40, Asn41 and Asp85) identified by the sequence mapping in the "apo"- and meditope-liganded meditope enabled antibody were essentially in the same positions as their counterparts in cetuximab, and formed similar interactions with the meditope. Important side chain rotamers of the meditope were also essentially the same as they were in the cQFD-cetuximab structure. As with cetuximab, significant alterations in the CDR loops were not observed.

These data demonstrate successful grafting of a meditope binding site onto a human Fab framework without significantly affecting antigen binding. The successful grafting of the meditope site further validates the initial cetuximab-meditope model obtained through diffraction data and the specificity of the interaction. The data further confirm the feasibility of enhancing meditope binding affinity through avidity, and show that a bivalent meditope is specific and binds with high affinity, but only in the presence of the cognate meditope enabled antibody, to cells overexpressing the antigen.

**EXAMPLE 3: MULTIVALENT, HIGH-AFFINITY MEDITOPES**

This example describes generation of multivalent meditopes with improved affinity, which is useful, for example, in antibody pre-targeted imaging and/or drug delivery. Specificity and affinity are commonly gained through avidity/multivalency (Mammen M, Choi S, & Whitesides G (1998) Polyvalent interactions in biological systems: Implications for design and use of multivalent ligands and inhibitor. Angew. Chem., Int. Ed. Eng 37:2755).

A bivalent meditope-Fc construct was made as described in Example 1, above; this compound was demonstrated to have enhanced apparent affinity as compared to the meditope to cell-associated cetuximab or cell-associated trastuzumab meditope enabled antibody. It was observed that the meditope-Fc (MFC) bound to MDA-MB-468 cells (an EGFR-overexpressing breast cancer cell line) pre-treated with cetuximab and remained bound despite an extensive washing procedure (essentially a ~$10^6$ fold dilution of the bivalent meditope). The meditope-Fc construct did not bind to untreated cells or cells pre-treated with M425, a murine anti-EGFR mAb that does not have the meditope binding site (Kamat, V. et al., Cancer Biol Ther 7, 726-733 (2008); Rodeck U, et al., Cancer Res 47(14):3692-3696 (1987)). It also was demonstrated that an FITC-labeled, monomeric cQFD meditope bound to cetuximab pre-treated MDA-MB-468 cells, but its signal, as measured by FACS, was reduced to background levels after a single wash (-100 fold dilution and consistent with its binding affinity) (Figure 11).

The stable and specific interaction of the meditope-Fc with cetuximab was also clearly seen by its co-localization on the surface of MDA-MB-468 cells (Figure 11). These *in vitro* studies confirmed that the avidity gained by creating a bivalent meditope facilitated its selective binding to cells that express pathological concentrations of EGFR only in the presence of cetuximab.

Studies with the meditope-Fc were carried out using the meditope enabled antibody trastuzumab. The meditope enabled antibody trastuzumab was tested on HER2-overexpressing SKBR3 breast cancer cells. By both FACS and

fluorescent microcopy, the grafted trastuzumab bound to the cells at similar levels as the parental trastuzumab. FACS analysis showed that the trastuzumab meditope enabled antibody and the parental trastuzumab we produced had the same range of affinity to cell surface HER2 as the commercial trastuzumab (Figure 12). As with cetuximab, microscopy demonstrated that the meditope-Fc co-localized with meditope enabled antibody trastuzumab on SKBR3 cells. The same specific binding was recapitulated. The meditope enabled antibody trastuzumab and the parental control only differed at the grafted residues, thus confirming that the specificity originated entirely from the meditope-enabling point mutations.

The following multivalent meditope-Fc constructs also were generated.

G**CQFDLSTRRLRC***GGSRSGGTSGTGSTSGSGSSGSTSGSGKSSEGSGQASKG*THTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK*GGSRSGGTSSSSSGSGSGSSGSTSGSGSSG***CQFDLSTRRLRC**G; (SEQ ID NO: 58; a tetravalent-meditope-Fc);

GPGGSDPG**CQFDLSTRRLRC***GGSRSGGTSGTGSTSGSGSSGSTSGSGKSSEGSGQA*SKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK*GGSRSGGTSSSSSGSGSGSSGSTSGSGSSG***CQFDLSTRRLRC**G (SEQ ID NO: 59; a tetravalent Meditope-Fc in which the Fc portion contained only the CH3);

GPGGSDPG**CQFDLSTRRLRC***GGSRSGGTSGTGSTSGSGSSGSTSGSGKSSEGSGQA*SKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO: 64; a meditope-Fc in which the Fc portion contained only the CH3).

SEQ ID NO: 237-239 disclose additional multivalent meditope peptides.

In each of the above sequences, leader and tail sequences are underlined, where applicable, meditope sequences are bolded, linker sequences are italicized, and Fc portions set forth in plain text.

The tetravalent meditope-Fc was shown to bind with high affinity via meditope enabled antibody bound to cell surface antigen. In some embodiments, each or one or more of the above tetravalent and bivalent constructs are shown to bind, e.g., with high affinity via meditope enabled antibody bound to cell surface antigen.

## EXAMPLE 4: ASSESSMENT OF ANTIGEN LOCALIZATION AND DISTRIBUTION FOLLOWING ADMINISTRATION OF MEDITOPE-ENABLED ANTIBODIES AND MULTIVALENT MEDITOPES

This example describes super-resolution and confocal microscopy studies carried out using various fluorescently-labeled molecules. These studies were carried out to assess antigen localization and distribution, including internalization and clustering, following administration of meditope-enabled antibodies (meditope enabled antibodies) and

multivalent meditopes. Localization of the antibodies and labeled meditopes also were assessed following the administration.

Photoactivatable Her2 and EGFR constructs were generated to observe distribution of these receptor antigens following administration of antigen-specific antibodies alone and in combination with multivalent meditopes. Fluorescent probes were conjugated to the antibodies, e.g., meditope enabled antibodies, and/or multivalent meditopes to permit observation of their localization using fluorescence. Super-resolution and confocal microscopy were performed.

***A. Materials and Methods***

To permit visualization of cell surface antigens targeted by meditope-enabled antibodies, photoactivatable HER2 and EGFR constructs were generated. Labeled multivalent meditopes, including tetravalent meditopes, also were generated to visualize localization of the meditopes.

*perbB2-paGFP (or HER2-paGFP) site directed mutagenesis*

perbB2-EGFP pcDNA3.1(+) plasmid was purchased from Adgene (plasmid # 39321). This plasmid was used for iterative site directed mutagenesis (SDM) to mutate the EGFP coding region to produce region encoding photoactivatable (pa)GFP. Various primer pairs were used for iterative SDM to alter 5 amino acids in the EGFP coding region of the plasmid.

After each round of SDM, resulting DNA was transformed into BP5alpha competent cells (BIOPIONEER) and plated on LB/ampicillin agar plates at 37 °C. Single colonies were selected and amplified in 10 mL LB to purify plasmid DNA for use in a subsequent round of SDM. After the final round of SDM, the plasmid was subjected to Sanger sequencing at the City of Hope (COH) Integrative Genomics Core.

A Clustal alignment comparing the amino acid sequence of the protein encoded by the addgene construct versus that encoded by the HER2-paGFP vector was performed. The following mutations were found: L1335F, T1336S, V1434A, T1474H, and A1477K.

*Generation of perbB1-paGFP (or EGFR-paGFP)*

EGFR-GFP in pEGFP-N1 vector was obtained from Addgene (Plasmid ID 32751, Ullrich/Joseph Schelessinger, Yale University Axel Ullrich, Max Planck Institute of Biochemistry). PAGFP in an N1 vector was originally obtained from Jennifer Lippinott-Schwartz; it contained A206K mutation.

The EGFR-GFP DNA was sequenced using primers to map the construct. AgeI and BsrGI cut sites were used for cloning a PAGFP insert in place of GFP. Briefly, the vector and insert plasmids were digested using AgeI and BsrGI restriction enzymes (Thermo Scientific) for 30 minutes at 37 °C. The digested plasmids were visualized on a 2% agarose gel. The bands of appropriate sizes were cut and purified to generate vector and insert DNA and ligated using T4 DNA ligase enzyme (Thermo Scientific) for 30 minutes at room temperature. The ligation reaction product was transformed into competent BP5$\alpha$ bacterial cells and plated on kanamycin resistant plates. The plates were incubated overnight at 37 °C. Colonies were picked and grown in kanamycin supplemented LB broth. DNA was extracted and sent for sequencing to confirm EGFR-PAGFP construct.

*Production of tetravalent meditope-Fc (M4FC) and fluorescently labeled M4FC*

A tetravalent meditope-Fc having the amino acid sequence of SEQ ID NO: 58 and described Example 3 (M4FC) was produced and used in the studies in this example. The nucleotide sequence used to produce the M4FC is provided in SEQ ID NO: 223.

To produce the vector encoding this MM4Fc, the DNA was synthesized by DNA2.0. The construct was excised with BamHI and BglII and cloned into the secretion pAcGP67A vector from BD Biosciences.

*Baculovirus construction:*

Co-transfection was carried out using the BD BaculoGold Bright DNA from the BD BaculoGold™ Transfection Kit following the manufacturer's recommended protocol. 2 million Sf9 cells (Gibco) were plated onto a 60mm tissue culture plate in 2 mL of SFM-900 II medium (Invitrogen). After the cells had attached firmly, the SFM was removed and 1 mL of Transfection Buffer A was added with constant swirling. Meanwhile, 0.5 $\mu$g of BD BaculoGold Baculovirus DNA and 2 $\mu$g of pAcGP67A-M4FC vector were mixed and let sit for 5 min before 1 mL of Transfection Buffer B was mixed into the DNA mixture. The solution was then added drop-by-drop to the insect cells with constant rocking of the plate. The plate was incubated at 27 °C for 4 h, after which the transfection buffer was removed and replaced with 5 mL of

SFM. The plate was placed inside a 10 cm tissue culture dish with a wet kimwipe and incubated at 27 °C until all cells were dead.

*Baculovirus amplification:*

Inoculate 300 μL of P1 virus to a 150mm tissue culture plate were seeded with 20 × $10^6$ Sf9 cells in 40 mL SFM-900 II medium (Invitrogen). It was confirmed that all cells were dead by day 7. If not, volume P1 was increased at least by 3-fold. Cell killed by virus stayed attached to the plate; cells dying from overgrowth became detached. P2 was amplified in similar fashion, but confirming cells dead by day 5-6; P3 was amplified in similar fashion, but confirming cells dead by day 4-5. P3 obtained this way was assumed to be at titer=2 × $10^8$ pfu/mL.

*Expression:*

Hi-Five cells were inoculated in suspension culture (2 × $10^6$ cells/mL) with M.O.I.=3 (e.g., 3 mL P3 into 100 mL cells). An inoculation was deemed effective if it resulted in halted growth the following day and complete cell death by day 4. M.O.I of P3 was adjusted accordingly, as needed, up to 10. If needs to go beyond M.O.I=10, consider a P4 amplification.

In some cases, P3 was pre-incubated with cell culture of a higher density for 2 hour prior to bringing up to final volume to facilitate infection. Culture supernatant was generally harvested on day 3, or when >80% cells were positive for trypan blue, in order to prevent protein degradation due to presence of excess cell debris.

*Purification:*

Cell debris was spun down at 10,000 g or higher for at least 30 min at 4 °C. Supernatant was filtered through a 0.22 μm filter. In some cases, pH was assessed using a pH strip and if too acidic, pH was adjusted to 7-8 with 1M Tris, pH 8.0 or 10× PBS, pH 7.4; pH generally was close to neutral.

Either Protein A or Protein G was used for purification (with Protein A generally used for supernatants having a pH closer to 8). Columns were packed with Protein A or G agarose beads (Genscript) and equilibrated with 5 column volumes of 1xPBS. Filtered supernatant was passed through the column by gravity flow. In cases of large volumes (>1 L), columns were washed with 2-3 column volume 1xPBS intermittently. Columns were washed with 10 column volumes of 1xPBS.

0.7 column volume of 0.1 M glycine, pH 3.0, was applied and allowed to flow through. Protein was eluted with 2 column volumes of 0.1 M glycine, pH 3.0. It was ensured that eluting protein was dripping directly into a reservoir of 0.1 elution volume of 1M Tris, pH 9.0 for immediate neutralization and 0.1 column volume of 10x PBS to prevent precipitation. O.D. was measured at 280 nm to determine yield. (typicalyield was >30 mg/L).

To distinguish between M4FC and Fc only (degraded product), the protein preparation was assessed by analytical size exclusion chromatography. 5 μM cetuximab IgG was mixed with 6 μM crude M4FC in 80 μL final volume (adjust with 1xPBS), allowed to sit at room temperature for 5 min, and applied to a Superdex 200 10/300 column (GE Healthcare). Presence of the desired complex was verified by its elution around 10 mL.

Gel filtration size exclusion chromatography was used for the final round of purification, followed by buffer exchange by dialysis into 1x PBS. M4FC was concentrated to around 150 μM.

*Storage:*

M4FC was generally stable at 4 °C at most for one month. For long-term storage, the protein was flash-frozen in small aliquots and stored at -80 °C. Thawed M4FC was re-sized to remove aggregates.

*Alexa Fluor labeling:*

Typically, the M4FC protein was labeled in a 50 μL reaction. The protein concentration was adjusted to 2 mg/mL and mixed with 0.1 volume of 1 M sodium bicarbonate. Alexa Fluor powder/flake size of a 20 μL pipet tip was added directly to the protein mix and incubated at room temperature and protected from light for 30-60 min, depending on the amount of dye added and the degree of labeling desired. For fluorescent microscopy, the proteins generally were labeled with 3-5 dyes. Unreacted dye was separated by a Sephadex G50-Fine (GE Healthcare)-packed Bio-Spin column (Bio-Rad). The column was packed by filling it with 50% PBS-equilibrated Sephadex slurry and compacting the beads with a 3-5 min, 1,100 g spin in a 15 mL tube holder in a swing bucket centrifuge. The reaction mix was loaded on the top of the Sephadex column, and the labeled protein was collected in the eluate with a 2 min, 1,100 g spin. The labeled protein was buffer-exchanged into fresh PBS using a 10kDa MWCO Amicon Ultra-0.5 mL centrifugal filter unit (Millipore) and

0.22 μm filtered.

*Super-resolution Imaging of EGFR-PAGFP*

Coverslips were cleaned using Hellmanex II and basic piranha etch protocol. For PALM microscopy, cells were grown on clean coverslips coated with fibronectin-like engineered protein (25 μg/ml in PBS, pH 7.4, Sigma). MDA-MB-453 cells (originally obtained from the American Type Culture Collection, ATCC) were cultured in Phenol Red-free Dulbecco's Modified Eagle Medium: Nutrient Mixture F-12 (DMEM/F12) supplemented with 20% fetal bovine serum, 1 mM sodium pyruvate, 100 units/ml penicillin, 100 units/ml streptomycin, and 2.5 mM L-glutamine. 2 ug of EGFR-PAGFP construct was transiently transfected in MDA-MB-453 cells using FuGENE 6 (Promega) transfection reagent per manufacturer's instructions. Approximately 24 h after transfection, cells were washed quickly in phosphate buffered saline (PBS) pH = 7.4 at 37°C and fixed in 4% (w/v) paraformaldehyde and 0.2% (w/v) glutaraldehyde (Electron Microscopy Sciences) for 30 min at room temperature in PBS. Quenching was done with filter-sterilized 25 mM glycine in PBS for 10 min, and cells were finally washed three times with PBS. Coverslips were incubated with 1:4000 diluted TetraSpeck beads (Invitrogen) in PBS for 5 min that served as fiducial markers to correct for drift during image acquisition. Coverslips were imaged immediately after preparation in Attofluor cell chambers (Invitrogen) supplemented with PBS.

*Super-resolution Imaging of Her2-PAGFP*

Cells were plated on clean and coated coverslips. Her2-paGFP was transiently transfected into MDA-MB-453 (3 ug of DNA, Fugine HD transfection reagent, 48h) and MDA-MB-468 (2ug DNA, JetPrime transfection reagent, 24h or 48h) cells. Cells were incubated with 10 nM trastuzumab for 10 min, washed, fixed as described hereinabove and imaged. PC-PALM was used to define the distribution of Her2.

***B. Assessment of HER2 distribution following administration of meditope-enabled anti-HER2 antibody and multivalent meditopes***

Cells were assessed at the steady state (without addition of antibody) or following incubation for 10 minutes with trastuzumab which was not meditope-enabled). Distribution of Her2 was observed to follow single exponential distribution, implying single level of organization. In steady state (without antibody addition, at 24 hours post-transfection, based on assessment of fourteen areas from ten different cells, the transfected MDA-MB-468 cells displayed an average of 5 HER2 proteins per cluster. Density indicated that two populations co-existed: half of the observed areas had 3-5 proteins per cluster, while the other half of the observed areas had 6-9 proteins per cluster.

Following the 10 minute incubation with trastuzumab, Her2-paGFP transfected MDA-MB-468 cells displayed an average of 6 proteins per cluster.

In another study, the effects were compared to those observed following incubation with a multivalent meditope in combination with meditope-enabled antibody. The HER2-paGFP was expressed in MDA-468 cells as described above. Cells were incubated with 10 nM meditope-enabled Trastuzumab v2.0 with a heavy chain of SEQ ID NO: 9 and a light chain is the sequence of SEQ ID NO: 6 with 83E at 37 °C for 10 min. Cells were quickly washed once with pre-warmed (37 °C) media and then incubated with fresh 10 nM tetravalent meditope (M4Fc) at 37 °C for two different time points (3 min and 10 min).

The light chain sequence for 83E meditope enabled antibody trastuzumab is:

DIQMTQSPILLSASVGDRVTITCRASQDVNTAVAWYQQRTNGSPRLLIYSASFLYSGVPSRFSGSRSGT
DFTLTISSLQPEDEADYYCQQHYTTPPTFGAGTKVEIKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPVTK
SFNRGEC (SEQ ID NO: 241).

The VL sequence for 83E meditope enabled antibody trastuzumab is:

DIQMTQSPILLSASVGDRVTITCRASQDVNTAVAWYQQRTNGSPRLLIYSASFLYSGVPSRFSGSRSGT
DFTLTISSLQPEDEADYYCQQHYTTPPTFGAGTKVEIKRTVAAPSVFIFPP (SEQ ID NO: 242).

As shown in Figure 13, cells incubated with 10 nM M4Fc (tetravalent meditope) for 3 minutes were observed to have more dense HER2-paGFP, with 196 ± 41 molecules/$\mu m^2$ (n=9, #cells = 5) compared to 41 ± 8 molecules/$\mu m^2$

for cells incubated with 10 nM M4Fc for 10 minutes (n=8, #cells = 4). In addition, more clustering was observed following the 3-minute compared to the 10-minute incubation with M4Fc, as demonstrated by the higher number of HER2 proteins per cluster and larger cluster radius. After pre-incubation with a meditope-enabled antibody, addition of the tetravalent meditope Fc led by three minutes to rapid accumulation and clustering of the receptor antigens compared to incubation with a non-meditope-enabled form of the antibody alone. By 10 minutes, this incubation led to rapid internalization of these receptors as compared to incubation with the non-meditope-enabled form of the antibody alone. These data demonstrate that addition of a multivalent meditope affected the distribution and internalization of the cell-surface receptor antigen in comparison to incubation with antibody alone.

***C. Assessment of antibody and meditope-localization following administration of meditope-enabled antibody and multivalent meditope***

Effects on the endocytic process following incubation with meditope-enabled antibodies alone and with multivalent meditopes were assessed by confocal microscopy. Specifically, the effects a bivalent meditope Fc (MFC) and the tetravalent meditope-Fc described above (M4FC) were assessed by fluorescent microscopy. Alexa Fluor-labeled cetuximab without or in combination with each of these meditopes was co-incubated with MDA-MB-468 cells for 15 minutes, 1 hour and 40 minutes, 3 hours and 35 minutes, and 6 hours and 35 minutes. At the end of the incubation, the cells were fixed by formaldehyde, and where appropriate, Alexa Fluor-labeled antibodies were applied to stain for specific cellular markers before cover slips were mounted and the samples analyzed. Binding of the meditopes depended on the presence of the meditope-enabled antibody (cetuximab); meditope applied alone did not result in cell binding.

The bivalent meditope-Fc (MFC) co-localized with cetuximab almost completely throughout the co-incubation period; images demonstrated that it was endocytosed. The localization became more punctate, along with some separation of the meditope and antibody was observed after 4.5 hours of co-incubation. Staining of markers of early endosome (EEA1), lysosome (LAMP1 and LAMP2) and golgi (GM130) showed that except for a very minor overlapping of EEA1 and cetuximab/MFC, usually at the most concentrated spots in the cells, there was no co-localization of cetuximab/MFC within the classical compartments of the endocytotic pathway. APPL1 endosomes participate in growth factor receptor trafficking and signaling, are distinct from EEA1 early endosomes, and represents an early endocytic intermediate common to a subset of clathrin-derived endocytic vesicles and macropinosomes. Staining of APPL1 also showed that the intracellular cetuximab/MFC were not part of APPL1 endosomes.

The presence of MFC promoted the formation of the punctates. Incubation with cetuximab alone resulted in some punctate formation; however, the dots were much more pronounced. Using ImagePro software to count the dots, there were approximately twice as many dots in observed in the cells incubated with cetuximab +MFC as opposed to cetuximab alone. The dots also were on average twice as bright in those cells (75 dots at 200 lumin/pixel$^2$ in the +MFC cell vs. 37 dots at 100 lumin/pixel$^2$ in the cetuximab only cells). The appearance of the punctates was also observed to depend on the bivalent interaction of the antibody to MFC. When cetuximab Fab portions (monovalent) were used instead (incubated with MFC), even after four hours of co-incubation, the treated cells were devoid of the intracellular punctuates and the staining pattern of both the cells incubated with Fab and MFC was reminiscent of an early co-incubation time point of IgG+MFC. The punctate formation could also been seen when both cetixumab and M425 (anther anti-EGFR mAb) were applied to MDA-MB-468, indicating that the punctates arose from receptor crosslinking.

When co-incubated, the tetravalent meditope-Fc (M4FC) also showed overlapping location with cetuximab. However, unlike the bivalent MFC which was observed even following overnight incubation with the cells, the M4FC became barely detectable after more than 3.5 h of incubation. Moreover, the internalization of M4FC occurred faster than MFC and was observed as early as 20 min.

These studies demonstrated a rapid loss of fluorescence signal of a fluorescently conjugated tetravalent conjugated meditope, suggesting that this combination of multivalent meditope and agent is rapidly shuttled to the lysosome and proteolyzed.

Drugs that block specific endocytic pathways (such as Dynasore, for clathrin-mediated endocytosis and EIPA for micropinocytosis) are used to treat cells and observe effects on localization of meditope-enabled antibodies, e.g., cetuximab, and multivalent meditopes, e.g., meditope-Fc.

In one example, Dynasore, a cell permeable inhibitor of dynamin inhibitor that can block clathrin-meditated endocytosis, was used. Cells were pretreated with 50 μM of Dynasore for one hour before addition of M4FC and cetuximab for an additional three hours. Following this treatment, both cetuximab and M4FC were observed to be accumulating in the perinuclear region. Cells were stained with M425 (another anti-EGFR antibody) post-incubation to assess location of the antigen. The intracellular retention of the multivalent meditope and meditope-enabled antibody were more pronounced when contrasted with the localization of EGFR, as demarked by M425 staining. Without the formation of clathrin-coated vesicles (in the presence of Dynasore), less cetuximab was seen on the plasma membrane. The effect may also indicate a separation of cetuximab from EGFR. The plasma membrane association of M4FC was already weaker than cetuximab, and the dynamin blockage caused a more prominent intracellular presence.

Together, these data indicate that a multivalent meditope can be conjugated with agents, such as imaging, therapeutic, and/or cytotoxic agents, e.g., cytotoxins, in order to effectively release drugs to targeted cells. These data indicate that the multivalent meditopes c be used in combination with meditope-enabled antibodies to induce the internalization of receptor antigens which have long life times, non-internalizing antigens, or slow-internalizing antigens, such as CEA, including such antigens that are tumor-specific.

## EXAMPLE 5: BINDING STUDIES USING 83E MEDITOPE ENABLED ANTIBODIES

The effect of altering residue 83 in the VH region of a meditope-enabled antibody was examined using a variety of techniques. As discussed below, the use of in silico modeling as well as in vitro and ex vivo binding studies demonstrate the ability of this alteration of the meditope binding pocket to increase meditope binding affinity to a meditope-enabled antibody.

A graphical representation of the crystal structure of the I83E mutant trastuzumab meditope enabled antibody was superimposed on meditope enabled antibody trastuzumab, version 1, which has an isoleucine at position 83 of the VL. The amino acid substitution made to meditope-enable the antibody failed to significantly alteration in backbone of the target antibody, or in the current figure the antibody fragment.

The model illustrates the effect of changing the residue at position 83 from isoleucine to glutamate. The glutamate side chain appears to form an electrostatic bond with the meditope. As this interaction is mostly buried, the dielectric is predicted to range from ~4 (inside of protein) from 80 (average water dielectric). The force between two charges is q1*q2*R/4*pi*e*r|R|^2 - where R is the vector between the two charges, q is the charge and e is the dielectric. While the dielectric at the position of Arg9 is not known, it is likely much less than 80. For argument's sake, if it is 4 then the interaction between the glutamate and the arginine is ~20 stronger. This relationship may explain the increase in affinity between the I83E versions of the meditope enabled antibody antibodies and the meditope, discussed below.

The binding of a meditope to a meditope-enabled antibody containing the I83E modification was next examined in an in vitro binding study using surface plasmon resonance (SPR) and a meditope enabled antibody pertuzumab with the I83E modification. An SPR sensosogram showed the binding interaction of meditope CQFDLSTRRLKC (SEQ ID NO:1) with immobilized pertuzumab I83E IgG. Concentrations were two fold dilutions from approximately 10 um to 156 nM. The data indicate a high Kd, so high in fact that no Kd could be fit to the data. Further optimization of the experiment will be required due to the long off rate observed.

In the next study, the binding of a meditope enabled antibody UCHT1 antibody with the I83E modification was compared to an UCHT1 antibody lacking this substitution. T cells enriched from the PBMCs of a healthy donor were cultured overnight in RPMI Complete Medium. Cells were harvested and 1ug of parental UCHT1 (not meditope enabled) or meditope-enabled UCHT1 was added to the cells at 4°C for 15 minutes. Cells were washed with PBS + 2% BSA then stained with AlexaF-647 labeled meditope at 4°C for 30 minutes. Cells were washed and resuspended in DAPI solution. Samples were run on the CYAN machine and analyzed using FlowJo Software. Cells are gated on $DAPI^-$ lymphocytes. The results demonstrate that altering the meditope binding site in the antibody by changing residue 83 to a glutamate does not negative affect antigen binding as demonstrated by the clear signal shift in two different cell types.

## EXAMPLE 6: RECEPTOR ENDOCYTOSIS USING AN MEDITOPE ENABLED ANTIBODY M5A ANTI-CEA ANTIBODY

Carcinoembryonic antigen (CEA) is a tumor marker for cancers such as colorectal carcinoma and others and is the focus of a tremendous amount of research. In this example, a meditope enabled antibody anti-CEA antibody (M5A) containing the 83E alteration was used to binding affinity.

LS174T cells, a human CEA-producing colonic tumor cell line, were used to study antibody binding. The LS174T cells were trypsinized and filtered with 40 $\mu$m filter to remove debris, and then were spun down at 200 g for 5 min. The cell medium was removed, and the cells were washed with 1 ml of 0.3% BSA-PBS. ~ $1 \times 10^6$ cells were used in each treatment and resuspended in 100 $\mu$l of wash buffer, and Alexa Fluor 647-conjugated M5A mAbs were added to a final concentration of 100 nM and incubated for ~5 min at room temperature. Each treatment was then split into two, and either Alexa Fluor 488-M4FC or MFC was added to a final concentration of 250 nM and the samples were further incubated for ~15 min. At the end of incubation, cells were washed once and resuspended in 1 ml of wash buffer supplemented with SYTOX blue for dead cell exclusion.

Cells were gated with forward and side scatter first and then with SYTOX blue.

The binding of the meditopes correlate with the affinity measured by SPR, with the tighter binders showing more cells bound with the meditopes. Moreover, the higher valency of the tetravalent meditope could be seen to make up the difference in affinity between the 13M and the 13M (I83E). These results show that the use of a multivalent meditope can improve the binding characteristics of a meditope enabled antibody antibody with a lower affinity for the meditope.

## EXAMPLE 7: HETERO-BIVALENT MPL: AFFIBODY FUSION PROTEINS FOR SURFACE RECEPTOR CROSS LINKING APPLICATIONS

There are a number of modified staphylococcal protein A variants which bind to surface cell receptors including, but not limited to, HER2 and IGF1R in addition to other proteins of interest for detection of disease. These are small proteins which have been deimmunized and are being developed for tissue imaging applications as a replacement to larger and more expensive monoclonal antibody (mAb) and mAb fragments. Non-limiting examples of this technology can be found in J Mol Biol. 2010 Apr 30; 398(2):232-47; Proc Natl Acad Sci USA. 2010 Aug 24;107(34):15039-44; Curr Pharm Biotechnol. 2010 Sep 1;11(6):581-9; Protein Eng Des Sel. 2011 Apr;24(4):385-96; and Eur J Nucl Med Mol Imaging. 2013 Feb;40(3):439-49.

Fusion proteins of meditope protein L (MPL) have been developed as tools for promoting cell surface protein clustering, with several of these affibody proteins in order to link meditope enabled antibodies (meditope enabled antibodies) to other surface cell receptors through geometries which are not accessible by using mAbs alone. The following affibody fusion proteins were produced:

-MPL-zHER2 (anti-human epithelial growth factor receptor affibody)
-zIGF1R-MPL (anti-insulin growth factor receptor affibody)

### *A. Materials/Methods*

*DNA constructs*

His6-SMT-MPL-zHER2 encoding sequence was ordered from Genscript in a pet28b+ modified vector as shown as shown in SEQ ID NO: 232 (nucleic acid) and SEQ ID NO: 233 (amino acid). An N-terminal S residue is present on the final purified protein as a result of the ULP1 protease cleavage step to remove the His6-SMT3 tag.

*zIGF1R-MPL*

zIGF1R- MPL was ordered from DNA 2.0 in the PJ411 vector as a tag-less construct. The nucleic acid sequence is shown in SEQ ID NO: 234 and the amino acid sequence is shown in SEQ ID NO: 235.

### *B. Protein Expression/Purification*

*His6-SMT3-MPL-zHER2*

The plasmid was transformed into BL21 (DE3) competent cells and plated on Kan LB agar plates overnight at 37 C. Single colonies were selected and grown in 50 mL LB media with Kan as starter cultures O/N for large scale expression. 10 mL of overnight culture was used to inoculate 1L LB with Kan in 2L shaker flasks. Flasks were shaken at 37 C until OD600 values of roughly 0.6 were achieved. Protein expression was induced with 200 μL of 1M IPTG and cultures were allowed to continue shaking for 4 hrs. Cells were then harvested by centrifugation and resuspended in 1X PBS buffer pH 7.4. Cells were frozen at -20C until purification.

Prior to purification, cells were thawed to room temperature and lysed by French pressure cell. Cell lysates were clarified by centrifugation at 4C for 45 minutes at 45,000 rpm. Cell lysates were filtered prior to affinity chromatography purification. Ni-NTA affinity chromatography was used for initial purification using PBS and imidazole gradients for washing and elution. The eluted protein was cleaved overnight at 4C with ULP1 enzyme with 10 mM DTT while dialyzing into PBS. The O/N dialyzed protein was then further purified by reverse Ni-NTA chromatography. The column flow through and wash was then concentrated and applied to a preparative grade S75 gel filtration column for final purification. The final material was concentrated, aliquot into small volumes, frozen and stored at -80C.

*zIGFIR-MPL*

The plasmid was transformed into BL21 (DE3) competent cells and plated on Kan LB agar plates O/N at 37 C. Single colonies were selected and grown in 50 mL LB media with Kan as starter cultures O/N for large scale expression. 12 mL of overnight culture was used to inoculate 1L LB with Kan in 2L shaker flasks. Flasks were shaken at 37 C until OD600 values of roughly 0.6 were achieved. Protein expression was induced with 200 μL of 1M IPTG and cultures were allowed to continue shaking for 4 hrs. Cells were then harvested by centrifugation and resuspended in 1X PBS buffer pH 7.4. Cells were frozen at -20C until purification.

Prior to purification, cells were thawed to room temperature and lysed by French pressure cell. Ammonium

Sulfate was added (30% m/v) and the lysate was stirred for 1 hr by magnetic stir bar at 4C. The protein solution was centrifuged at 3400 rpm for 20 min. the supernatant and pellet were carefully separated. The pellet was resuspended in 20 mL PBS and dialyzed O/N with 1 buffer change after 2.5 hrs. The dialyzed material was concentrated and applied to a preparative grade S75 gel filtration column for final purification. The final material was concentrated, aliquot into small volumes, frozen and stored at -80C.

### *C. Binding Assays:*

MPL-zHER2 binding interactions with both meditope enabled trastuzumab (through the MPL portion of the fusion protein) and to HER2, through the zHER2 affibody portion of the fusion protein were monitored by surface plasmon resonance (SPR). Additional optimization may be required to calculate binding affinities due to the long off rate of the very stable interaction.

SPR was carried out at 25 C using HBS-EP+ as running buffer. Either meditope enabled antibody trastuzumab or the extracellular portion of HER2 were immobilized on a series S CM5 sensor chip using EDC/NHS amine coupling. 2 fold dilutions of analyte protein (MPL-zHER2 or zIGF 1R-MPL) were flown over the immobilized surface at 30 μL/min flow rate.

SEC binding assay was carried out at 4 C on an analytical superdex 200 gel filtration column in PBS buffer. Different ratios of meditope enabled antibody trastuzumab IgG and zIGF1R-MPL were first pre-incubated on ice for 30 min. prior to loading. An SPR sensogram showing the interaction of MPL-zHER2 with immobilized meditope enabled trastuzumab IgG was generated. An SPR sensogram showing the binding interaction of MPL-zHER2 with immobilized HER2 was also generated.

*zIGF1R-MPL binding interactions with meditope enabled trastuzumab*

An SPR sensogram of the interaction of zIGF1R-MPL with immobilized meditope enabled trastuzumab yielded a calculated $K_D$ of 0.93 nM.

## EXAMPLE 8: INCREASING ANTIBODY INTERNALIZATION USING MEDITOPE-ENABLED ANTIBODY / MEDITOPE-Fc CROSSLINKING

### *A. Antibody Production*

Meditope-enabled gemtuzumab antibodies having a light chain comprising SEQ ID NO: 224 and a heavy chain comprising SEQ ID NO: 225 were produced in transfected FREESTYLE™ 293-F cells (Invitrogen, Carlsbad, CA). The cells were cultured in FREESTYLE™ 293 Expression Medium (Invitrogen, Carlsbad, CA). On the day of transfection, cells were collected and centrifuged at 300g for 5 minutes at room temperature, yielding a final concentration of $1.1 \times 10^6$ cells per ml.

DNA for transfection was prepared as follows: 1μg of endotoxin-free expression plasmid was added to Optimem medium (Invitrogen, Carlsbad, CA) to a total volume of 33.5μl for each ml of transfection volume. Separately, 1.33 μl 293Fectin™ (Invitrogen, Carlsbad, CA) was added to 32.17 μl Optimem medium (Invitrogen, Carlsbad, CA) for each ml of transfection volume at room temperature, mixed gently, and incubated at room temperature for 5 minutes. The DNA mixture and 293Fectin™ mixture were then combined, mixed, and incubated for 20-30 minutes at room temperature.

The DNA transfection mixture was added to the FREESTYLE™ 293-F cells (Invitrogen, Carlsbad, CA) in a shaker flask at a ratio of 67 μl of transfection mixture per ml of cells. The transfected cells were incubated for 4-6 days at 125 RPM at 85% humidity, 8% $CO_2$, and 37 °C. Cells were then collected and centrifuged for 10 minutes at 3000g. The supernatant was collected from the centrifuged cells and sterilized using a 0.22 micron filter. The flow-through was stored in a sterile 20mM histidine + 5% sucrose buffer at 4 °C at a final concentration of 5mg/ml, as analyzed by spectrophotometry (Nanodrop 2000c, Thermo Scientific, Wilmington, DE). Production of intact antibody was confirmed by visualization on a standard protein gel.

### *B. Antibody Labeling*

Meditope-enabled gemtuzumab antibodies were labeled with pHrodo™ Red SE (ThermoFisher Scientific, Waltham, MA) according to manufacturer's instructions. The pHrodo™ dye is pH sensitive; its fluorescence increases as pH decreases. Thus, quantifying the fluorescence of pHrodo™-labeled antibodies indicates the rates of antibody endocytosis. Briefly, pHrodo™ was prepared in dry dimethylsulfoxide (DMSO) (Invitrogen) to achieve a final concentration of 2-5 μM of dye. 2mg of antibody was dissolved in 2mL of phosphate buffered saline (PBS) at pH 9. Fluorescent probe was then added slowly in four 50ul increments to the antibody solution and incubated with each addition for 15 minutes

with rocking at room temperature. Meditope-Fc protein (SEQ ID NO: 236) was labeled with Alexa Fluor 488.

### *C. Fluoresence Microscopy*

HL-60 cells, a human promyelocytic leukemia cell line were stained for fluorescence microscopy to determine receptor-mediated endocytosis rates of the pHrodo™-labeledantibody. Cells were stained in Nunc® Lab-Tek® II chambered 8 well coverglass polystyrene chambers (0.7 $cm^2$/well). Cells were stained using either 100 nM antibody and 100 nM of Alexa Fluor 488®-labeled meditope-Fc or 100 nM antibody alone (control) for 30 minutes at 37 °C in serum-free media. Nuclei of HL-60 cells were also stained with Hoechst 33342 (2 μg/mL final concentration for 20 minutes).

At the appropriate time points, coverslips were washed in ice-cold BSA/PBS before fixation in 4% paraformaldehyde (PFA). Wells were subsequently mounted in Fluoromount G (Southern Biotechnology, Birmingham, AL). Cells were imaged with a confocal microscope using appropriate wavelength settings for pHrodo™-labeled antibodies, Alexa Fluor 488-labeled meditope-Fc, and Hoechst 33342-labeled nuclei. Z sectioning permitted visualization of the internalized materials and all quantitative measurements were taken at the same defined section height. Total fluorescence of each stain inside and outside of imaged cells (n=9) were calculated using Imaged analysis at a single time point of 30 minutes. Total staining for gemtuzumab alone was calculated as 6604.57±463.95. Total staining for gemtuzumab bound to meditope-Fc was calculated as 11611.83±1140.44, indicating that HL-60 cells internalize antibody bound to meditope-Fc at nearly twice the rate of antibody alone (p=2.44E-08).

## EXAMPLE 9: INCREASING ANTIBODY INTERNALIZATION USING MEDITOPE-ENABLED ANTIBODY / MEDITOPE-Fc CROSSLINKING

### *A. Antibody Production*

Meditope-enabled MOR208 antibodies having a light chain comprising SEQ ID NO: 226 and a heavy chain comprising SEQ ID NO: 227 were produced in transfected FREESTYLE™ 293-F cells (Invitrogen, Carlsbad, CA) human embryonic kidney 293 (HEK-293) as described above.

### *B. Antibody Labeling*

Raji-B cells, a human B lymphocyte cell line derived from human lymphoblast, CCL-86™, ATCC®, were stained for fluorescence microscopy to determine receptor-mediated endocytosis rates of the pHrodo™-labeled CD-19 antibody. Cells were stained in Nunc® Lab-Tek® II chambered 8 well coverglass polystyrene chambers (0.7 $cm^2$/well). Cells were stained using either 100 nM antibody and 100 nM of Alexa Fluor 488®-labeled meditope-Fc (SEQ ID NO: 236) or 100 nM antibody alone (control) for 30 minutes at 37 °C in serum-free media. Nuclei of Raji-B cells were also stained with Hoechst 33342 (2 μg/mL final concentration for 20 minutes).

Cells were imaged with a confocal microscope using appropriate wavelength settings for pHrodo™-labeled antibodies, Alexa Fluor 488-labeled meditope-Fc, and Hoechst 33342-labeled nuclei. Z sectioning permitted visualization of the internalized materials and all quantitative measurements were taken at the same defined section height. Cells were visualized and quantified at each time point for each visualization event. Total fluorescence of each stain inside and outside of imaged cells were calculated using Imaged analysis at a single time point of 30 minutes. Total staining for MOR-208 alone was calculated as 1797.71±194.97 (n=8). Total staining for MOR-208 bound to meditope-Fc was calculated as 4430.43±632.59 (n=8). The results demonstrate that Raji-B cells internalize MOR208 antibody bound to meditope-Fc at a rate more than twice that of antibody alone (p=3.94E-03).

## EXAMPLE 10: MULTIVALENT MEDITOPE CELL LABELING

### *A. Multivalent Meditope Production*

Bivalent meditopes (BVM) and trivalent meditopes (TVM) were expressed from codon optimized genes in pET28b+ plasmids with an N-terminal His6-SMT3-TVM tag (GenScript). The BVM had a nucleic acid sequence of SEQ ID NO: 228 and a peptide sequence of SEQ ID NO: 229. The TVM had a nucleic acid sequence of SEQ ID NO: 230 and a peptide sequence of SEQ ID NO: 231.

Plasmids were transformed into BL21(DE3) competent cells for protein expression. Overnight cultures in terrific broth (TB) and kanamycin (Kan) were used to inoculate 1 L cultures of TB/Kan, which were and grown to O.D.600 = 0.5-0.6. Cultures were induced with IPTG at a final concentration of 500 μM and grown overnight on a shaker at 18°C or for four hours at 37 °C. Cells were harvested by centrifugation at 4000 rpm at 4°C for 30 minutes. Culture supernatants were discarded and cells were resuspended in PBS containing 300 mM NaCl and 10 mM imidazole and frozen at -20°C

until purification.

Cells were lysed by French pressure cell with DNASE1 and PMSF. Lysates were clarified by centrifugation at 45,000 rpm at 4°C for 30-45 min. Proteins were purified by immobilized metal ion affinity chromatography (IMAC) (Ni-NTA Qiagen). The His6-SMT tag was then cleaved by His6-ULP1, followed by separation by reverse IMAC. The reverse column flow through and wash was concentrated and applied to a HiLoad prep75 26/600 PG gel filtration column (GE). Representative chromatograms for both proteins are displayed in Figure 16A (TVM) and Figure 16B (BVM). Absorbance measurements at 280 nm, 230 nm, and 260 nm are shown. Fractions 21-23 were chosen for the TVM and fractions 26-29 were chosen for the BVM for further analysis.

SPR analysis of the chosen fractions indicated that the BVM and TVM bound meditope enabled antibody trastuzumab antibodies (Figure 17A and Figure 17B, respectively). TVM product was confirmed by HPLC (Figure 18A). The major peak at 4.2 minutes of Figure 18A is the correct monomeric TVM molecular weight and the peak at 5.3 minutes is a dimer. The fraction containing the major peak was analyzed by mass spectroscopy (Figure 18B).

### *B. Microscopy*

TVM was labeled with NHS-Alexa Fluor 647 following the manufacturer's protocol (Thermo Fisher Scientific, Waltham MA) 0.1 M sodium bicarbonate buffer pH 8.0 at room temperature for 1 hour). Labeled TVM was added to SKBR3 cells, HTB-30™, ATCC®, with meditope enabled antibody trastuzumab or parental trastuzumab, as described in Example 2.

Cells labeled with meditope enabled antibody trastuzumab showed normal morphology and remained attached to the plate. In contrast, cells labeled with meditope enabled antibody trastuzumab and trivalent meditope showed a rounded morphology and frequently detached from the surface of the coverslides. Cells labeled with meditope enabled antibody trastuzumab and trivalent meditope that could be imaged showed fluorescence corresponding to TVM-alexafluor 647 along the perimeter of the cell. This change in morphology was not observed with parental trastuzumab and trivalent meditope, indicating that the change in morphology and cell detachment were due to the combined treatment of the multivalent meditope with the meditope enabled antibody.

## EXAMPLE 11: ASSESSMENT OF CYTOTOXIC EFFECTS OF MEDITOPE-DRUG CONJUGATES

Cytotoxic effects on cells following incubation with meditope-enabled antibodies and multivalent meditopes conjugated to cytotoxins are assessed. A bivalent meditope Fc (MFC) and the tetravalent meditope-Fc described above (M4FC) are conjugated to auristatin

HL-60 cells are plated in a 96-well tissue culture plate at a density of 1000 cells/well. The cells are incubated overnight at 37 °C at 5% CO2. The next day the cells are incubated in triplicate under the following conditions: 1) unstained control; 2) meditope-enabled gemtuzumab antibodies alone; 3) meditope-enabled gemtuzumab antibodies with unlabeled MFC; and 4) meditope-enabled gemtuzumab antibodies with MFC coupled to auristatin. The cells are cultured for six days. At the end of the incubation period, 12 μl of Alamar Blue or Presto Blue is added to each well and incubated for four hours. The plates are read using a BioTek Synergy H4 plate reader using 540 Excitation and 590 Emission wavelengths.

Percentage cell survival is then calculated in each well and data is analyzed using non-linear fitting of sigmoidal dose response. IC50 values are calculated using Prism graphing software and cytotoxic effects of meditope-enabled gemtuzumab antibodies with MFC coupled to auristatin were compared with controls used in the experiment.

The data show that the meditope-enabled gemtuzumab antibodies with MFC coupled to auristatin induce cytotoxicity in HL-60 cells. Thus, the results demonstrate that meditope-enabled gemtuzumab antibodies with MFC coupled to auristatin cytotoxin can selectively deliver a cytotoxic drug to HL-60 cells.

## EXAMPLE 12: TESTING MULTIVALENT MEDITOPE VALENCY

Multivalent meditope binding was tested against various ligand densities to determine valency.

Meditope-enabled trastuzumab version 2 (light chain of SEQ ID NO: 6 and heavy chain of SEQ ID NO: 9) was immobilized on a CM5 chip using amine coupling chemistry (EDC/NHS) at varying densities. The low density channel was approximately 150 RU (Figure 19A), the medium density channel was approximately 750 RU (Figure 19B), and the high density channel was approximately 1500 RU (Figure 19C).

Trivalent meditope (TVM) (SEQ ID NO: 231) concentrations were prepared as a 5-fold dilution series from 1 μM to 2.6 pM in HBS-EP+. Samples were flowed over all four channels at 30 μL/min at 37°C. Sensograms were double referenced to the blank immobilization on channel 1 and with two "0" concentration buffer blanks.

A control meditope peptide was run at the beginning and end of the experiment to assess for loss of surface activity during the experiment. Less than 5% decrease in signal was observed on the most dense ligand surface after

the experiment was completed. Regeneration buffer for the experiment was a pulse of 10 mM glycine pH 3.0 with variable time from 2-10 seconds followed by a 30 sec pulse of running buffer HBS-EP+.

Data fits were performed using BiaEvaluation software with the 1:1 fit model. Data fit quality to the 1:1 binding model decreased as the surface density of ligand increased and the off rate was not well determined due to lack of curvature. Data are shown in Table 5.

TABLE 5

| Density (RU) | 150 | 750 | 1500 |
|---|---|---|---|
| $K_a$ ($M^{-1}s^{-1}$) | 8700 | 10000 | 9800 |
| $K_d$ ($s^{-1}$) | 4.5 E-4 | 1.4 E-4 | 8.0 E-5 |
| $K_D$ (M) | 5.12 E-8 | 1.4 E-8 | 8.2 E-9 |
| $t_{1/2}$ (hr) | 0.4 | 1.3 | 2.4 |

The data indicate that the number of individual multivalent meditopes that bind to more than one meditope enabled antibody increases as meditope enabled antibody density increases. As the density increases, the distance between individual meditope enabled antibodies on the chip decreases. Therefore, the chance that two or more antibodies will be within reach of a single multivalent meditope increases, and more multivalent binding is expected.

This effect was observed. The data showed increasing deviation from 1: 1 fit as ligand density increased, which is indicative of avidity (Figure 20). The calculated dissociation constant at the low density surface is comparable to a monovalent meditope, previously calculated to be 38 nM, though the kinetic constants are different. The half-life for the monovalent meditope at 37 °C was 4.7 minutes and the half-life calculated for the trivalent meditope on the most densely immobilized surface is 145 min.; a >30 fold improvement. Data for the higher densities show increasing valency and a longer off-rate. The data indicate that multivalent meditopes are capable of multivalent binding corresponding to increasing ligand densities.

The following items are preferred embodiments:

1. A method for altering the distribution of a cell surface antigen, comprising:

contacting a cell with a plurality of meditope enabled antibodies or antigen binding fragments thereof capable of binding to a cell surface antigen;
contacting a meditope binding site of a first meditope enabled antibody or antigen binding fragment thereof of the plurality of meditope enabled antibodies with a first meditope from a multivalent meditope;
contacting a meditope binding site of a second meditope enabled antibody or antigen binding fragment thereof of the plurality of meditope enabled antibodies with a second meditope from the multivalent meditope, resulting in crosslinking of the first and second meditope-enabled antibodies, whereby crosslinking the first and second meditope enabled antibodies with the multivalent meditope alters the distribution of the cell surface antigen.

2. A method for increasing co-localization of a cell surface antigen, comprising:

contacting a cell with a plurality of meditope enabled antibodies or antigen binding fragments thereof capable of binding to a cell surface antigen;
contacting a meditope binding site of a first meditope enabled antibody or antigen binding fragment thereof of the plurality of meditope enabled antibodies with a first meditope from a multivalent meditope;
contacting a meditope binding site of a second meditope enabled antibody or antigen binding fragment thereof of the plurality of meditope enabled antibodies with a second meditope from the multivalent meditope, resulting in crosslinking of the first and second meditope-enabled antibodies, whereby crosslinking the first and second meditope enabled antibodies with the multivalent meditope increases the co-localization of the cell surface antigen or receptor.

3. A method for increasing cellular internalization of a meditope enabled antibody, comprising:

contacting a cell expressing a cell surface antigen with a plurality of meditope enabled antibodies or antigen binding fragments thereof, whereby said plurality of meditope enabled antibodies or antigen binding fragments thereof bind to the cell surface antigen;
contacting a meditope binding site of a first meditope enabled antibody or antigen binding fragment thereof of

the plurality of meditope enabled antibodies with a first meditope from a multivalent meditope;
contacting a meditope binding site of a second meditope enabled antibody or antigen binding fragment thereof of the plurality of meditope enabled antibodies with a second meditope from the multivalent meditope, resulting in crosslinking of the first and second meditope-enabled antibodies, whereby crosslinking the first and second meditope enabled antibodies with the multivalent meditope increases cellular internalization of the first and second meditope enabled antibodies bound to the cell surface antigen.

4. A method for increasing cellular internalization of a cell surface antigen, comprising:

contacting a first cell surface antigen with a first meditope enabled antibody or antigen binding fragment thereof that binds to the cell surface antigen;
contacting a second cell surface antigen with a second meditope enabled antibody or antigen binding fragment thereof that binds to the second cell surface antigen;
contacting a meditope binding site of the first meditope enabled antibody or antigen binding fragment thereof of the plurality of meditope enabled antibodies with a first meditope from a multivalent meditope;
contacting a meditope binding site of the second meditope enabled antibody or antigen binding fragment thereof of the plurality of meditope enabled antibodies with a second meditope from the multivalent meditope, thereby crosslinking of the first and second meditope enabled antibodies, and increasing cellular internalization of the first and second cell surface antigen.

5. A method of increasing the efficacy of an antibody therapy, comprising:

administering to a subject an effective amount of a meditope enabled antibody or antigen binding fragment thereof that specifically binds to a cell surface antigen and an effective amount of a multivalent meditope;
forming a complex of a first meditope of the multivalent meditope bound to a first meditope enabled antibody or antigen binding fragment thereof bound to a first cell surface antigen; and a second meditope of the multivalent meditope bound to a second meditope enabled antibody or fragment thereof bound to a second cell surface antigen, resulting in crosslinking of the first and second meditope-enabled antibodies, whereby crosslinking the first and second antibodies increases the efficacy of the antibody therapy.

6. A method of decreasing a dosage of an antibody therapy needed to achieve a desired therapeutic effect in a subject comprising

administering to a subject an effective amount of a meditope enabled antibody or fragment thereof and an effective amount of a multivalent meditope;
contacting a first meditope of the multivalent meditope to a first meditope enabled antibody or fragment thereof;
contacting a second meditope of the multivalent meditope to a second meditope enabled antibody or fragment thereof, resulting in crosslinking of the first and second meditope enabled antibodies, whereby crosslinking the first and second antibodies decreases the dosage of the antibody therapy needed to achieve the desired therapeutic effect in a subject.

7. A method of crosslinking a first meditope enabled antibody or antigen binding fragment thereof and a second meditope enabled antibody or antigen binding fragment thereof comprising:

contacting a cell with a first meditope enabled antibody or antigen binding fragment thereof, wherein said first meditope enabled antibody or antigen binding fragment thereof comprises a first meditope binding site;
contacting said first meditope binding site with a multivalent meditope, said multivalent meditope comprising a first meditope attached to a second meditope;
contacting said multivalent meditope with a second meditope enabled antibody or antigen binding fragment thereof, wherein said second meditope enabled antibody or antigen binding fragment thereof comprises a second meditope binding site;
allowing said first meditope binding site to bind said first meditope, said second meditope binding site to bind said second meditope, said first meditope enabled antibody or antigen binding fragment thereof to bind to said cell and said second meditope enabled antibody or antigen binding fragment thereof to bind to said cell, thereby crosslinking said first meditope enabled antibody or antigen binding fragment thereof and said second meditope enabled antibody or antigen binding fragment thereof.

8. The method of any one of the preceding items, wherein the cell surface antigen is a receptor capable of receptor-mediated endocytosis.

9. The method of any one of the preceding items, wherein the multivalent meditope comprises an immunoglobulin Fc region or portion thereof linked to a meditope.

10. The method of any one of the preceeding items, wherein a meditope of the multivalent meditope is coupled to a linker.

11. The method of any one of the preceding items, wherein the linker comprises a sequence of SEQ ID NO: 199, 200, 201, 202, 203, or 204, or a variant thereof.

12. The method of any one of the preceding items, wherein the multivalent meditope is coupled to a therapeutic agent or diagnostic agent.

13. The method of item 12, wherein:

the therapeutic agent is selected from the group consisting of a chemotherapeutic agent, a therapeutic antibody, a toxin, a radioisotope, an enzyme, a chelator, a boron compound, a photoactive agent, a dye, a metal, a metal alloy, and a nanoparticle; or
the diagnostic agent is an imaging agent selected from the group consisting of a fluorescent substance, a luminescent substance, a dye, and a radioactive isotope.

14. The method of any one of the preceding items, wherein the first meditope enabled antibody binds to a different epitope than the second meditope enabled antibody.

15. The method of any of the preceding items, wherein the meditope enabled antibody or antigen-binding fragment thereof specifically binds to an antigen expressed by a disease or condition of a cell or tissue thereof.

16. The method of item 15, wherein the disease or condition is a cancer.

17. The method of any one of the preceding items, wherein the meditope enabled antibody or antigen-binding fragment thereof and the meditope are administered sequentially.

18. The method of any one of the preceding items, wherein at least one of the meditope enabled antibodies or antigen binding fragments thereof competes for antigen binding with, or binds to the same epitope as an antibody or antigen-binding fragment thereof selected from the group consisting of abagovomab, abciximab, adalimumab, adecatumumab, alemtuzumab, altumomab, altumomab pentetate, anatumomab, anatumomab mafenatox, arcitumomab, atlizumab, basiliximab, bectumomab, ectumomab, belimumab, benralizumab, bevacizumab, brentuximab, canakinumab, capromab, capromab pendetide, catumaxomab, certolizumab, clivatuzumab tetraxetan, daclizumab, denosumab, eculizumab, edrecolomab, efalizumab, etaracizumab, ertumaxomab, fanolesomab, fontolizumab, gemtuzumab, girentuximab, golimumab, ibritumomab, igovomab, infliximab, ipilimumab, labetuzumab, mepolizumab, muromonab, muromonab-CD3, natalizumab, necitumumab nimotuzumab, ofatumumab, omalizumab, oregovomab, palivizumab, panitumumab, ranibizumab, rituximab, satumomab, sulesomab, ibritumomab, ibritumomab tiuxetan, tocilizumab, tositumomab, trastuzumab,-ustekinumab, visilizumab, votumumab, zalutumumab, brodalumab, anrukinzumab, bapineuzumab, dalotuzumab, demcizumab, ganitumab, inotuzumab, mavrilimumab, moxetumomab pasudotox, rilotumumab, sifalimumab, tanezumab, tralokinumab, tremelimumab, and urelumab; or
the meditope-enabled antibody or fragment specifically binds to an antigen selected from the group consisting of: CA-125, glycoprotein (GP) IIb/IIIa receptor, TNF-alpha, CD52, TAG-72, Carcinoembryonic antigen (CEA), interleukin-6 receptor (IL-6R), IL-2, interleukin-2 receptor a-chain (CD25), CD22, B-cell activating factor, interleukin-5 receptor (CD125), VEGF, VEGF-A, CD30, IL-1beta, prostate specific membrane antigen (PSMA), CD3, EpCAM, EGF receptor (EGFR), MUC1, human interleukin-2 receptor, Tac, RANK ligand, C5 or other complement proteins, CD11a, alpha-v beta-3 integrin, HER2, neu, CD15, CD20, Interferon gamma, CD33, CA-IX, CTLA-4, IL-5, CD3 epsilon, CAM, Alpha-4-integrin, IgE, IgE Fc region, an RSV antigen, F (or fusion) protein of respiratory syncytial virus (RSV), NCA-90 (granulocyte cell antigen), IL-6, GD2, GD3, IL-12, IL-23, IL-17, CTAA16.88, beta-amyloid, IGF-1 receptor (IGF-1R), delta-like ligand 4 (DLL4), alpha subunit of granulocyte macrophage colony stimulating factor receptor, hepatocyte growth factor, IFN-alpha, nerve growth factor, IL-13, CD326, CD19, PD-L1, CD47, and CD137.

19. The method of any of the preceding items, wherein the multivalent meditope comprises a peptide having the

formula:

X1-X2-X3-X4-X5-X6-X7-X8-X9-X10-X11-X12 **(formula VI)**

wherein:

X1 is Cys, Gly, β-alanine, diaminopropionic acid, β-azidoalanine, or null;
X2 is Gln or null;
X3 is Phe, Tyr, β,β'-diphenyl-Ala, His, Asp, 2-bromo-L-phenylalanine, 3-bromo-Lphenylalanine, 4-bromo-L-phenylalanine, Asn, Gln, a modified Phe, a hydratable carbonyl-containing residue, or a boronic acid-containing residue;
X4 is Asp or Asn;
X5 is Leu; β,β'-diphenyl-Ala; Phe; a non-natural analog of phenylalanine, tryptophan, or tyrosine; a hydratable carbonyl-containing residue; or a boronic acid-containing residue;
X6 is Ser or Cys;
X7 is Thr, Ser or Cys;
X8 is Arg, a modified Arg, or a hydratable carbonyl-containing residue or boronic acid-containing residue;
X9 is Arg or Ala;
X10 is Leu; Gln; Glu; β,β'-diphenyl-Ala; Phe; a non-natural analog of phenylalanine, tryptophan, or tyrosine; a hydratable carbonyl-containing residue; or a boronic acid-containing residue;
X11 is Lys; and
X12 is Cys, Gly, 7-aminoheptanoic acid, β- alanine, diaminopropionic acid, propargylglycine, isoaspartic acid, or null.

20. The method of any of the preceding items, wherein the multivalent meditope comprises the amino acid sequence of SEQ ID NO: 1, 2, 15, 16, 17, 18, 19, 20, 21, 22, 23, 24, 25, 26, 27, 28, 29, 30, 31, 32, 34, 35, 36, 37, 38, 39, 40, 41, 42, 43, 44, 45, 46, 47, 48, 49, 50, 51, 52, 53, 54, 55, 186, 187, 188, 189, or 207, or a cyclic peptide derived therefrom.

21. A multivalent meditope, comprising:
a first meditope and a second meditope coupled to a first linker, wherein the first linker comprises a PASylation sequence, a sortase sequence, an Ssp $I_c$ intein sequence, an Ssp $I_N$ intein sequence, and/or an aldehyde tag.

22. The multivalent meditope of item 21, further comprising a second linker.

23. The multivalent meditope of item 21, wherein the first linker comprises an Ssp $I_c$ intein sequence and an Ssp $I_N$ intein sequence.

24. The multivalent meditope of item 22, wherein the first linker comprises an Ssp $I_c$ intein sequence and the second linker comprises an Ssp $I_N$ intein sequence.

25. The multivalent meditope of item 21, wherein the sortase sequence comprises the sequence LPXTG (SEQ ID NO: 249), wherein X is any amino acid.

26. The multivalent meditope of item 21, 22, or 25, wherein the first linker and/or second linker further comprises a glycine residue.

27. The multivalent meditope of any of items 21-26, wherein the aldehyde tag comprises the sequence of SEQ ID NO: 247.

28. The multivalent meditope of any one of items 21-27, wherein the first and/or second linker further comprises a lysine, a tyrosine, a glutamic acid, an aspartic acid and/or an unnatural amino acid residue.

29. The multivalent meditope of any one of items 21-28, wherein the first and/or second linker is conjugated to a therapeutic or diagnostic agent.

## Claims

1. A meditope enabled antibody or antigen binding fragment thereof that specifically binds to a cell surface antigen and a multivalent meditope for use in a method of increasing the efficacy of an antibody therapy, said method comprising:

    administering to a subject an effective amount of a meditope enabled antibody or antigen binding fragment thereof that specifically binds to a cell surface antigen and an effective amount of a multivalent meditope;
    forming a complex of a first meditope of the multivalent meditope bound to a first meditope enabled antibody or antigen binding fragment thereof bound to a first cell surface antigen; and a second meditope of the multivalent meditope bound to a second meditope enabled antibody or fragment thereof bound to a second cell surface antigen, resulting in crosslinking of the first and second meditope-enabled antibodies, whereby crosslinking the first and second antibodies increases the efficacy of the antibody therapy.

2. A meditope enabled antibody or fragment thereof and a multivalent meditope for use in a method of decreasing a dosage of an antibody therapy needed to achieve a desired therapeutic effect in a subject, said method comprising

    administering to a subject an effective amount of a meditope enabled antibody or fragment thereof and an effective amount of a multivalent meditope;
    contacting a first meditope of the multivalent meditope to a first meditope enabled antibody or fragment thereof;
    contacting a second meditope of the multivalent meditope to a second meditope enabled antibody or fragment thereof, resulting in crosslinking of the first and second meditope enabled antibodies, whereby crosslinking the first and second antibodies decreases the dosage of the antibody therapy needed to achieve the desired therapeutic effect in a subj ect.

3. The meditope enabled antibody or fragment thereof and a multivalent meditope for the use of any one of the preceding claims, wherein the cell surface antigen is a receptor capable of receptor-mediated endocytosis.

4. The meditope enabled antibody or fragment thereof and a multivalent meditope for the use of any one of the preceding claims, wherein the multivalent meditope comprises an immunoglobulin Fc region or portion thereof linked to a meditope, and/or wherein a meditope of the multivalent meditope is coupled to a linker.

5. The meditope enabled antibody or fragment thereof and a multivalent meditope for the use of claim 4, wherein the linker comprises a sequence of SEQ ID NO: 199, 200, 201, 202, 203, or 204, or a variant thereof.

6. The meditope enabled antibody or fragment thereof and a multivalent meditope for the use of any one of the preceding claims, wherein the multivalent meditope is coupled to a therapeutic agent or diagnostic agent.

7. The meditope enabled antibody or fragment thereof and a multivalent meditope for the use of claim 6, wherein:

    the therapeutic agent is selected from the group consisting of a chemotherapeutic agent, a therapeutic antibody, a toxin, a radioisotope, an enzyme, a chelator, a boron compound, a photoactive agent, a dye, a metal, a metal alloy, and a nanoparticle; or
    the diagnostic agent is an imaging agent selected from the group consisting of a fluorescent substance, a luminescent substance, a dye, and a radioactive isotope.

8. The meditope enabled antibody or fragment thereof and a multivalent meditope for the use of any one of the preceding claims, wherein the first meditope enabled antibody binds to a different epitope than the second meditope enabled antibody.

9. The meditope enabled antibody or fragment thereof and a multivalent meditope for the use of any one of the preceding claims, wherein the meditope enabled antibody or antigen-binding fragment thereof specifically binds to an antigen expressed by a disease or condition of a cell or tissue thereof.

10. The meditope enabled antibody or fragment thereof and a multivalent meditope for the use of claim 9, wherein the disease or condition is a cancer.

11. The meditope enabled antibody or fragment thereof and a multivalent meditope for the use of any one of the preceding

claims, wherein the meditope enabled antibody or antigen-binding fragment thereof and the meditope are administered sequentially.

**12.** The meditope enabled antibody or fragment thereof and a multivalent meditope for the use of any one of the preceding claims, wherein at least one of the meditope enabled antibodies or antigen binding fragments thereof competes for antigen binding with, or binds to the same epitope as an antibody or antigen-binding fragment thereof selected from the group consisting of abagovomab, abciximab, adalimumab, adecatumumab, alemtuzumab, altumomab, altumomab pentetate, anatumomab, anatumomab mafenatox, arcitumomab, atlizumab, basiliximab, bectumomab, ectumomab, belimumab, benralizumab, bevacizumab, brentuximab, canakinumab, capromab, capromab pendetide, catumaxomab, certolizumab, clivatuzumab tetraxetan, daclizumab, denosumab, eculizumab, edrecolomab, efalizumab, etaracizumab, ertumaxomab, fanolesomab, fontolizumab, gemtuzumab, girentuximab, golimumab, ibritumomab, igovomab, infliximab, ipilimumab, labetuzumab, mepolizumab, muromonab, muromonab-CD3, natalizumab, necitumumab nimotuzumab, ofatumumab, omalizumab, oregovomab, palivizumab, panitumumab, ranibizumab, rituximab, satumomab, sulesomab, ibritumomab, ibritumomab tiuxetan, tocilizumab, tositumomab, trastuzumab,-ustekinumab, visilizumab, votumumab, zalutumumab, brodalumab, anrukinzumab, bapineuzumab, dalotuzumab, demcizumab, ganitumab, inotuzumab, mavrilimumab, moxetumomab pasudotox, rilotumumab, sifalimumab, tanezumab, tralokinumab, tremelimumab, and urelumab; or
the meditope-enabled antibody or fragment specifically binds to an antigen selected from the group consisting of: CA-125, glycoprotein (GP) IIb/IIIa receptor, TNF-alpha, CD52, TAG-72, Carcinoembryonic antigen (CEA), interleukin-6 receptor (IL-6R), IL-2, interleukin-2 receptor a-chain (CD25), CD22, B-cell activating factor, interleukin-5 receptor (CD125), VEGF, VEGF-A, CD30, IL-1beta, prostate specific membrane antigen (PSMA), CD3, EpCAM, EGF receptor (EGFR), MUC1, human interleukin-2 receptor, Tac, RANK ligand, C5 or other complement proteins, CD11a, alpha-v beta-3 integrin, HER2, neu, CD15, CD20, Interferon gamma, CD33, CA-IX, CTLA-4, IL-5, CD3 epsilon, CAM, Alpha-4-integrin, IgE, IgE Fc region, an RSV antigen, F (or fusion) protein of respiratory syncytial virus (RSV), NCA-90 (granulocyte cell antigen), IL-6, GD2, GD3, IL-12, IL-23, IL-17, CTAA16.88, beta-amyloid, IGF-1 receptor (IGF-1R), delta-like ligand 4 (DLL4), alpha subunit of granulocyte macrophage colony stimulating factor receptor, hepatocyte growth factor, IFN-alpha, nerve growth factor, IL-13, CD326, CD19, PD-L1, CD47, and CD137.

**13.** The meditope enabled antibody or fragment thereof and a multivalent meditope for the use of any one of the preceding claims, wherein the multivalent meditope comprises a peptide having the formula:

X1-X2-X3-X4-X5-X6-X7-X8-X9-X10-X11-X12 **(formula VI)**

wherein:

X1 is Cys, Gly, β-alanine, diaminopropionic acid, β-azidoalanine, or null;
X2 is Gln or null;
X3 is Phe, Tyr, β,β'-diphenyl-Ala, His, Asp, 2-bromo-L-phenylalanine, 3-bromo-Lphenylalanine, 4-bromo-L-phenylalanine, Asn, Gln, a modified Phe, a hydratable carbonyl-containing residue, or a boronic acid-containing residue;
X4 is Asp or Asn;
X5 is Leu; β,β'-diphenyl-Ala; Phe; a non-natural analog of phenylalanine, tryptophan, or tyrosine; a hydratable carbonyl-containing residue; or a boronic acid-containing residue;
X6 is Ser or Cys;
X7 is Thr, Ser or Cys;
X8 is Arg, a modified Arg, or a hydratable carbonyl-containing residue or boronic acid-containing residue;
X9 is Arg or Ala;
X10 is Leu; Gln; Glu; β,β'-diphenyl-Ala; Phe; a non-natural analog of phenylalanine, tryptophan, or tyrosine; a hydratable carbonyl-containing residue; or a boronic acid-containing residue;
X11 is Lys; and
X12 is Cys, Gly, 7-aminoheptanoic acid, β- alanine, diaminopropionic acid, propargylglycine, isoaspartic acid, or null.

**14.** The meditope enabled antibody or fragment thereof and a multivalent meditope for the use of any one of the preceding claims, wherein the multivalent meditope comprises the amino acid sequence of SEQ ID NO: 1, 2, 15, 16, 17, 18, 19, 20, 21, 22, 23, 24, 25, 26, 27, 28, 29, 30, 31, 32, 34, 35, 36, 37, 38, 39, 40, 41, 42, 43, 44, 45, 46, 47, 48, 49, 50, 51, 52, 53, 54, 55, 186, 187, 188, 189, or 207, or a cyclic peptide derived therefrom.

**15.** The meditope enabled antibody or fragment thereof and a multivalent meditope for the use of any one of the preceding claims, wherein the use is in a method of treatment of cancer or a tumor.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62059146
- US 20030228309 A1
- US 20130017997 A
- WO 9852976 A
- WO 02079232 A
- WO 003317 A
- WO 2009051555 A
- EP 2065402 A1
- US 5558864 A
- US 8133982 B
- WO 2011112978 A
- WO 2010042562 A
- US 20110150760 A
- US 20120315276 A
- US 20040067503
- US 61597708
- US 27020711
- US 8658774 B
- US 2011055656 W
- US 1232938 W
- US 44380412
- US 8962804 B
- US 61749830
- US 76476213


### Non-patent literature cited in the description


- *Biochem J.,* 01 January 2004, vol. 377 (1), 159-69
- *J Nanobiotechnology.,* 03 February 2014, vol. 12, 5
- **MATZKU et al.** *Int. J. Cancer,* 1988, vol. 2, 11-14
- **REILLY et al.** *Clin. Pharmacokinet.,* 1995, vol. 28, 126-142
- **SCHMITD et al.** *Cancer Immunol Immunother.,* December 2008, vol. 57 (12), 1879-1890
- **MYER et al.** *Cancer Res,* 1993, vol. 53, 3956-3963
- **DECHANT M et al.** Complement-dependent tumor cell lysis triggered by combinations of epidermal growth factor receptor antibodies. *Cancer Res,* 01 July 2008, vol. 68 (13), 4998-5003
- **SCHEUER W et al.** Strongly enhanced antitumor activity of trastuzumab and pertuzumab combination treatment on HER2-positive human xenograft tumor models. *Cancer Res,* 15 December 2009, vol. 69 (24), 9330-6
- **CARDARELLI PM et al.** Binding to CD20 by anti-B1 antibody or F(ab')(2) is sufficient for induction of apoptosis in B-cell lines. *Cancer Immunol Immunother,* 18 December 2001, vol. 51 (1), 15-24
- **TRAIL et al.** *Antibodies,* 2013, vol. 2, 113-129
- **H.L.. PEREZ et al.** Antibody-drug conjugates: current status and future directions. *Drug Discov Today,* 2013
- **BOUDOUSQ V et al.** Comparison between Internalizing Anti-HER2 mAbs and Non-Internalizing Anti-CEA mAbs in Alpha-Radioimmunotherapy of Small Volume Peritoneal Carcinomatosis Using Pb. *PLoS ONE,* 2013, vol. 8 (7), e69613
- **ACKERMAN et al.** *Cancer Immunol Immunother.,* July 2008, vol. 57 (7), 1017-1027
- **JAZAYERI JA ; CARROLL GJ.** Fc-based cytokines: prospects for engineering superior therapeutics. *BioDrugs,* 2008, vol. 22 (1), 11-26
- **KAMAT, V. et al.** *Cancer Biol Ther,* 2008, vol. 7, 726-733
- **CHOU, P. Y. et al.** *Biochemistry,* 1974, vol. 13, 222-45
- **GARNIER J ; GIBRAT J F ; ROBSON B.** GOR method for predicting protein secondary structure from amino acid sequence. *Methods Enzymol,* 1996, vol. 266, 540-553
- **STICKLER, M. et al.** *J Immunol Methods,* 2003, vol. 281, 95-108
- **STURNIOLO, T. et al.** *Nat Biotechnol,* 1999, vol. 17, 555-61
- **STURNIOLO, T. et al.** *Nature Biotechnology,* 1999, vol. 17, 555
- **STURNIOLO, T. et al.** *Nat Biotechnol,* 1999, vol. 17, 555
- **SINGH, H. ; RAGHAVA, G.P.S.** ProPred: Prediction of HLA-DR binding sites. *Bioinformatics,* 2001, vol. 17 (12), 1236-37
- **SINGH, H. ; RAGHAVA, G. P. S.** *Bioinformatics,* 2003, vol. 19 (8), 1009-14
- **HANS-GEORG RAMMENSEE et al.** *Immunogenetics,* 1999, vol. 50, 213-219

- **KIM Y ; SIDNEY J ; PINILLA C ; SETTE A ; PETERS B.** *BMC Bioinformatics,* 2009, vol. 10, 394
- **J. HAKENBERG ; A. NUSSBAUM ; H. SCHILD ; H.-G. RAMMENSEE ; C. KUTTLER ; H.-G. HOLZHÜTTER ; P.-M. KLOETZEL ; S.H.E. KAUFMANN ; H.-J. MOLLENKOPF.** MAPPP - MHC-I Antigenic Peptide Processing Prediction. *Applied Bioinformatics,* 2003, vol. 2 (3), 155-158
- **NIELSEN M ; LUNDEGAARD C ; LUND O ; KESMIR C.** *Immunogenetics,* 2005, vol. 57 (1-2), 33-41
- **PARKER et al.** Scheme for ranking potential HLA-A2 binding peptides based on independent binding of individual peptide side-chains. *J. Immunol.,* 1994, vol. 152, 163-175
- **HANS-GEORG RAMMENSEE et al.** SYFPEITHI: database for MHC ligands and peptide motifs. *Immunogenetics,* 1999, vol. 50, 213-219
- **RECHE et al.** Prediction of MHC Class I binding peptides using profile motifs. *Hum Immunol.,* 2002, vol. 63 (9), 701-9
- **GREENBAUM et al.** *Journal of Molecular Recognition,* 2007, vol. 20 (2), 75-82
- **MORATH et al.** *Mol Pharm,* 04 May 2015, vol. 12 (5), 1431-42
- **SCOTT et al.** *Proc Natl Acad Sci USA.,* 23 November 1999, vol. 96 (24), 13638-43
- **ANTOS et al.** *J Biol Chem.,* 05 June 2009, vol. 284 (23), 16028-36
- **HUTCHINS et al.** *JMB,* 2011, vol. 406 (4), 595
- **GU et al.** *Nature Nanotechnology,* 2009, vol. 4, 245-248
- **DAVIES, J.S.** The Cyclization of Peptides and Depsipeptides. *J. Peptide Sci.,* 2003, vol. 9, 471-501
- **CARRICO et al.** *Nature Chemical Biology,* 2007, vol. 3, 321-322
- **KABAT E.A. et al.** Sequences of Proteins of Immunological Interest. NIH, 1991
- **KETTLEBOROUGH et al.** Humanization of a mouse monoclonal antibody by CDR-grafting: the importance of framework residues on loop conformation. *Protein Eng.,* 1991, vol. 4, 773-783
- **NELSON AL.** Antibody fragments: hope and hype. *MAbs,* 2010, vol. 2 (1), 77-83
- **GOLDENBERG DM et al.** Pretargeted molecular imaging and radioimmunotherapy. *Theranostics,* 2012, vol. 2 (5), 523-540
- **PAGEL JM et al.** Comparison of a tetravalent single-chain antibody-streptavidin fusion protein and an antibody-streptavidin chemical conjugate for pretargeted anti-CD20 radioimmunotherapy of B-cell lymphomas. *Blood,* 2006, vol. 108 (1), 328-336
- **KOEFOED K et al.** Rational identification of an optimal antibody mixture for targeting the epidermal growth factor receptor. *MAbs,* 2011, vol. 3 (6), 584-595
- **SPANGLER JB et al.** Combination antibody treatment down-regulates epidermal growth factor receptor by inhibiting endosomal recycling. *Proc Natl Acad Sci USA,* 2010, vol. 107 (30), 13252-13257
- **BASELGA J et al.** Pertuzumab plus trastuzumab plus docetaxel for metastatic breast cancer. *N Engl J Med,* 2012, vol. 366 (2), 109-119
- **MAMMEN M ; CHOI S ; WHITESIDES G.** Polyvalent interactions in biological systems: Implications for design and use of multivalent ligands and inhibitor. *Angew. Chem., Int. Ed. Eng,* 1998, vol. 37, 2755
- **VALEUR, B.** Molecular Fluorescence: Principles and Applications. John Wiley and Sons, 2002
- Handbook of Fluorescent Probes and Research Products, Molecular Probes. 2002, vol. 9
- The Handbook--A Guide to Fluorescent Probes and Labeling Technologies, Invitrogen
- **DESJARLAIS JR ; LAZAR GA.** Modulation of antibody effector function. *Exp Cell Res,* 2011, vol. 317 (9), 1278-1285
- **WOLF E ; HOFMEISTER R ; KUFER P ; SCHLERETH B ; BAEUERLE PA.** BiTEs: bispecific antibody constructs with unique anti-tumor activity. *Drug Discov Today,* 2005, vol. 10 (18), 1237-1244
- **KABAT et al.** Sequences of Proteins of Immunological Interest. Public Health Service, National Institutes of Health, 1991
- **AL-LAZIKANI et al.** *JMB,* 1997, vol. 273, 927-948
- **MACCALLUM et al.** *J. Mol. Biol.,* 1996, vol. 262, 732-745
- Antibody-antigen interactions: Contact analysis and binding site topography. *J. Mol. Biol.,* vol. 262, 732-745
- **LEFRANC MP et al.** IMGT unique numbering for immunoglobulin and T cell receptor variable domains and Ig superfamily V-like domains. *Dev Comp Immunol,* January 2003, vol. 27 (1), 55-77
- **HONEGGER A ; PLÜCKTHUN A.** Yet another numbering scheme for immunoglobulin variable domains: an automatic modeling and analysis tool. *J Mol Biol,* 08 June 2001, vol. 309 (3), 657-70
- Remington: The Science and Practice of Pharmacy. Lippincott Williams & Wilkins, Philadelphia, 2005
- Handbook of Pharmaceutical Salts. Properties, Selection and Use. Wiley-VCH, 2002
- **S. BERGE et al.** *Journal of Pharmaceutical Sciences,* 1977, vol. 66 (1), 1-19
- **P. GOULD.** *International J. of Pharmaceutics,* 1986, vol. 33, 201-217
- **ANDERSON et al.** The Practice of Medicinal Chemistry. Academic Press, 1996
- The Orange Book. Food & Drug Administration
- **CHO et al.** *Nature,* 2003, vol. 421, 756-760

- Polyvalent interactions in biological systems: Implications for design and use of multivalent ligands and inhibitor. **MAMMEN M, CHOI S ; WHITESIDES G.** Angew. Chem., Int. Ed. Eng. 1998, vol. 37, 2755
- **RODECK U et al.** *Cancer Res,* 1987, vol. 47 (14), 3692-3696
- *J Mol Biol.,* 30 April 2010, vol. 398 (2), 232-47
- *Proc Natl Acad Sci USA.,* 24 August 2010, vol. 107 (34), 15039-44
- *Curr Pharm Biotechnol.,* 01 September 2010, vol. 11 (6), 581-9
- *Protein Eng Des Sel.,* April 2011, vol. 24 (4), 385-96
- *Eur J Nucl Med Mol Imaging.,* February 2013, vol. 40 (3), 439-49